(19) Europäisches Patentamt
European Patent Office
Office européen des brevets

(11) EP 2 681 205 B1

(12) **EUROPEAN PATENT SPECIFICATION**

(45) Date of publication and mention of the grant of the patent:
**16.11.2016 Bulletin 2016/46**

(21) Application number: **12708291.5**

(22) Date of filing: **02.03.2012**

(51) Int Cl.:
***C07D 333/40*** (2006.01) ***C07C 233/00*** (2006.01)
***A61K 31/381*** (2006.01) ***A61P 19/10*** (2006.01)

(86) International application number:
**PCT/EP2012/053653**

(87) International publication number:
**WO 2012/117097 (07.09.2012 Gazette 2012/36)**

(54) **BIARYL DERIVATIVES AS SELECTIVE 17BETA-HYDROXYSTEROID DEHYDROGENASE TYPE 2 INHIBITORS**

BIARYL-DERIVATE ALS SELECTIVE 17-BETA-HYDROXYSTEROID-DEHYDROGENASE-2-HEMMER

DERIVÉS DE BIARYL EN TANT QU'INHIBITEURS SÉLECTIFS DE LA 17-BÊTA-HYDROXYSTÉROÏDE DÉSHYDROGÉNASE DE TYPE 2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.03.2011 EP 11156733**

(43) Date of publication of application:
**08.01.2014 Bulletin 2014/02**

(73) Proprietor: **Universität des Saarlandes**
**66123 Saarbrücken (DE)**

(72) Inventors:
- **HARTMANN, Rolf**
  **66111 Saarbrücken (DE)**
- **MARCHAIS-OBERWINKLER, Sandrine**
  **66440 Blieskastel (DE)**
- **XU, Kuiying**
  **66123 Saarbrücken (DE)**
- **WERTH, Ruth**
  **66125 Dudweiler (DE)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-03/068217**
**WO-A1-2008/124610**
**WO-A1-2008/156601**
**WO-A2-2004/110357**
**DE-A1- 2 946 481**

- **BROZIC P ET AL: "Derivatives of pyrimidine, phthalimide and anthranilic acid as inhibitors of human hydroxysteroid dehydrogenase AKR1C1", CHEMICO-BIOLOGICAL INTERACTIONS, ELSEVIER SCIENCE IRLAND, IR, vol. 178, no. 1-3, 16 March 2009 (2009-03-16), pages 158-164, XP025909728, ISSN: 0009-2797, DOI: DOI:10.1016/J.CBI.2008.10.019 [retrieved on 2008-10-22]**
- **GUNN D ET AL: "4,5-Disubstituted cis-pyrrolidinones as inhibitors of type II 17beta-hydroxysteroid dehydrogenase. Part 2. SAR", BIOORGANIC & MEDICINAL CHEMISTRY LETTERS, PERGAMON, ELSEVIER SCIENCE, GB, vol. 15, no. 12, 15 June 2005 (2005-06-15) , pages 3053-3057, XP004940184, ISSN: 0960-894X, DOI: DOI:10.1016/J.BMCL.2005.04.025**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 681 205 B1

- KUIYING XU ET AL: "Triazole ring-opening leads to the discovery of potent nonsteroidal 17-hydroxysteroid dehydrogenase type 2 inhibitors", EUROPEAN JOURNAL OF MEDICINAL CHEMISTRY, EDITIONS SCIENTIFIQUE ELSEVIER, PARIS, FR, vol. 46, no. 12, 4 October 2011 (2011-10-04), pages 5978-5990, XP028108430, ISSN: 0223-5234, DOI: 10.1016/J.EJMECH.2011.10.010 [retrieved on 2011-10-10]
- MICHAEL E. KORT ET AL: "Discovery and Biological Evaluation of 5-Aryl-2-furfuramides, Potent and Selective Blockers of the Na v 1.8 Sodium Channel with Efficacy in Models of Neuropathic and Inflammatory Pain", JOURNAL OF MEDICINAL CHEMISTRY, vol. 51, no. 3, 1 February 2008 (2008-02-01), pages 407-416, XP0055196919, ISSN: 0022-2623, DOI: 10.1021/jm070637u
- JONATHAN CLAYDEN ET AL: "Nucleophilic addition to electron-rich heteroaromatics: dearomatizing anionic cyclizations of pyrrolecarboxamides", ORGANIC LETTERS, AMERICAN CHEMICAL SOCIETY, US, vol. 6, no. 4, 1 January 2004 (2004-01-01) , pages 609-611, XP002444823, ISSN: 1523-7060, DOI: 10.1021/OL0364071

## Description

The invention relates to selective, non-steroidal 17beta-hydroxysteroid dehydrogenase type 2 (17β-HSD2) inhibitors their production and use, notably for the treatment and prophylaxis of sex steroid deficient diseases like osteoporosis in men and women.

## Background of the Invention

Estrogens and androgens play a crucial role in the development, growth and function of all tissues involved in reproduction and fertility. It is also well known, that estradiol (E2) and testosterone/dihydrotestosterone (T/DHT) the most active estrogen and androgen, respectively, can be involved in a series of hormone-sensitive diseases. For example estrogens or androgens are often responsible for the development of breast cancer or prostate cancer respectively, via stimulation of cell proliferation in the corresponding tissues (Travis, R.C. et al., Breast Cancer Res., 5:239-247 (2003); Wilding, G., Cancer Surv., 14:113-130 (1992)) or insufficient levels of E2 and T/DHT predispose the human skeleton to osteoporosis in both men and women (Pietschmann, P. et al., Gerontology, 55:3-12 (2009)).

Osteoporosis is a systemic skeletal disease characterized by deterioration of bone tissue and low bone mass, resulting in increased fragility of the bone and higher risk of fractures of the hip, spine and wrist. Osteoporotic fractures lead to pain, occasional disability and more important, they increase mortality (Cree, M. et al., J. Am. Geriatr. Soc., 48:283-288 (2000)).

In healthy individuals, bone mass is maintained by a balance between bone resorption and bone formation performed by the osteoclasts (OCs) and osteoblasts (OBs), respectively. This process of bone remodelling facilitates repair of microdamage, provides calcium uptake and therefore brings stability and strength to the bone.

Bone loss is, however, accelerated in post-menopausal women and in elderly men. The mechanisms by which elderly people, both men and women, lose bone are not fully understood and remain under investigation. Decreased quantity of sex hormones is one important factor causing bone loss. In women at menopause, estrogen deficiency (Cree, M. et al., J. Am. Geriatr. Soc., 48:283-288 (2000)) and in older men, estrogen and androgen insufficiency (Fink, H.A. et al., J. Clin. Endocrinol. Metab., 91:3908-3915 (2006); Meier, C. et al., Arch. Intern. Med., 168:47-54 (2008)) result in a disproportionate increase in bone loss as compared with bone formation and often lead to osteoporosis.

Since OBs and OCs express estrogen receptors (Hoyland, J.A. et al., Bone, 20:87-92(1997)) and respond to estrogen treatments, the most potent estrogen, E2 must have a direct effect on maintenance of bone mineral density. There are also substantial evidence that androgens like testosterone (T) and dihydrotestosterone (DHT) may as well be involved in bone formation, increasing OB activity and therefore protecting the bones against osteoporosis (Vanderschueren, D. et al., Endocr. Rev., 25:389-425 (2004), Vanderschueren, D. et al., Curr. Opin. Endocrinol. Diabetes Obes., 15:250-254 (2008)). T and DHT might act via activation of the androgen receptor (AR) which is present in the OBs (Bland, R., Clin. Sci., 98:217-240(2000)) or could be the precursor of estrogens (the enzyme aromatase, responsible for the transformation of androgens in estrogens has been identified in the OBs). Controlled increase of E2 and T in bones of osteoporotic patients will simultaneously lower bone resorption (effect of E2) and raise bone formation (effect of T) improving bone loss and osteoporotic fractures.

Augmentation of E2 and T levels in bones might be achieved by inhibition of the enzyme 17β-hydroxysteroid dehydrogenase type 2 (17β-HSD2) which has been identified in OBs (Dong, Y. et al., J. Bone Miner. Res., 13:1539-1546 (1998); Feix, M. et al., Mol. Cell. Endocrinol., 171:163-164 (2001); Janssen, J:M:et al., J. Cell. Biochem., 75:528-37 (1999)). A restricted increase in E2 and T in OCs and OBs is important to avoid unwanted side-effects such as induction of breast cancer or prostate cancer. This might be achieved via an intracrine mechanism (Labrie, F., Mol. Cell. Endocrinol. 78:C113-118 (1991)), i.e. the transformation of E2 and T in inactive precursors should be blocked dominantly in the bone cells.

17β-HSD2 (Wu, L. et al., J. Biol. Chem., 268:12964-12969 (1993); Lu, M., J. Biol. Chem., 277:22123-22130 (2002)) belongs to the hydroxysteroid dehydrogenase (HSD) superfamily. The HSD enzymes play pivotal roles not only in the activation but also in the inactivation of steroid hormones. Depending on the need of the cell, they modulate the potency of the sex hormones (Penning, T.M. et al., Endocr. Rev., 18:281-305 (1997)). For example, 17β-HSD1 activates E1 into the very potent E2, while 17β-HSD2 oxidizes E2 into E1 thus decreasing the action of the potent E2. It is therefore believed that in physiology 17β-HSD2 protects the cell against excessive level of active estrogen.

To date, fourteen different 17β-HSDs have been identified (Lukacik, P. et al., Mol. Cell. Endocrinol. 248:61-71 (2006); Luu-The, V. et al., Best Pract. Res. Clin. Endo-crinol. Metab., 22:207-221 (2008)) and twelve different subtypes have been cloned from human tissues. They all belong to the Short-Chain Dehydrogenase/Reductase family (except type 5). The 17β-HSDs show little amino acid identity (20-30%) and are membrane-bound or soluble enzymes. The X-ray structures of six human subtypes (type 1, 4, 8, 10, 11, 14) are known (Moeller, G. et al., Mol. Cell. Endocrinol. 301:7-19 (2009)). All of them are NAD(H)- or NADP(H)-dependent. The 17β-HSDs play therefore a key role in hormonal regulation and function in humans. They differ in tissue distribution, catalytic preference (oxidation or reduction), substrate

specificity and sub-cellular localisation. It is likely that all 17β-HSDs, modulating estrogen and androgen action, are expressed in the different estrogen/androgen-dependent tissues but certainly at different concentrations. In diseased tissues, the ratio between the different subtypes is changed compared to healthy tissues: one enzyme subtype might be overexpressed or completely absent, leading to higher/lower concentrations of the specific steroids. Selective inhibition of one subtype could therefore be a good strategy to influence the level in estrogen and androgen in hormone sensitive diseases.

17β-HSD2 (EC1.1.1.51) (Wu, L. et al., J. Biol. Chem., 268:12964-12969 (1993); Lu, M., J. Biol. Chem., 277:22123-22130 (2002)) is a trans-membrane protein localized in the endoplasmic reticulum. Its 3D-structure remains unknown. 17β-HSD2 shows a broad tissue distribution in adult: it is expressed in liver, small intestine, endometrium, urinary tracts and bone osteoblastic (Dong, Y. et al., J. Bone Miner. Res., 13:1539-1546 (1998); Feix, M. et al., Mol. Cell. Endocrinol., 171:163-164 (2001); Eyre, L.J. et al., J. Bone Miner. Res., 13:996-1004 (1998)) and osteoclastic (van der Eerden, B.C.J. et al., J. Endocrinol., 180:457-467 (2004)) cells. It has a predominant oxidative activity on hydroxy groups at the position C17 of the steroids: it converts the highly active 17β-hydroxysteroid estrogen E2 as well as the 17β-hydroxysteroid androgens T and DHT into their inactive keto forms using the cofactor $NAD^+$. The broad tissue distribution together with the oxidative activity of 17β-HSD2 suggests that this enzyme may play an essential role in the inactivation of highly active estrogens and androgens, resulting in diminished sex hormone action in target tissues.

Only very few inhibitors of 17β-HSD2 have been described: Poirier et al. (Sam, K.M., J. Med. Chem., 38:4518-4528 (1995); Poirier, D. et al., Mol. Cell. Endocrinol., 171:119-128 (2001); Bydal, P. et al., Eur. J. Med. Chem., 44:632-644 (2009)) reported on the potent steroidal C17-spirolactone **1** (inhibitory activity of 70% at 1 μM and 40% at 100 nM). Cook et al. (Cook, J.H. et al., Tetrahedron Letters, 46:1525-1528 (2005); Gunn, D., Bioorg. Med. Chem. Lett., 15:3053-3057 (2005); Wood, J. et al., Bioorg. Med. Chem. Lett., 16:4965-4968 (2006)) found, using high through-put screening methods, the first new class of potent non-steroidal inhibitors of 17β-HSD2: the 4,5-disubstituted cis-pyrrolidines. The most active compound described is **2** ($IC_{50}$= 10 nM). It has been recently proven in monkeys, using an osteoporosis model for *in vivo* evaluation of the 17β-HSD2 inhibitor **3,** that inhibition of this enzyme helps to maintain a sufficient local level of E2 and T in bones when the level of circulating active sex steroid drops (Bagi, C.M. et al., J. Musculoskelet. Neuronal Interact., 8:267-280 (2008)).

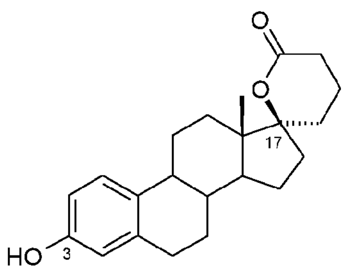

**1**

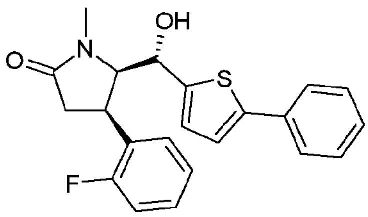

**2**

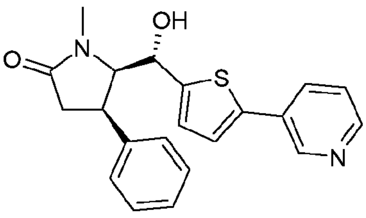

**3**

This modulation of steroid levels might be useful for a variety of indications like prevention and treatment of diseases caused by a misbalance of OB/OC activity like osteoporosis and osteopenia (Lindsay, R. et al., Obst. Gynecol., 76:290-295 (1990); Turner, R.T. et al., Endocr. Rev., 16:275-300 (1994); Meczekalski, B. et al., Gynecol. Endocrinol., 26:652-657 (2010)), post-menopausal symptoms, vaginal atrophy (Krychman, J.L. et al., J. Sex. Med., doi:10.1111/j.1743-6109.2010. 021148 (2010); Mac Bride, M.B., Mayo Clin. Proc., 85:87-94 (2010); Smith, A.L et al., Discov. Med., 10:500-510 (2010)); colon cancer (Oduwole O.O. et al., J. Steroid. Biochem. Mol. Biol. 87:133-140 (2003)), neuronal diseases (Behl, C. et al., Biochem. Biophys. Res. Commun., 216:473-482 (1995)) like Alzheimer (Pike, C.J., Front Neuroendocrinol., 30:239-258 (2009)) and Parkinson's disease (Bourque, M. et al., Front Neuroendocrinol., 30:142-157 (2009)), Depression (Schmidt, P.J. et al., Ann. N. Y. Acad. Sci., 179:70-85 (2009)) anxiety, hypercholesterolemia (Karjalainen, A. et al., Arterioscler. Thromb. Vase. Biol., 4:1101-1106 (2000)), cardiovascular diseases (Traish, A.M. et al., J. Androl., 30:477-494 (2009) ; Xing D. et al., Arterioscler. Thromb.Vasc. Biol. 29:289-295 (2009)); hair loss (Mooradian, A.D. et al., Endocr. Rev., 8:1-28 (1987); Georgala S et al., Dermatology 208:178-9 (2004)), rheumatic diseases and inflammatory diseases (Cutolo, M. et al., Ann. N. Y. Sci., 1193:36-42 (2010); Islander U. et al., Mol. Cell. Endocrinol. Doi:10.1016/j.mce. 2010.05.018 (2010)).

**Short Description of the Invention**

It was now found an improved series of new non-steroidal inhibitors which show, besides a high potency at the protein level (17β-HSD2: $IC_{50}$ < 100 nM, selectivity factor over 17β-HSD1 of up to > 100 and very weak binding affinity

to the estrogen receptors α and β: % RBA < 0.1).

(1) Described is a compound having 17beta-hydroxysteroid dehydrogenase type 2 (17β-HSD2) inhibitory activity of the formula (I)

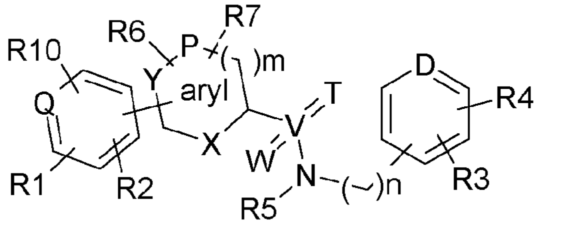

(I),

wherein

R1 and R3 are independently selected from H, -OH, alkoxy, acyloxy, alkyl, $-N(R8)_2$, -SR8, halogen, haloalkyl and phenyl;
R2, R4, R6 and R7 are independently selected from H, -OR8, -COR8, -COOR8, -CONHR8, -OCOR8, -SR8, $-SON(R8)_2$, $-SO_2N(R8)_2$, $-N(R8)_2$, -NHCOR8, -NHSOR8, $-NHSO_2R8$, -halogen, $-NO_2$, -CN, $-SO_2R8$, $-CH_2N(R8)_2$, $-CH_2OR8$, aryl optionally substituted with up to three R8, and alkyl optionally substituted with up to three R8; R5 is lower alkyl;
R8 is selected from H, alkyl optionally substituted with up to three R9, -OH, $-NO_2$ lower alkoxy, lower alkyl, halogen, haloalkyl, $-NH_2$, -CN, -NHCOR9, -CONHR9, $NHSO_2R9$, $-SO_2NHR9$, a 5- or 6-membered aromatic or heteroaromatic aryl ring optionally substituted with up to three R9, a homoaromatic ring that might be condensed with a 5- or 6-membered, aliphatic or aromatic heterocyclic ring and that (condensed) homoaromatic ring might optionally be substituted with up to three R9;
R9 is selected from -OH, alkoxy, halogen, haloalkyl, lower alkyl, $-NH_2$, $-NO_2$, -CN and phenyl;
R10 is selected from H, halogen, haloalkyl, lower alkyl;
m is an integer selected from 0 and 1;
n is an integer selected from 0 to 2;
the aryl ring is a 5-or 6-membered heteroaromatic ring which carries up to 3 heteroatoms X Y and P independently selected from N, S, O and said substituents R6 or R7 may be bound to heteroatoms or to ring carbon atoms;
X, Y and P are independently selected from CH, N, N(H), S and O;
Q and D are independently selected from CH and N;
V is C or S;
W is O or S; and
T, if V is S, represents O or is absent, or, if V is C, is absent;

or a salt thereof.
(2) In a preferred embodiment of the aspect (1) above, the compound has the formula (II)

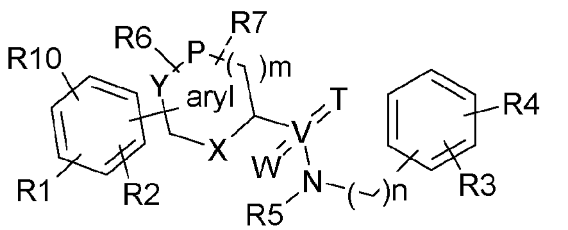

(II),

wherein the variables R1, R2, R3, R4, R5, R6, R7, R10, the aryl ring, n, m, X, Y, P, V, W and T have the same meaning as in (1) above, or a salt thereof.
(3) In a further preferred embodiment of the aspect (1) above, the compound has the formula (III)

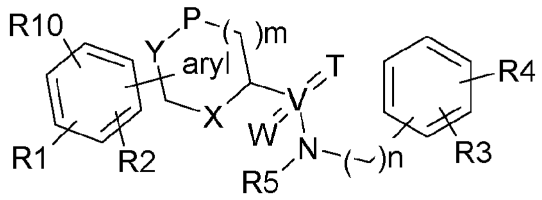

(III),

wherein the variables R1, R2, R3, R4, R5, R10, the aryl ring, n, m, X, Y, P, V, W and T have the same meaning as in (1) above, or a salt thereof.

(4) The invention provides a compound having 17beta-hydroxysteroid dehydrogenase type 2 (17f3-HSDE2) inhibitory activity of the formula (IV)

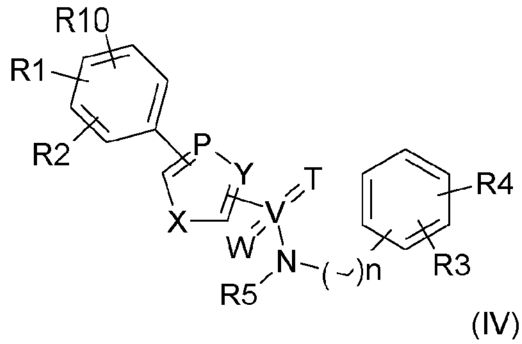

(IV)

wherein the variables R1, R2, R3, R4, R5, R10, n, X, Y, P, V, W and T have the same meaning as in claim 1, or a salt thereof. for use in the treatment and/or prophylaxis of sex steroid deficient-diseases. Further compounds and compounds for use according to the invention are the ones as claimed by the current set of claims. The embodiments disclosed in the description and which are not part of the claimed subject-matter, as the ones described under the aspects (5) to (14) below, are provided only as reference embodiments.

(5) In a preferred embodiment of the aspect (4) above, the compound has the formula (IVa)

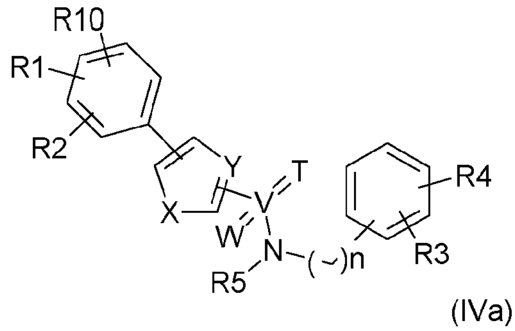

(IVa)

wherein the variables R1, R2, R3, R4, R5, R10, n, X, Y, V, W and T have the same meaning as in (1) above, or a salt thereof.

(6) In a preferred embodiment of the aspect (5) above, the compound has the formula (IVb)

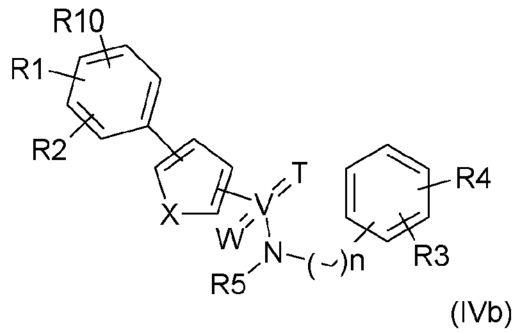

(IVb)

wherein the variables R1, R2, R3, R4, R5, RI0 n, X, V, W and T have the same meaning as in (1) above, or a salt thereof.

(7) In a further preferred embodiment of the aspect (1) above, the compound has the formula (Va) or (Vb)

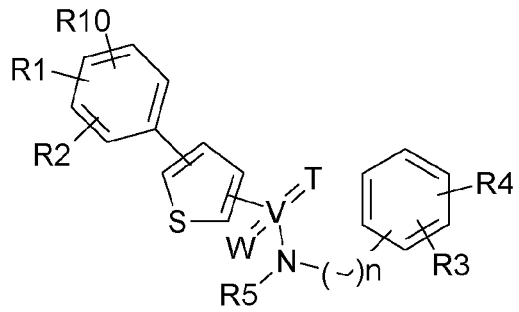

(Va)

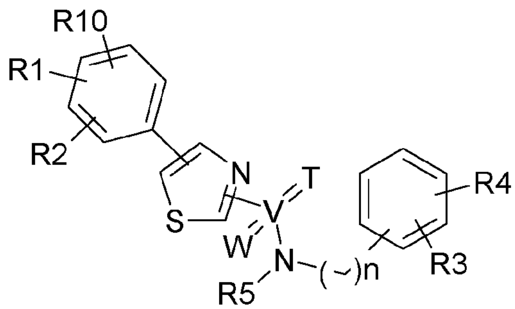

(Vb) ,

wherein the variables R1, R2, R3, R4, R5, R10, n, V, W and T have the same meaning as in (1) above, or a salt thereof.

(8) In a further preferred embodiment of the aspect (1) above, the compound has the formula (VIa) or (VIb)

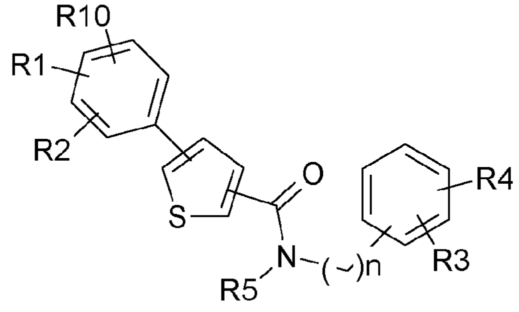

(VIa)

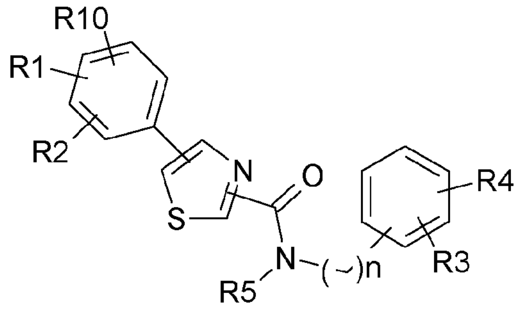

(VIb) ,

wherein the variables R1, R2, R3, R4, R5, R10, and n have the same meaning as in (1) above, or a salt thereof.

(9) In a further preferred embodiment of the aspect (1) above, the compound has the formula (VIIa) or (VIIb)

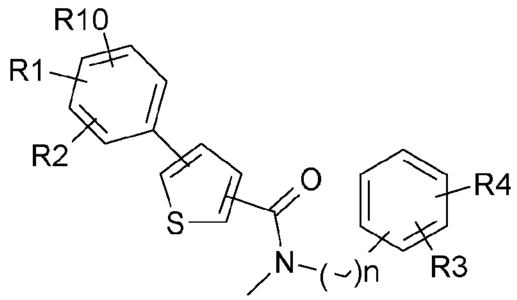

(VIIa)

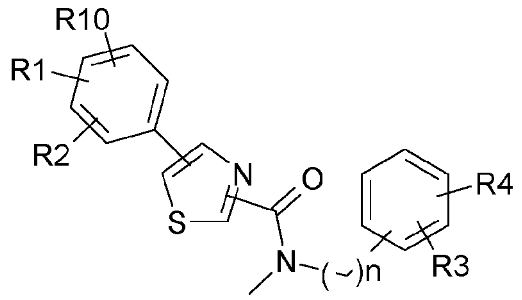

(VIIb) ,

wherein the variables R1 and R3 are independently selected from H, -OH, -OMe, -Me, $-NMe_2$ and phenyl; R2, R4 and R10 are independently selected from H and halogen; and n is 0 or 1; or a salt thereof.

(10) In still a further preferred embodiment of the aspect (1) above, the compound has the formula (VIIIa), (VIIIb) or (VIIIc)

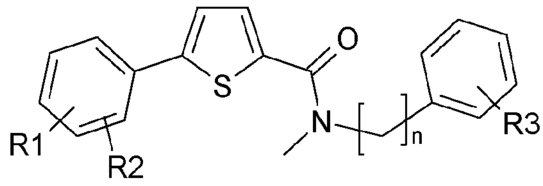

(VIIIa)

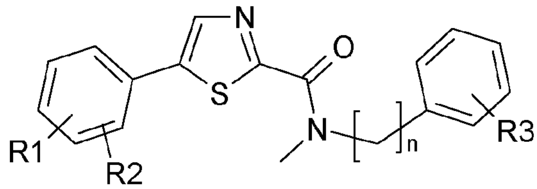

(VIIIb)

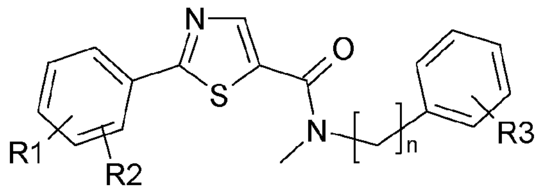

(VIIIc) ,

wherein R1 and R3 are independently selected from -OH, -$OCH_3$ and -$CH_3$; R2 is H, fluoro or methyl; and n is 0 or 1; or a salt thereof.

(11) A process for producing a compound formula (I) or a salt thereof according to any one of aspects (1) to (10) above, which comprises condensing an activated acyl or sulfonyl compound of the formula (B) with a secondary amine of the formula (C), followed by a cross-coupling reaction with a compound of the formula (A)

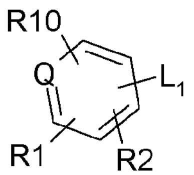

A

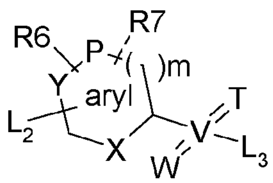

B

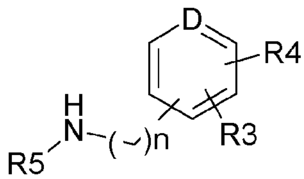

C

wherein all the variables are as defined in (1) above, and L1, L2 and L3 are leaving groups.

(12) A compound having 17β-HSD2 inhibitory activity according to any one of aspects (1) to (10) above or a pharmaceutically acceptable salt thereof for use in the treatment and/or prophylaxis of sex steroid deficient-diseases.

(13) A pharmaceutical composition or medicament comprising at least one of the claimed compounds or a pharmaceutically acceptable salt thereof, and optionally a suitable carrier or excipient.

(14) The use of a compound of aspects (1) to (10) above or a pharmaceutically acceptable salt thereof for preparing a medicament for the treatment and prophylaxis of osteoporotic diseases.

**Detailed Description of the Invention**

In the compounds of formula (I) the variables and the terms used for their characterization have the following meanings:

"Alkyl residues" and "alkoxy residues" within the meaning of the invention may be straight-chain, branched-chain or cyclic, and saturated or (partially) unsaturated. Preferable alkyl residues and alkoxy residues are saturated or have one or more double and/or triple bonds. Of straight-chain or branched-chain alkyl residues, preferred are those having from 1 to 6 carbon atoms, preferably those having from 1 to 4 carbon atoms. Of the cyclic alkyl residues, more preferred are mono- or bicyclic alkyl residues having from 3 to 6 carbon atoms.

"Lower alkyl" and "lower alkoxy" residues within the meaning of the invention are straight-chain, branched-chain or cyclic saturated lower alkyl residues and lower alkoxy residues or those having a double or triple bond. Of the straight-chain ones, those having from 1 to 6 carbons atoms, especially 1 to 3 carbon atoms, are particularly preferred.

"Aryls" and "homocyclic aromatic groups" within the meaning of the present invention include, if not specified otherwise, mono-, bi- and tricyclic aryl residues having from 3 to 18 ring atoms, which may optionally be anellated with one or more saturated rings. Particularly preferred are anthracenyl, dihydronaphthyl, fluorenyl, hydrindanyl, indanyl, indenyl, naphthyl, naphthenyl, phenanthrenyl, phenyl and tetralinyl. "Halogen" includes fluorine, chlorine, bromine and iodine.

The aryl in the formulas (I) to (III) include five- and six-membered arylene and heteroarylene residnes that are apparent to a skilled person, including phenyl, pyidin, pyrrol, furan, thiophen, imidazol, oxazol, thiazol, triazol etc.

"Salts" and "pharmaceutically acceptable salts" within the meaning of the present invention include salts of the compounds with organic acids (such as lactic acid, acetic acid, amino acid, oxalic acid, etc.), inorganic acids (such as HCl, HBr, phosphoric acid etc.), and, if the compounds have acid substituents, also with organic or inorganic bases including amino acids. Preferred are salts with HCl.

"Pharmacologically suitable carriers" within the meaning of the present invention are selected by the skilled person, depending on the desired dosage form.

In preferred embodiments of aspects (1) to (8) the variables, if present, have the following meaning:

R1 and R3 are independently selected from H, -OH, alkoxy, alkyl, $-N(R8)_2$, halogen, haloalkyl and phenyl;
R2, R4, R6 and R7 are independently selected from H, -OR8, -COR8, -COOP8, -CONHR8, -OCOR8, $-SO_2N(R8)_2$ $-N(R8)_2$, -NHCOR8, $-NHSO_2R8$, halogen, $-CH_2N(R8)_2$, $-CH_2OR8$, aryl optionally substituted with up to three R8, $C_{1-6}$ alkyl optionally substituted with up to three R8 and halo $C_{1-6}$ alkyl;
R5 is lower alkyl, preferably is methyl, isopropyl or cyclopropyl;
R8 is selected from H, $C_{1-6}$ alkyl optionally substituted with up to three R9, halogen, halo $C_{1-6}$ alkyl, -OH, lower alkoxy, $-NH_2$, and 5- or 6-membered aromatic or heteroaromatic ring optionally substituted with up to three R9, preferably is phenyl or benzyl;
R9 is selected from -OH, alkoxy, halogen, haloalkyl, $C_{1-4}$ alkyl, $-NH_2$ and phenyl; X, Y and P are independently selected from CH, N, N(H) and S; and/or
n is 0 or 1.

In the process of aspect (11) the leaving groups L1, L2 and L3 vary depending on the type of condensation reaction, but are selected to avoid any interference with each other (i.e. are "orthogonal" to allow the subsequent condensation reactions without cross reactions), or are ligands that can be converted into such orthogonal leaving groups. Preferably L1 is a $-B(OH)_2$ group, L2 is Br or OTf (trifluoromethanesulfonate) and L3 is Cl.

In a preferred embodiment of the inhibitor for use in the treatment and/or prophylaxis of sex steroid deficient-diseases of aspects (12) above, or in the pharmaceutical composition or medicament of aspects (13) and (14) above is for the treatment and prophylaxis of sex steroid deficient diseases including diseases caused by a misbalance of OB/OC activity like osteoporosis and osteopenia, bone repair (such as bone fracture repair, bone healing, fracture healing, bone grafting, healing of bone implants and the like) post-menopausal symptoms, vaginal atrophy, colon cancer, neuronal diseases like Alzheimer and Parkinson's disease, depression, anxiety, hypercholesterolemia, cardiovascular diseases, hair loss, rheumatic diseases and inflammatory diseases, preferably for the treatment and prophylaxis of osteoporosis.

Particular preferred compound of aspects (1) to (10) above and preferred compounds for use in the treatment and/or prophylaxis of sex steroid deficient-diseases of aspects (10) above are the following compounds (1) to (85):

3'-Methoxy-*N*-(3-methoxybenzyl)-*N*-methylbiphenyl-4-carboxamide (1), 3'-hydroxy-*N*-(3-hydroxybenzyl)-*N*-methylbiphenyl-4-carboxamide (2), *N*-(3-methoxy-benzyl)-*N*,3'-dimethylbiphenyl-4-carboxamide (3), *N*-(3-hydroxybenzyl)-*N*,3'-di-methylbiphenyl-4-carboxamide (4), 4'-Methoxy-*N*-(3-methoxybenzyl)-*N*-methylbi-phenyl-3-carboxamide (5), 4'-hydroxy-*N*-(3-hydroxybenzyl)-*N*-methylbiphenyl-3-carboxamide (6), 3'-methoxy-*N*-(3-methoxybenzyl)-*N*-methylbiphenyl-3-carbox-amide (7), 3'-hydroxy-*N*-(3-hydroxybenzyl)-*N*-methylbiphenyl-3-carboxamide (8), 4'-methoxy-*N*-(3-methoxyphenyl)-*N*-methylbiphenyl-3-carboxamide (9), 4'-hydroxy-*N*-(3-hydroxyphenyl)-*N*-methylbiphenyl-3-carboxamide (10), 3'-methoxy-*N*-(3-methoxyphenyl)-*N*-methylbiphenyl-3-carboxamide (11), 3'-hydroxy-*N*-(3-hy-droxyphenyl)-*N*-methylbiphenyl-3-carboxamide (12), 2'-methoxy-*N*-(3-methoxy-phenyl)-*N*-methylbiphenyl-3-carboxamide (13), 2'-hydroxy-*N*-(3-hydroxyphenyl)-N-methylbiphenyl-3-carboxamide (14), *N*-(3-methoxyphenyl)-*N*-methylbiphenyl-3-carboxamide (15), *N*-(3-hydroxyphenyl)-*N*-methylbiphenyl-3-carboxamide (16), *N*-(3-methoxyphenethyl)-5-(3-methoxyphenyl)-N-methylthiophene-2-carboxamide (17), *N*-(3-hydroxyphenethyl)-5-(3-hydroxyphenyl)-*N*-methylthiophene-2-carbox-amide (18), *N*-(3-methoxyphenethyl)-*N*-methyl-5-phenylthiophene-2-carboxamide (19), *N*-(3-hydroxyphenethyl)-*N*-methyl-5-phenylthiophene-2-carboxamide (20), 5-(3-fluorophenyl)-*N*-(3-methoxyphenethyl)-*N*-methylthiophene-2-carboxamide (21), 5-(3-fluorophenyl)-*N*-(3-hydroxyphenethyl)-*N*-methylthiophene-2-carboxamide (22), *N*-(3-methoxybenzyl)-5-(3-methoxyphenyl)-*N*-methylthiophene-2-carbox-amide (23), *N*-(3-hydroxybenzyl)-5-(3-hydroxyphenyl)-*N*-methylthiophene-2-carb-oxamide (24), *N*-(3-methoxybenzyl)-5-(4-methoxyphenyl)-N-methylthiophene-2-carboxamide (25), *N*-(3-hydroxybenzyl)-5-(4-hydroxyphenyl)-*N*-methylthiophene-2-carboxamide (26), *N*-(3-Methoxybenzyl)-5-(2-methoxyphenyl)-*N*-methylthio-phene-2-carboxamide (27), *N*-(3-hydroxybenzyl)-5-(2-hydroxyphenyl)-*N*-methyl-thiophene-2-carboxamide (28), *N*-(3-methoxybenzyl)-*N*-methyl-5-phenylthiophene-2-carboxamide (29), *N*-(3-hydroxybenzyl)-*N*-methyl-5-phenylthiophene-2-carbox-amide (30), 5-(4-cyanophenyl)-*N*-(3-methoxybenzyl)-*N*-methylthiophene-2-carbox-amide (31), 5-(4-cyanophenyl)-*N*-(3-hydroxybenzyl)-*N*-methylthiophene-2-carbox-amide (32), *N*-benzyl-5-(3-methoxyphenyl)-*N*-methylthiophene-2-carboxamide (33), *N*-benzyl-5-(3-hydroxyphenyl)-*N*-methylthiophene-2-carboxamide (34), *N*-benzyl-5-(4-methoxyphenyl)-*N*-methylthiophene-2-carboxamide (35), *N*-benzyl-5-(4-hydroxyphenyl)-*N*-methylthiophene-2-carboxamide (36), *N*-benzyl-5-(2-meth-oxyphenyl)-*N*-methylthiophene-2-carboxamide (37), *N*-benzyl-5-(2-hydroxy-phenyl)-*N*-methylthiophene-2-carboxamide (38), *N*-(3-hydroxybenzyl)-5-(4-meth-oxyphenyl)-*N*-methylthiophene-2-carboxamide (39), 5-(3-(dimethylamino)phenyl)-*N*-(3-hydroxybenzyl)-*N*-methylthiophene-2-carboxamide (40), 5-(3-fluorophenyl)-N-(3-hydroxybenzyl)-*N*-methylthiophene-2-carboxamide (41), 5-(4-fluorophenyl)-*N*-(3-hydroxybenzyl)-*N*-methylthiophene-2-carboxamide (42), 5-(2-fluoro-3-meth-oxyphenyl)-N-(3-hydroxybenzyl)-*N*-methylthiophene-2-carboxamide (43), 5-(2-fluoro-3-hydroxyphenyl)-*N*-(3-hydroxybenzyl)-*N*-methylthiophene-2-carboxamide (44), *N*-(3-hydroxybenzyl)-5-(3-methoxyphenyl)-*N*-methylthiophene-2-carbox-amide (45), *N*-(3-Hydroxybenzyl)-*N*-methyl-5-m-tolylthiophene-2-carboxamide (46), *N*,5-bis(3-methoxyphenyl)-*N*-methylthiophene-2-carboxamide (47), *N*,5-bis(3-hydroxyphenyl)-*N*-methylthiophene-2-carboxamide (48), 5-(2-methoxy-phenyl)-*N*-(3-methoxyphenyl)-*N*-methylthiophene-2-carboxamide (49), 5-(2-hydroxyphenyl)-*N*-(3-hydroxyphenyl)-*N*-methylthiophene-2-carboxamide (50), *N*-(3-methoxyphenyl)-*N*-methyl-5-phenylthiophene-2-carboxamide (51), *N*-(3-hydroxy-phenyl)-*N*-methyl-5-phenylthiophene-2-carboxamide (52), 5-(4-cyanophenyl)-*N*-(3-methoxyphenyl)-*N*-methylthiophene-2-carboxamide (53), 5-(4-cyanophenyl)-*N*-(3-hydroxyphenyl)-*N*-methylthiophene-2-carboxamide (54), *N*-(3-methoxyphenyl)-5-(4-methoxyphenyl)-*N*-methylthiophene-2-carboxamide (55), 5-(2-fluoro-3-methoxyphenyl)-*N*-(3-methoxyphenyl)-*N*-methylthiophene-2-carboxamide (56), 5-(2,4-dimethoxyphenyl)-*N*-(3-methoxyphenyl)-*N*-methylthiophene-2-carboxamide (57), 5-(3-(dimethylamino)phenyl)-*N*-(3-methoxyphenyl)-*N*-methylthiophene-2-carbox-amide (58), 5-(3-fluorophenyl)-*N*-(3-methoxyphenyl)-*N*-methylthiophene-2-carboxamide (59), 5-(3,4-difluorophenyl)-*N*-(3-methoxyphenyl)-*N*-methylthio-phene-2-carboxamide (60), 5-(3-fluoro-4-methoxyphenyl)-*N*-(3-methoxyphenyl)-*N*-methylthiophene-2-carboxamide (61), 5-(4-fluoro-3-methoxyphenyl)-*N*-(3-methoxyphenyl)-N-methylthiophene-2-carboxamide (62), *N*-(3-methoxyphenyl)-*N*-methyl-5-(3-(methylthio)phenyl)thiophene-2-carboxamide (63), *N*-(3-methoxy-phenyl)-*N*-methyl-5-m-tolylthiophene-2-carboxamide (64), 5-(2,6-difluoro-3-methoxyphenyl)-*N*-(3-methoxyphenyl)-N-methylthiophene-2-carboxamide (65), 5-(2-fluoro-3-methylphenyl)-N-(3-methoxyphenyl)-N-methylthiophene-2-carboxamide (66), 5-(3-methoxyphenyl)-*N*-methyl-*N*-phenylthiophene-2-carboxamide (67), 5-(3-hydroxyphenyl)-*N*-methyl-*N*-phenylthiophene-2-carboxamide (68), *N*-methyl-*N*,5-diphenylthiophene-2-carboxamide (69), 5-(3-methoxyphenyl)-*N*-methyl-*N*-m-tolylthiophene-2-carboxamide (70), 5-(3-hydroxyphenyl)-*N*-methyl-*N*-m-tolylthio-phene-2-carboxamide (71), 5-(2-fluoro-3-methoxyphenyl)-*N*-methyl-*N*-(m-tolyl-thiophene)-2-carboxamide (72), 5-(2-fluoro-3-methoxyphenyl)-*N*-(4-methoxy-phenyl)-*N*-methylthiophene-2-carboxamide (73), *N*-(2-fluorophenyl)-*N*-methyl-5-m-tolylthiophene-2-carboxamide (74), 5-(3-methoxyphenyl)-*N*-methyl-*N*-(3-(tri-fluoromethyl)phenyl)thiophene-2-carboxamide (75), *N*-(biphenyl-3-yl)-5-(3-meth-oxyphenyl)-*N*-methylthiophene-2-carboxamide (76), 5-(2-fluoro-3-methoxy-phenyl)-*N*-(3-methoxyphenyl)thiophene-2-carboxamide (77), 5-(2-fluoro-3-meth-oxyphenyl)-*N*-(3-methoxyphenyl)-N-phenylthiophene-2-carboxamide (78), N-(3-methoxyphenyl)-N-methyl-2-(3-methylphenyl)-1,3-thiazole-5-carboxamide (79), 2-(2-fluoro-3-methoxyphenyl)-N-(3-hydroxybenzyl)-N-methyl-1,3-thiazole-5-carbox-amide (80), N-(3-methoxyphenyl)-N-methyl-5-(3-methylphenyl)-1,3-thiazole-2-carboxamide (81), N-cyclopropyl-N-(3-methoxyphenyl)-5-(3-methylphenyl)thiophene-2-carboxamide (82), N-cyclopropyl-5-(2-fluoro-3-methoxyphenyl)-N-(3-hy-droxybenzyl)thiophene-2-carboxamide (83), N-(3-methoxyphenyl)-N-methyl-5-(3-methylphenyl)thiophene-2-sulfonamide (84) and 5-(2-fluoro-3-methoxyphenyl)-N-(3-hydroxybenzyl)-N-methylthiophene-2-sulfonamide (85), or a salt thereof.

Particularly preferred among compounds (1) to (85) are compounds (28), (30), (39), (43), (44), (46), (47), (55), (56), (58), (64), (65), (72), (76) and (81), or a salt thereof.

In the following the chemical structures of the particularly preferred inhibitors (1) to (78) (together with compounds (79)-(85) herein after also referred to as "title compounds") are shown.

Title compounds: 1-22:

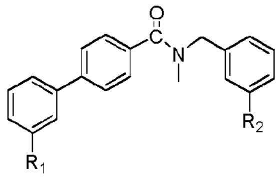

| cmpd | $R_1$ | $R_2$ |
|---|---|---|
| 1 | $OCH_3$ | $OCH_3$ |
| 2 | OH | OH |
| 3 | *m*-$CH_3$ | $OCH_3$ |
| 4 | *m*-$CH_3$ | OH |

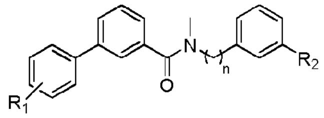

| cmpd | n | $R_1$ | $R_2$ |
|---|---|---|---|
| 5 | 1 | *p*-$OCH_3$ | $OCH_3$ |
| 6 | 1 | p-OH | OH |
| 7 | 1 | *m*-$OCH_3$ | $OCH_3$ |
| 8 | 1 | m-OH | OH |
| 9 | 0 | *p*-$OCH_3$ | $OCH_3$ |
| 10 | 0 | p-OH | OH |
| 11 | 0 | *m*-$OCH_3$ | $OCH_3$ |
| 12 | 0 | m-OH | OH |
| 13 | 0 | *o*-$OCH_3$ | $OCH_3$ |
| 14 | 0 | *o*-OH | OH |
| 15 | 0 | - | $OCH_3$ |
| 16 | 0 | - | OH |

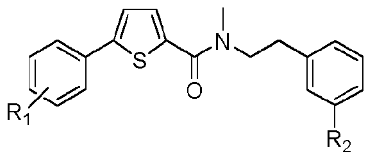

| cmpd | $R_1$ | $R_2$ |
|---|---|---|
| 17 | *m*-$OCH_3$ | $OCH_3$ |
| 18 | m-OH | OH |
| 19 | | $OCH_3$ |
| 20 | | OH |
| 21 | m-F | $OCH_3$ |
| 22 | m-F | OH |

Title compounds: 23-46:

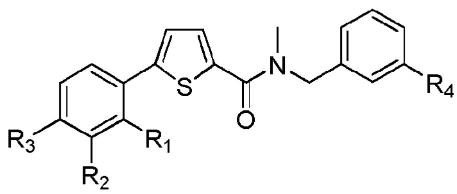

| cmpd | $R_1$ | $R_2$ | $R_3$ | $R_4$ |
|---|---|---|---|---|
| 23 | | $OCH_3$ | | $OCH_3$ |
| 24 | | OH | | OH |
| 25 | | | $OCH_3$ | $OCH_3$ |
| 26 | | | OH | OH |
| 27 | $OCH_3$ | | | $OCH_3$ |
| 28 | OH | | | OH |
| 29 | | | | $OCH_3$ |
| 30 | | | | OH |

(continued)

| cmpd | $R_1$ | $R_2$ | $R_3$ | $R_4$ |
|---|---|---|---|---|
| 31 | | | CN | $OCH_3$ |
| 32 | | | CN | OH |
| 33 | | $OCH_3$ | | |
| 34 | | OH | | |
| 35 | | | $OCH_3$ | |
| 36 | | | OH | |
| 37 | $OCH_3$ | | | |
| 38 | OH | | | |
| 39 | | | $OCH_3$ | OH |
| 40 | | $N(Me)_2$ | | OH |
| 41 | | F | | OH |
| 42 | | | F | OH |
| 43 | F | $OCH_3$ | | OH |
| 44 | F | OH | | OH |
| 45 | | $OCH_3$ | | OH |
| 46 | | $CH_3$ | | OH |

Title compounds: 47-78:

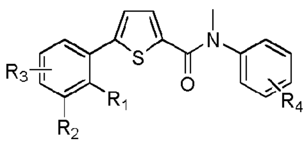

47-76

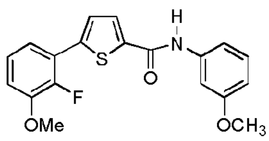

77

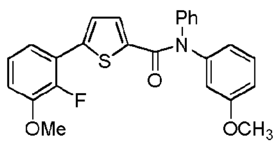

78

| cmpd | $R_1$ | $R_2$ | $R_3$ | $R_4$ |
|---|---|---|---|---|
| 47 | | $OCH_3$ | | *m*-$OCH_3$ |
| 48 | | OH | | m-OH |
| 49 | $OCH_3$ | | | *m*-$OCH_3$ |
| 50 | OH | | | *m*-OH |
| 51 | | | | *m*-$OCH_3$ |
| 52 | | | | *m*-OH |
| 53 | | | p-CN | *m*-$OCH_3$ |
| 54 | | | p-CN | *m*-OH |
| 55 | | | *p*-$OCH_3$ | *m*-$OCH_3$ |
| 56 | F | $OCH_3$ | | *m*-$OCH_3$ |
| 57 | $OCH_3$ | | *m*-$OCH_3$ | *m*-$OCH_3$ |
| 58 | | $N(CH_3)_2$ | | *m*-$OCH_3$ |
| 59 | | F | | *m*-$OCH_3$ |
| 60 | | F | *p*-F | *m*-$OCH_3$ |
| 61 | | F | *p*-$OCH_3$ | *m*-$OCH_3$ |
| 62 | | $OCH_3$ | p-F | *m*-$OCH_3$ |
| 63 | | $SCH_3$ | | *m*-$OCH_3$ |
| 64 | | $CH_3$ | | *m*-$OCH_3$ |
| 65 | F | $OCH_3$ | *o*-F | *m*-$OCH_3$ |
| 66 | F | $CH_3$ | | *m*-$OCH_3$ |
| 67 | | $OCH_3$ | | |

(continued)

| cmpd | $R_1$ | $R_2$ | $R_3$ | $R_4$ |
|---|---|---|---|---|
| 68 | | OH | | |
| 69 | | | | |
| 70 | | $OCH_3$ | | *m*-CH3 |
| 71 | | OH | | *m*-$CH_3$ |
| 72 | F | $OCH_3$ | | *m*-$CH_3$ |
| 73 | F | $OCH_3$ | | *p*-$OCH_3$ |
| 74 | | $CH_3$ | | o-F |
| 75 | | $OCH_3$ | | *m*-$CF_3$ |
| 76 | | $OCH_3$ | | m-ph |

The synthesis of the compounds of aspects (1) to (10) above according to aspect (11) of the invention is performed according to the following schemes:

Scheme 1: Synthesis of compounds 1-4[a]

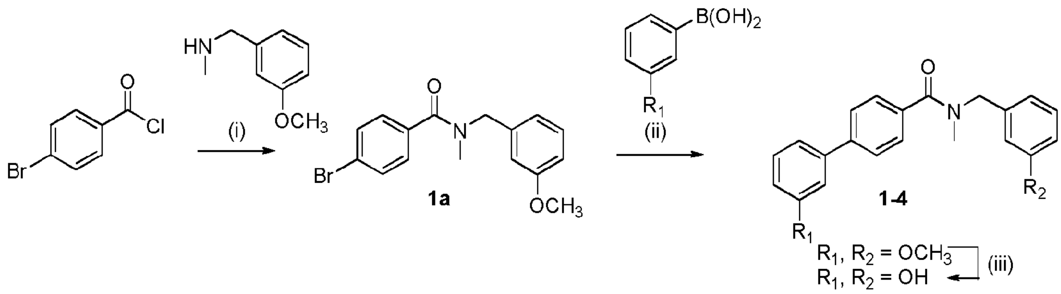


[a]Reagents and conditions: (i) Method A: $NEt_3$, $CH_2Cl_2$, rt, 3 h; (ii) Method B: DME (or To-luene), 2 N $Na_2CO_3$, $Pd(PPh_3)_4$, 80°C, 4-16h; (iii) Method C: $BF_3{\cdot}S(CH_3)_2$, $CH_2Cl_2$, rt, 3-14 h.

Scheme 2: Synthesis of compounds 5-8[a]

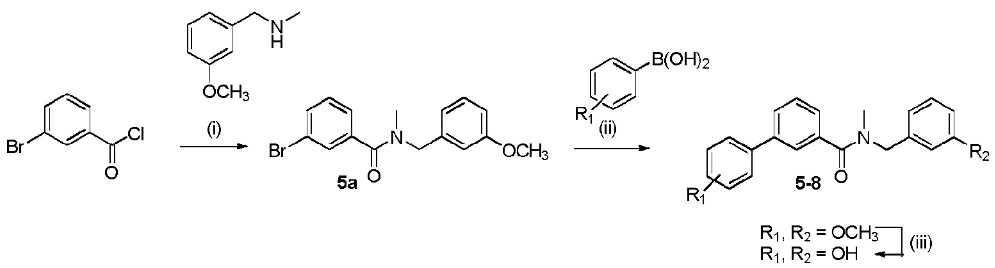


[a]Reagents and conditions: (i) Method A: $NEt_3$, $CH_2Cl_2$, rt, 3 h; (ii) Method B: DME (or To-luene), 2 N $Na_2CO_3$, $Pd(PPh_3)_4$, 80 °C 4-16 h; (iii) Method C: $BF_3.S(CH_3)_2$, $CH_2Cl_2$, rt, 3-14 h.

Scheme 3: Synthesis of compounds 9-16[a]

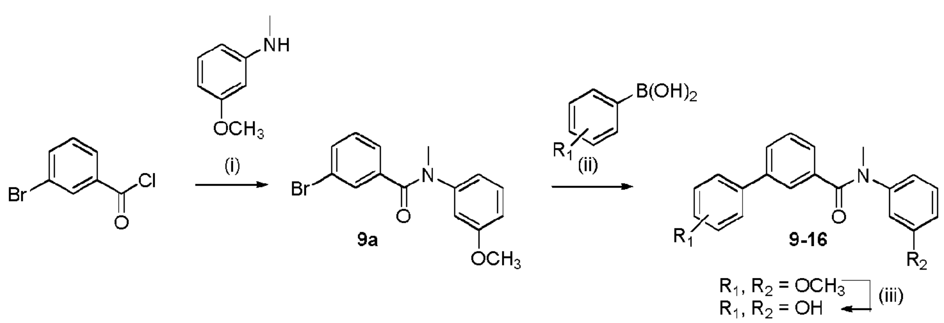

[a]Reagents and conditions: (i) Method A: $NEt_3$, $CH_2Cl_2$, rt, 3 h; (ii) Method B: DME (or Toluene), 2 N $Na_2CO_3$, $Pd(PPh_3)_4$, 80 °C 4-16 h; (iii) Method C: $BF_3.S(Me)_2$, $CH_2Cl_2$, rt, 3-14 h.

Scheme 4: Synthesis of compounds 17-22[a]

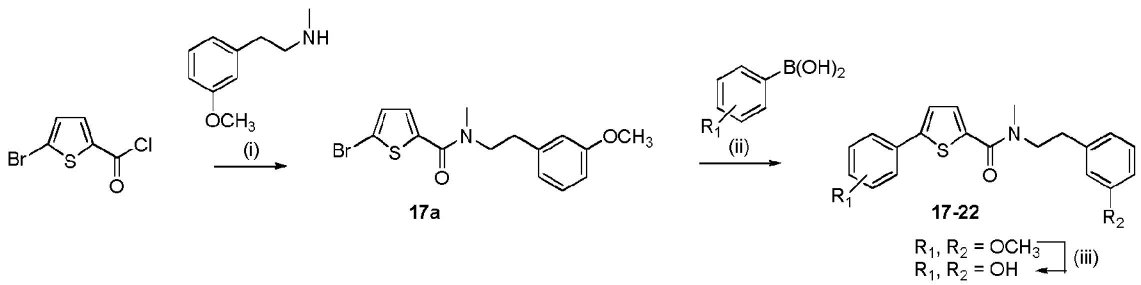


[a]Reagents and conditions: (i) Method A: $NEt_3$, $CH_2Cl_2$, rt, 3h; (ii) Method B: DME (or Toluene), 2 N $Na_2CO_3$, $Pd(PPh_3)_4$, 80 °C 4-16 h; (iii) Method C: $BF_3{\cdot}S(Me)_2$, $CH_2Cl_2$, rt, 3-14 h.

Scheme 5: Synthesis of compounds 23-46[a]

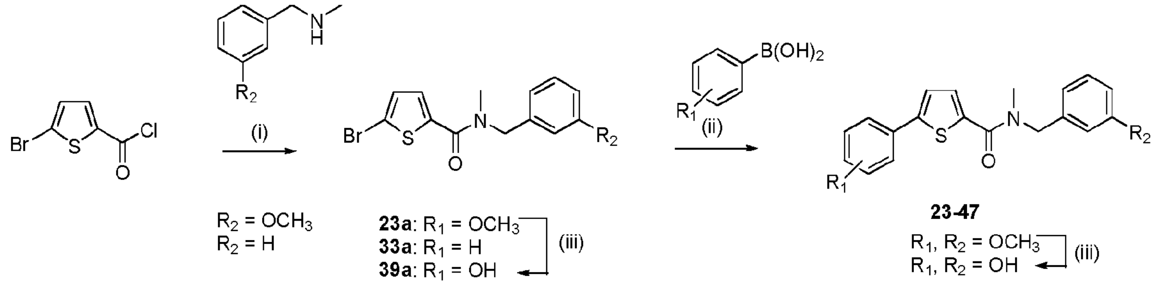


[a]Reagents and conditions: (i) Method A: $NEt_3$, $CH_2Cl_2$, rt, 3 h; (ii) Method B: DME (or Toluene), 2 N $Na_2CO_3$, $Pd(PPh_3)_4$, 80 °C 4-16 h; (iii) Method C: $BF_3{\cdot}S(Me)_2$, $CH_2Cl_2$, rt, 3-14 h.

Scheme 6: Synthesis of compounds 47-76[a]

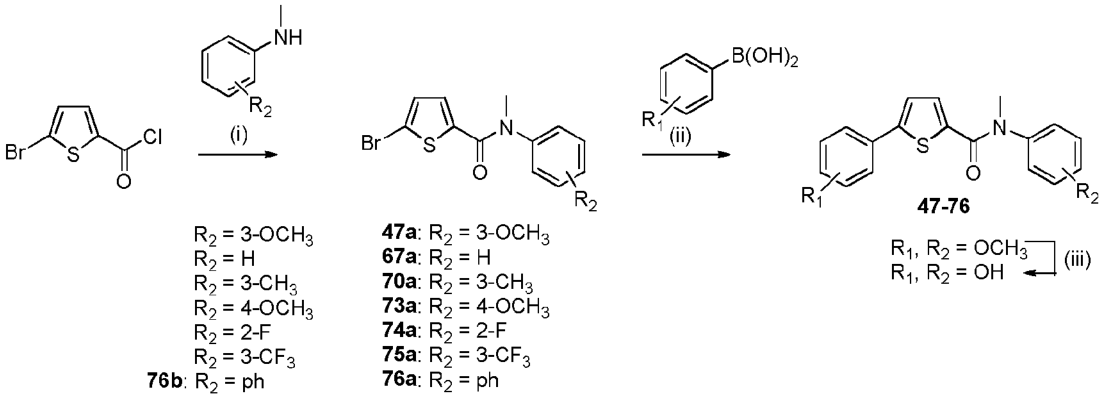


[a]Reagents and conditions: (i) Method A: $NEt_3$, $CH_2Cl_2$, rt, 3 h; (ii) Method B: DME (or Toluene), 2N $Na_2CO_3$, $Pd(PPh_3)_4$, 80 °C 4-16h; (iii) Method C: $BF_3{\cdot}S(Me)_2$, $CH_2Cl_2$, rt, 3-14 h.

Scheme 7: Synthesis of compounds 77 and 78[a]

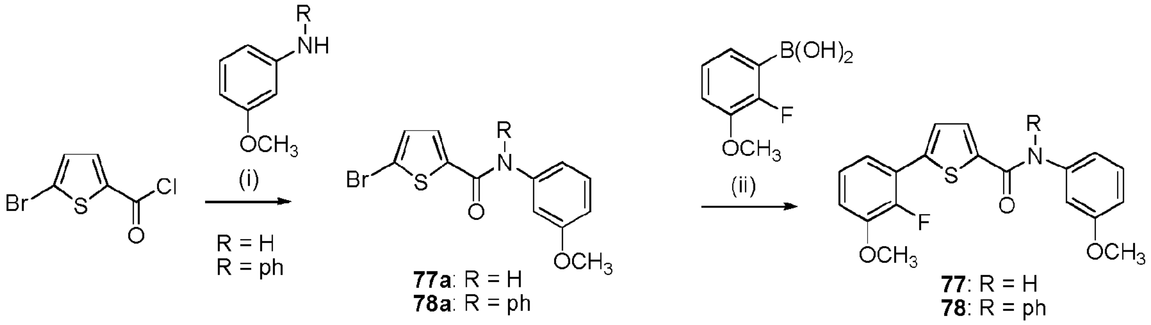


**77a**: R = H
**78a**: R = ph

**77**: R = H
**78**: R = ph

[a]Reagents and conditions: (i) Method A: $NEt_3$, $CH_2Cl_2$, rt, 3h; (ii) Method B: DME, 2N $Na_2CO_3$, $Pd(PPh_3)_4$, 80 °C, 16 h.

Scheme 8: Synthesis of compound 79[a]

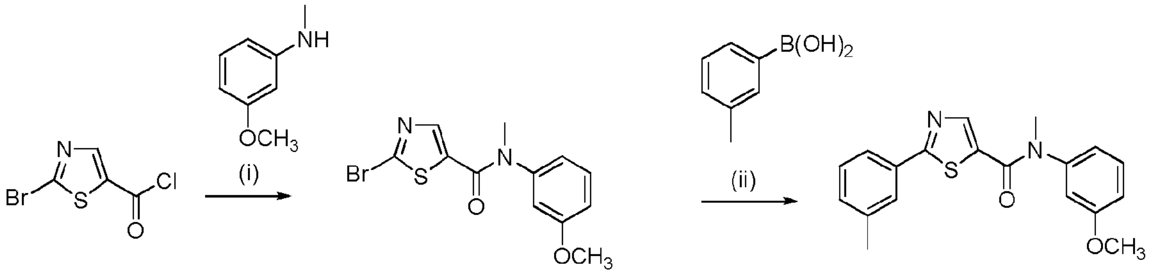


**79a**

**79**

[a]Reagents and conditions: (i) Method A: $NEt_3$, $CH_2Cl_2$, rt, overnight; (ii) DME/EtOH/$H_2O$ (1:1:1), $CS_2CO_3$, $Pd(PPh_3)_4$, microwave (150°C, 150 W, 5 bar, 15 min).

Scheme 9: Synthesis of compound 80[a]

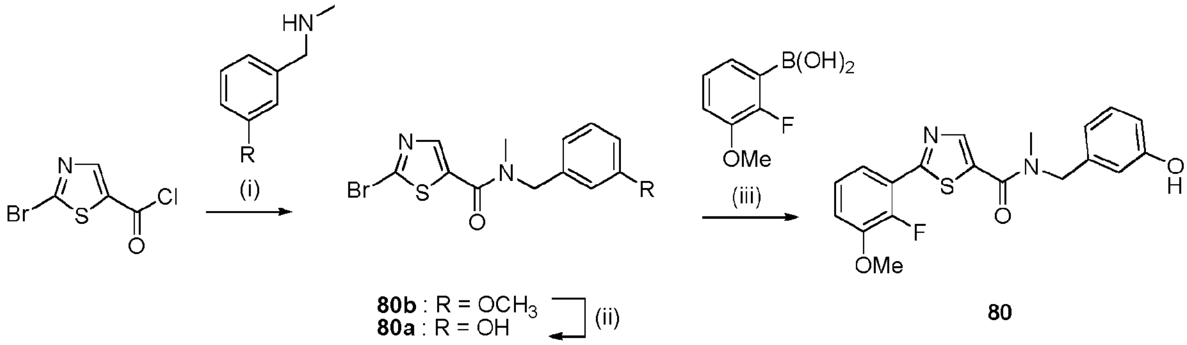


**80b** : R = $OCH_3$
**80a** : R = OH

**80**

[a]Reagents and conditions: (i) Method A: $NEt_3$, $CH_2Cl_{2/}$ rt, overnight; (ii) Method C: $BF_3 \cdot S(Me)_2$, $CH_2Cl_2$, rt, 3-14 h; (iii) DME/EtOH/$H_2O$ (1:1:1), $CS_2CO_3$, $Pd(PPh_3)_4$, microwave (150 °C, 150 W, 5 bar, 15 min).

Scheme 10: Synthesis of compound 81[a]

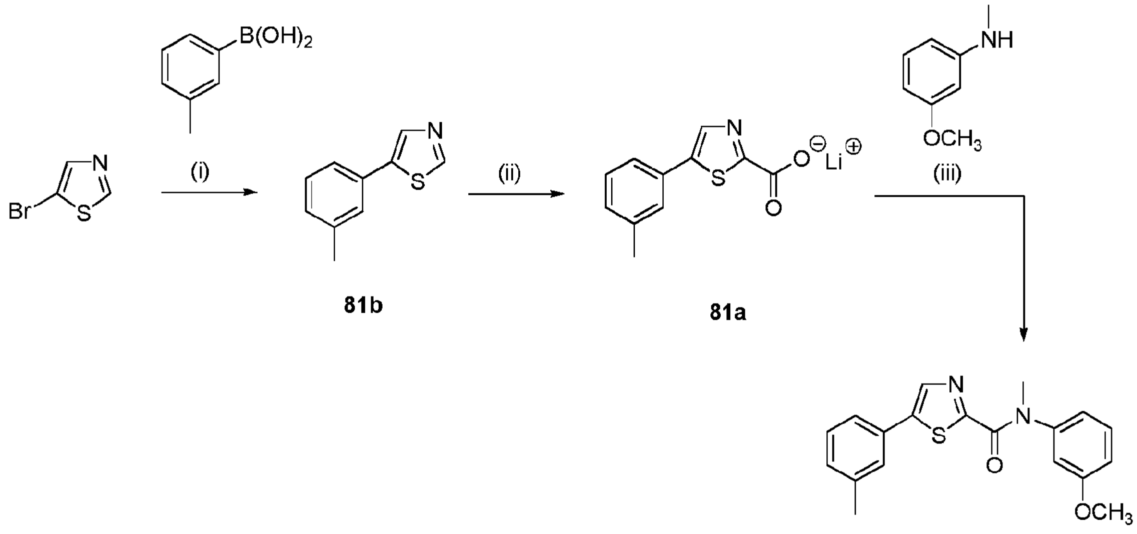


81

[a]Reagents and conditions: (i) DME/EtOH/$H_2O$ (1:1:1), $CS_2CO_3$, $Pd(PPh_3)_4$, microwave (150 °C, 150 W, 5 bar, 15 min); (ii) a) dry THF, *n*-BuLi, -78 °C to 0 °C, 4h, b) dry $CO_2$ gas, -78°C, 5h, rt, overnight ; (iii) a) oxalyl chloride, DMF cat., $CH_2Cl_2$, b) Method A: $NEt_3$, $CH_2Cl_2$, rt, overnight.

Scheme 11: Synthesis of compound 82[a]

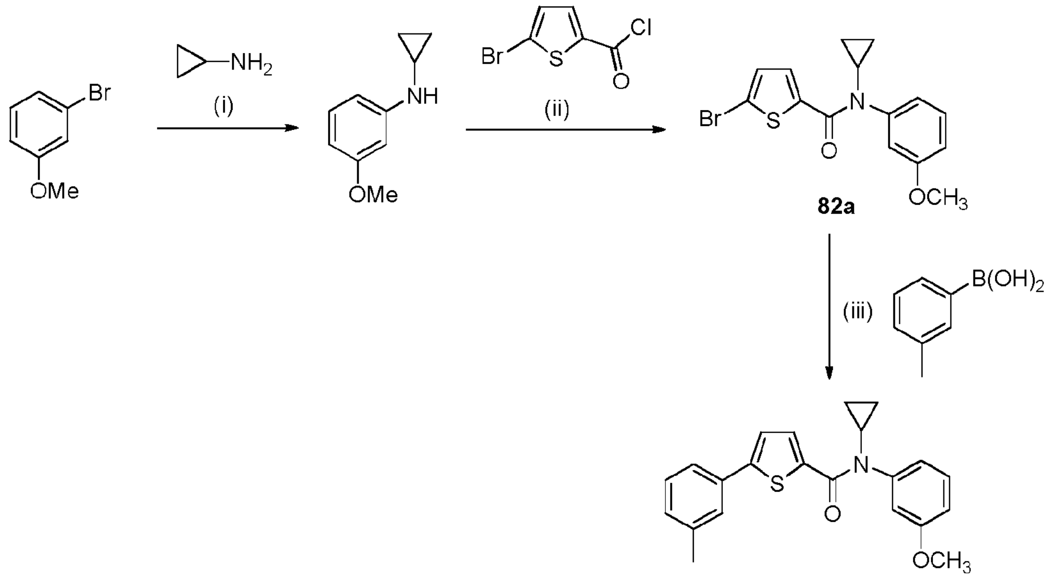


82

[a]Reagents and conditions: (i) BINAP, $NaO^tBu$, $Pd_2(dba)_3$, dry toluene, 80 °C, overnight; (ii) Method A: $NEt_3$, $CH_2Cl_2$, rt, overnight; (iii) DME/EtOH/$H_2O$ (1:1:1), $CS_2CO_3$, $Pd(PPh_3)_4$, microwave (150 °C, 150 W, 5 bar, 15 min).

Scheme 12: Synthesis of compound 83[a]

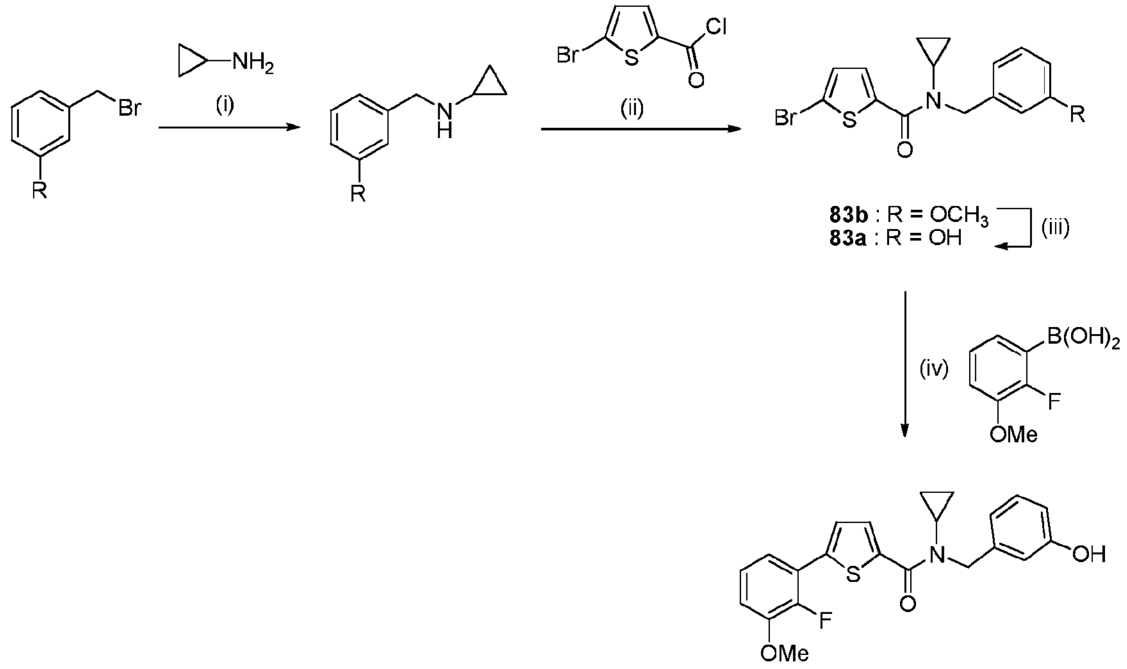


83

[a]Reagents and conditions: (i) BINAP, $NaO^tBu$, $Pd_2(dba)_3$, dry toluene, 80 °C, overnight; (ii) Method A: $NEt_3$, $CH_2Cl_2$, rt, overnight; (iii) Method C: $BF_3 \cdot S(Me)_2$, $CH_2Cl_2$, rt, overnight; (iv) DME/EtOH/$H_2O$ (1:1:1), $CS_2CO_3$, $Pd(PPh_3)_4$, microwave (150 °C, 150 W, 5 bar, 15 min).

Scheme 13: Synthesis of compounds 84[a]

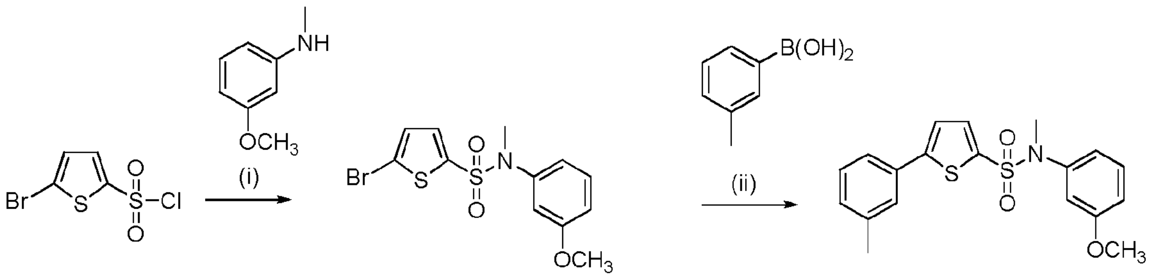


84a

84

[a]Reagents and conditions: (i) $Bu_4HSO_4$, NaOH 50%, $CH_2Cl_2$, rt, 5 h; (ii) DME/EtOH/$H_2O$ (1:1:1), $CS_2CO_3$, $Pd(PPh_3)_4$, microwave (150 °C, 150 W, 5 bar, 15 min).

Scheme 14: Synthesis of compounds 85[a]

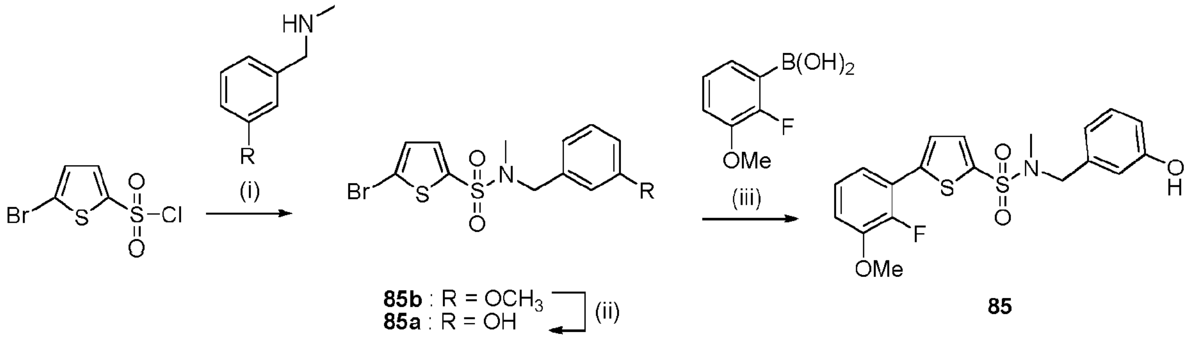


[a]Reagents and conditions: (i) $Bu_4HSO_4$, NaOH 50%, $CH_2Cl_2$, rt, 5 h; (ii) Method C: $BF_3 \cdot S(Me)_2$, $CH_2Cl_2$, rt, 3-14 h; (iii) DME/EtOH/$H_2O$ (1:1:1), $C_{S2}CO_3$, $Pd(PPh_3)_4$, microwave (150 °C, 150 W, 5 bar, 15 min).

The invention is described in more detail in the following examples, which are however not to be construed as limiting the invention.

## Examples

The examples which fall under the scope of the current set of claims represent embodiments of the invention. Al the other examples represent reference examples.

### Materials and Methods

Chemical names follow IUPAC nomenclature. Starting materials were purchased from Aldrich, Acros, Lancaster, Maybridge, Combi Blocks, Merck or Fluka and were used without purification.

Flash chromatography (CC) was performed on silica gel (70-200 μm) coated with silica, preparative thin layer chromatography (TLC) on 1 mm SIL G-100 $UV_{254}$ glass plates (Macherey-Nagel) and reaction progress was monitored by TLC on Alugram SIL G $UV_{254}$(Macherey-Nagel).

$^1$H-NMR spectra were measured on a Bruker AM500 spectrometer (500 MHz) at 300 K. Chemical shifts are reported in δ (parts per million: ppm), by reference to the hydrogenated residues of deuteriated solvent as internal standard ($CDCl_3$: δ= 7.24 ppm ($^1$H-NMR) and δ= 77 ppm ($^{13}$C-NMR), $CD_3OD$: δ= 5.84 ppm ($^1$H-NMR) and δ= 49.3 ppm ($^{13}$C-NMR), $CD_3COCD_3$: δ= 2.05 ppm ($^1$H-NMR) and δ= 30.8 ppm ($^{13}$C-NMR), $CD_3SOCD_3$ δ= 2.50 ppm ($^1$H-NMR) and δ= 39.5 ppm ($^{13}$C-NMR)). Signals are described as s, d, t, dd, ddd, m, dt, td and q for singlet, doublet, triplet, doublet of doublets, doublet of doublets of doublets, multiplet, doublet of triplets, triplet of doublets and quadruplets respectively. All coupling constants (J) are given in hertz (Hz).

Mass spectra (ESI) were recorded on a TSQ Quantum (Thermo Finnigan) instrument.

Tested compounds have > 95 % chemical purity as measured by HPLC. Several final compounds were purified via an Agilent Technologies Series 1200-preparative HPLC using a linear gradient run (solvents: acetonitrile, water) starting from 20% acetonitrile up to 100% over 36 min.

### Example 1. Preparation of title compounds 1-78.

Method A, general procedure for amidation: At 0 °C, a solution of bromoaryl carbonyl chloride (1 eq) in $CH_2Cl_2$ (2 ml/mol) was added drop wise to a solution of amine or *N*-substituted amine (1 eq) and triethylamine (1.15 eq) in $CH_2Cl_2$ (2 ml/mol). The mixture was kept stirring at 0 °C for 3 h and evaporated under reduced pressure. The residue was purified using flash chromatography (FC, *n*-hexane/ethyl acetate as eluent).

Method B, general procedure for Suzuki coupling: Arylbromide (1 eq), (substituted)aryl boronic acid (1 eq), sodium carbonate (2 eq) and tetrakis(triphenylphosphine) palladium (0.1 eq) in an oxygen free DME/water (1:1) solution was stirred at 80 °C for 4 to 16 hours under nitrogen. The reaction mixture was cooled to rt. The aqueous layer was extracted with dichloromethane. The combined organic layers were washed with brine, dried over sodium sulfate, filtered and concentrated to dryness. The product was purified by FC with hexane/ethyl acetate (20:1-3:1) as eluent.

Method C, general procedure for ether cleavage: To a solution of methoxyphenyl compounds (1 eq) in dry dichloromethane (5 ml/mmol reactant), borontrifluoride dimethyl sulfide complex (6 eq per methoxy function) was added drop wise at 0 °C and stirred for 3 to 14 h. After the reaction was finished, the reaction mixture was diluted with dichloromethane and $NaHCO_3$ (5% aqueous) was added to neutralize. The aqueous layer was extracted with dichloromethane.

The combined organic layers were washed with brine, dried over sodium sulfate, evaporated to dryness under reduced pressure. The product was purified by FC, with dichloromethane/methanol (100:1-50:1) or hexane/ethyl acetate (10:1-3:1) as eluent.

1.1. 3'-Methoxy-*N*-(3-methoxybenzyl)-N-methylbiphenyl-4-carboxamide (1). 4-Bromo-*N*-(3-methoxybenzyl)-*N*-methylbenzamide (1a).

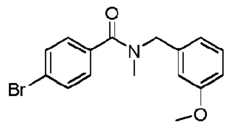

1a

The title compound was prepared by reaction of 4-bromobenzoylchloride (329 mg, 1.5 mmol) and 3-methoxybenzylamine (227 mg, 1.5 mmol) in presence of triethylamine (0.24 ml, 1.73 mmol) according to method A. Purification by FC (n-hexane/ ethyl acetate 6:1) afforded the desired product (468 mg, 93%); MS (ESI): 335 $(M+H)^+$; $^1H$ NMR ($CD_3COCD_3$): 2.80-2.92 (m, 3H), 3.80 (s, 3H), 4.52-4.69 (m, 2H), 6.79 (br s, 1H), 6.86 (dd, *J*= 8.5, 2.2 Hz, 1H), 6.95 (br s, 1H), 7.28 (t, J= 7.9 Hz, 1H), 7.44 (d, J= 7.9 Hz, 2H), 7.62 (br s, 2H); $^{13}C$ NMR ($CD_3COCD_3$): 55.3, 112.9, 113.6, 120.0, 123.1, 129.1, 129.4, 130.0, 131.7, 135.7, 139.1, 159.8.

3'-Methoxy-*N*-(3-methoxybenzyl)-*N*-methylbiphenyl-4-carboxamide (1).

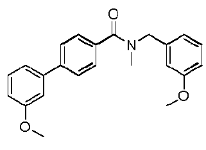

1

The title compound was prepared by reaction of 4-bromo-N-(3-methoxybenzyl)-N-methylbenzamide (1a) (67 mg, 0.2 mmol) and 3-methoxyphenylboronic acid (36 mg, 0.24 mmol) with tetrakis(triphenylphosphine) palladium (23 mg, 0.02 mmol) according to method B for 4 h. Purification by FC (*n*-hexane/ethyl acetate 6:1→3:1) afforded the desired compound (65 mg, 90%); MS (ESI): 362 $(M+H)^+$; $^1H$ NMR ($CD_3COCD_3$): 2.97 (s, 3H), 3.81 (s, 3H), 3.87 (s, 3H), 4.61-4.72 (m, 2H), 6.85 (dd, J= 8.2, 2.0 Hz, 2H), 6.96 (dd, *J*= 8.2, 2.0 Hz, 2H), 7.23-7.26 (m, 2H), 7.30 (t, *J*= 7.9 Hz, 1H), 7.38 (t, *J*= 8.0 Hz, 1H), 7.57 (d, J= 8.5 Hz, 2H), 7.72-7.73 (m, 2H); $^{13}C$ NMR ($CD_3COCD_3$): 55.5, 55.6, 113.3, 113.6, 114.2, 120.1, 127.7, 128.5, 130.6, 130.8, 136.8, 142.5, 142.8, 161.1, 161.2.

1.2. 3'-Hydroxy-*N*-(3-hydroxybenzyl)-*N*-methylbiphenyl-4-carboxamide (2).

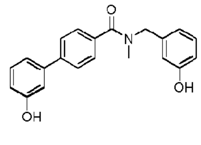

2

The title compound was prepared by reaction of 3'-methoxy-N-(3-methoxybenzyl)-N-methylbiphenyl-4-carboxamide (1) (36 mg, 0.1 mmol) with borontrifluoride dimethyl sulfide complex (0.13 ml, 1.2 mmol) according to method C for 14 h. Purification by FC ($CH_2Cl_2/CH_3OH$ 100:1→50:1) yielded the title compound (28 mg, 84%); MS: 334 $(M+H)^+$; $^1H$ NMR ($CD_3COCD_3$): 2.95 (s, 3H), 4.57-4.60 (m, 2H), 6.75 (dd, J= 8.2, 2.0 Hz, 2H), 6.85 (dd, J= 8.2, 2.0 Hz, 2H), 7.14-7.15 (m, 2H), 7.20 (t, *J*= 8.0 Hz, 1H), 7.29 (t, *J*= 8.0 Hz, 1H), 7.55 (d, *J*= 8.2 Hz, 2H), 7.67 (br s, 2H), 8.38 (br s, 1H), 8.48 (s, 1H); $^{13}C$ NMR ($CD_3COCD_3$): 114.7, 115.2, 115.7, 119.0, 127.6, 128.4, 130.4, 130.9, 136.7, 142.5, 142.9, 158.8, 158.9.

1.3. *N*-(3-Methoxybenzyl)-*N*,3'-dimethylbiphenyl-4-carboxamide (3).

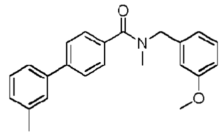

3

The title compound was prepared by reaction of 4-bromo-N-(3-methoxybenzyl)-N-methylbenzamide (1a) (53 mg, 0.15 mmol) and 3-methylphenylboronic acid (25 mg, 0.23 mmol) with tetrakis(triphenylphosphine) palladium (17 mg, 0.015 mmol) according to method B for 6 h. Purification by FC (*n*-hexane/ethyl acetate 6:1→3:1) afforded the desired compound (50 mg, 95%); MS (ESI): 346 $(M+H)^+$; $^1H$ NMR ($CD_3COCD_3$): 2.40 (s, 3H), 2.97 (s, 3H), 3.80 (s, 3H), 4.60-4.72 (m, 2H), 6.81-6.98 (m, 3H), 7.20 (d, J= 7.3 Hz, 1H), 7.30 (t, J= 8.0 Hz, 1H), 7.35 (t, J= 7.6 Hz, 1H), 7.47 (d, J= 7.6 Hz, 1H), 7.51 (s, 1H), 7.56 (d, J= 8.5 Hz, 2H), 7.71 (d, J= 7.3 Hz, 2H); $^{13}C$ NMR ($CD_3COCD_3$): 21.5, 55.5, 113.6, 124.9, 127.6, 128.5, 129.3, 129.7, 130.6, 136.6, 139.3, 141.0, 143.0, 161.1.

1.4. *N*-(3-hydroxybenzyl)-*N*,3'-dimethylbiphenyl-4-carboxamide (4).

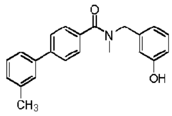

4

The title compound was prepared by reaction of *N*-(3-methoxybenzyl)-*N*,3'-dimethylbiphenyl-4-carboxamide (3) (35 mg, 0.1 mmol) with borontrifluoride dimethyl sulfide complex (0.06 ml, 0.6 mmol) according to method C for 6 h. purification by FC ($CH_2Cl_2/CH_3OH$ 100:1→50:1) yielded the title compound (30 mg, 89%); MS: 332 $(M+H)^+$; $^1H$ NMR ($CD_3COCD_3$): 2.40 (s, 3H), 2.96 (s, 3H), 4.55-4.70 (m, 2H), 6.69-6.89 (m, 3H), 7.20 (t, J= 7.9 Hz, 2H), 7.35 (t, J= 7.6 Hz, 1H), 7.47 (d, J= 7.9 Hz, 1H), 7.51 (s, 1H), 7.56 (d, J= 8.2 Hz, 2H), 7.70-7.71 (m, 2H), 8.36 (br s, 1H); $^{13}C$ NMR ($CD_3COCD_3$) : 21.5, 115.2, 124.9, 127.6, 128.5, 129.3, 129.7, 136.7, 139.3, 141.0, 143.0.

1.5. 4'-Methoxy-*N*-(3-methoxybenzyl)-*N*-methylbiphenyl-3-carboxamide (5). 3-Bromo-*N*-(3-methoxybenzyl)-*N*-methylbenzamide (5a).

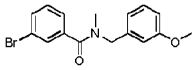

5a

The title compounds was prepared by reaction of 3-bromobenzoylchloride (217 mg, 1 mmol) and *N*-methyl-3-methoxybenzylamine (151 mg, 1 mmol) in presence of triethylamine (0.16 ml, 1.15 mmol) according to method A. The product was purified by FC (*n*-hexane/ethyl acetate 6:1), yielded 320 mg (98%) of the required compound; MS (ESI): 334 $(M+H)^+$; $^1H$ NMR ($CDCl_3$): 2.86-3.03 (m, 3H), 3.81 (s, 3H), 4.45-4.71 (m, 2H), 6.69-6.74 (m, 1H), 6.85 (d, *J*= 8.0 Hz, 1H), 6.89-6.92 (m, 1H), 7.24-7.26 (m, 1H), 7.28 (t, J= 7.9 Hz, 1H), 7.37 (d, *J*= 7.9 Hz, 1H), 7.53 (s, 1H), 7.60 (s, 1H); $^{13}C$ NMR ($CDCl_3$): 50.8, 55.2, 112.5, 112.9, 118.9, 120.5, 122.6, 125.2, 125.5, 129.7, 130.0, 132.7, 138.2.

4'-Methoxy-*N*-(3-methoxybenzyl)-*N*-methylbiphenyl-3-carboxamide (5).

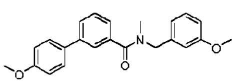

5

The title compound was prepared by reaction of 3-bromo-N-(3-methoxybenzyl)-N-methylbenzamide (5a) (67 mg, 2 mmol) and 4-methoxyphenylboronic acid (36 mg, 2.4 mmol) with tetrakis(triphenylphosphine) palladium (17 mg, 0.015 mmol) as catalyst according to method B for 4 h. Purification by FC (*n*-hexane/ethyl acetate 6:1) afforded the desired compound (70 mg, 97%); MS: 362 $(M+H)^+$; $^1H$ NMR ($CD_3COCD_3$): 2.97 (br s, 3H), 3.79 (s, 3H), 3.84 (s, 3H), 4.57-4.73 (m, 2H), 6.83-6.88 (m, 5H), 7.30 (t, *J*= 7.9 Hz, 1H), 7.40 (dt, *J*= 7.9, 1.3 Hz, 1H), 7.48-7.68 (m, 5H); $^{13}C$ NMR ($CD_3COCD_3$): 55.5, 55.6, 113.6, 114.5, 115.2, 125.7, 126.0, 128.1, 128.9, 129.7, 130.7, 133.4, 138.5, 141.6, 160.6.

1.6. 4'-Hydroxy-*N*-(3-hydroxybenzyl)-*N*-methylbiphenyl-3-carboxamide (6).

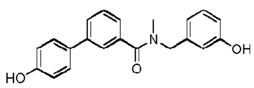

6

The title compound was prepared by reaction of 4'-methoxy-*N*-(3-methoxybenzyl)-N-methylbiphenyl-3-carboxamide (5) (40 mg, 0.11 mmol) with borontrifluoride dimethyl sulfide complex (0.14 ml, 1.32 mmol) according to method C for 4 h. Purification by FC ($CH_2Cl_2/CH_3OH$ 100:1→50:1) yielded the title compound (30 mg, 82%); MS: 334 $(M+H)^+$; $^1H$ NMR ($CD_3COCD_3$): 2.93-3.01 (m, 3H), 4.53-4.71 (m, 2H), 6.71-6.92 (m, 5H), 7.19 (t, *J*= 7.9 Hz, 1H), 7.36-7.51 (m, 4H), 7.63 (br s, 1H), 7.66 (s, 1H), 8.51-8.52 (m, 1H), 8.62 (br s, 1H); $^{13}C$ NMR ($CD_3COCD_3$): 115.2, 116.7, 125.5, 125.7, 128.1, 128.9, 129.7, 130.7, 132.2, 138.1, 139.9, 141.9, 158.3, 158.7.

1.7. 3'-Methoxy-*N*-(3-methoxybenzyl)-*N*-methylbiphenyl-3-carboxamide (7).

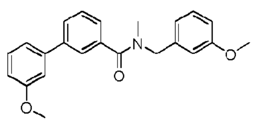

7

The title compound was prepared by reaction of 3-bromo-*N*-(3-methoxybenzyl)-N-methylbenzamide (5a) (67 mg, 0.2 mmol) and 3-methoxyphenylboronic acid (36 mg, 0.24 mmol) with tetrakis(triphenylphosphine) palladium (23 mg, 0.02 mmol) as catalyst according to method B for 4 h. Purification by FC (*n*-hexane/ethyl acetate 6:1) afforded the desired compound (71 mg, 98%); $^1H$ NMR ($CD_3COCD_3$): 2.98 (br s, 3H), 3.79 (s, 3H), 3.85 (s, 3H), 4.58-4.73 (m, 2H), 6.81 (br s, 1H), 6.86 (dd, *J*= 7.9, 2.2 Hz, 1H), 6.93-6.99 (m, 2H), 7.13 (br s, 1H), 7.22 (br s, 1H), 7.29 (t, *J*= 7.9 Hz, 1H), 7.36 (br s, 1H), 7.46 (dt, *J*= 7.6, 1.6 Hz, 1H), 7.52 (br s, 1H), 7.71 (br s, 1H), 7.73 (s, 1H); $^{13}C$ NMR ($CD_3COCD_3$): 54.9, 55.5, 55.6, 88.6, 113.2, 113.6, 114.2, 120.1, 126.2, 126.8, 128.7, 129.8, 130.5, 130.8, 138.5, 140.1, 141.8, 142.5, 161.1, 161.2.

1.8. 3'-Hydroxy-*N*-(3-hydroxybenzyl)-*N*-methylbiphenyl-3-carboxamide (8).

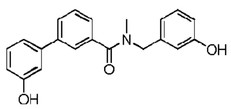

8

The title compound was prepared by reaction of 3'-methoxy-N-(3-methoxybenzyl)-N-methylbiphenyl-3-carboxamide (7) (54 mg, 0.15 mmol) with borontrifluoride dimethyl sulfide complex (0.19 ml, 1.8 mmol) according to method C for 5 h, purification by FC ($CH_2Cl_2/CH_3OH$ 100:1→50:1) yielded the title compound (46 mg, 92%); MS (ESI): 334 $(M+H)^+$ ; $^1H$ NMR ($CD_3COCD_3$): 2.96 (br s, 3H), 4.54-4.70 (m, 2H), 6.74-6.91 (m, 4H), 7.02-7.27 (m, 4H), 7.44 (d, *J*= 7.6 Hz, 1H), 7.51 (br s, 1H), 7.67 (br s, 1H), 7.69 (s, 1H), 8.37 (br s, 1H), 8.45 (s, 1H); $^{13}C$ NMR ($CD_3COCD_3$): 114.7, 115.2, 115.6, 119.0, 119.9, 126.2, 126.7, 128.6, 129.7, 130.9, 138.4, 139.9, 142.0, 142.5, 158.7, 158.8.

1.9. 4'-Methoxy-*N*-(3-methoxyphenyl)-*N*-methylbiphenyl-3-carboxamide (9). 3-Bromo-*N*-(3-methoxyphenyl)-*N*-methylbenzamide (9a).

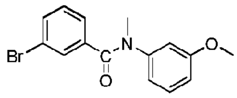

9a

The title compound was prepared by reaction of 3-bromobenzoylchloride (217 mg, 1 mmol) and 3-methoxy-*N*-methylaniline (137 mg, 1 mmol) in presence of triethylamine (0.16 ml, 1.15 mmol) according to method A. Purification by FC (*n*-hexane/ ethyl acetate 6:1) afforded the desired compound (300 mg, 94%); $^1$H NMR ($CD_3COCD_3$): 3.41 (s, 3H), 3.71 (s, 3H), 6.74-6.77 (m, 2H), 6.80 (t, *J*= 2.0 Hz, 1H), 7.15 (t, *J*= 8.0 Hz, 1H), 7.18 (t, *J*= 8.0 Hz, 1H), 7.27 (ddd, *J*= 7.9, 2.0, 0.9 Hz, 1H), 7.45 (ddd, *J*= 7.9, 2.0, 0.9 Hz, 1H), 7.52 (t, *J*= 2.0 Hz, 1H); $^{13}$C NMR ($CD_3COCD_3$): 38.2, 55.7, 113.4, 113.9, 120.2, 122.0, 128.0, 130.5, 130.7, 132.2, 133.0, 140.0, 146.8, 161.2, 168.8.

4'-Methoxy-*N*-(3-methoxyphenyl)-*N*-methylbiphenyl-3-carboxamide (9).

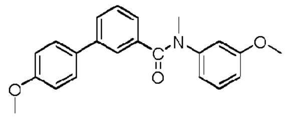

9

The title compound was prepared by reaction of 3-bromo-*N*-(3-methoxyphenyl)-*N*-methylbenzamide (9a) (80 mg, 0.25 mmol) and 4-methoxyphenylboronic acid (45 mg, 0.3 mmol) with tetrakis(triphenylphosphine) palladium (29 mg, 0.025 mmol) as catalyst according to method B for 4 h. Purification by FC (*n*-hexane/ethyl acetate 10:1→6:1) afforded the desired compound (82 mg, 94%); MS (ESI): 348 $(M+H)^+$; $^1$H NMR ($CD_3COCD_3$): 3.44 (s, 3H), 3.69 (s, 3H), 3.82 (s, 3H), 6.77 (ddd, *J*= 8.0, 2.5, 0.9 Hz, 1H), 6.79 (ddd, *J*= 8.0, 2.2, 0.9 Hz, 1H), 6.82 (t, *J*= 2.2 Hz, 1H), 6.97 (d, *J*= 8.8 Hz, 2H), 7.19 (t, *J*= 8.0 Hz, 1H), 7.26-7.31 (m, 2H), 7.38 (d, *J*= 8.8 Hz, 2H), 7.50 (dt, *J*= 7.3, 1.9 Hz, 1H), 7.52-7.53 (m, 1H); $^{13}$C NMR ($CD_3COCD_3$): 38.2, 55.6, 55.7, 113.1, 114.0, 115.1, 120.2, 127.6, 128.1, 128.7, 129.1, 130.6, 133.5, 138.2, 140.8, 147.5, 160.5, 161.2, 170.3.

1.10. 4'-Hydroxy-*N*-(3-hydroxyphenyl)-*N*-methylbiphenyl-3-carboxamide (10).

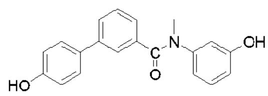

10

The title compound was prepared by reaction of 4'-methoxy-*N*-(3-methoxyphenyl)-N-methylbiphenyl-3-carboxamide (9) (35 mg, 0.1 mmol) with borontrifluoride dimethyl sulfide complex (0.13 ml, 1.2 mmol) according to method C for 8 h. Purification by FC ($CH_2Cl_2/CH_3OH$ 100:1→50:1) yielded the title compound (28 mg, 88%); $^1$H NMR ($CD_3COCD_3$): 3.42 (s, 3H), 6.67 (ddd, *J*= 6.9, 2.0, 0.9 Hz, 1H), 6.68-6.69 (m, 2H), 6.88 (d, *J*= 8.8 Hz, 2H), 7.09-7.12 (m, 1H), 7.24-7.29 (m, 2H), 7.31 (d, *J*= 8.8 Hz, 2H), 7.47 (dt, *J*= 6.9, 2.0 Hz, 1H), 7.52 (td, *J*= 1.6, 0.6 Hz, 1H), 8.50 (s, 1H), 8.55 (s, 1H); $^{13}$C NMR ($CD_3COCD_3$): 39.3, 115.5, 116.2, 117.6, 120.2, 128.4, 128.5, 129.1, 129.8, 129.9, 131.7, 133.4, 138.9, 142.1, 148.4, 159.2, 160.0, 171.4.

1.11. 3'-Methoxy-*N*-(3-methoxyphenyl)-*N*-methylbiphenyl-3-carboxamide (11).

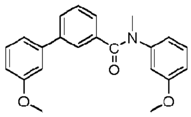 11

The title compound was prepared by reaction of 3-bromo-*N*-(3-methoxyphenyl)-N-methylbenzamide (9a) (80 mg, 0.25 mmol) and 3-methoxyphenylboronic acid (45 mg, 0.3 mmol) with tetrakis(triphenylphosphine) palladium (29 mg, 0.025 mmol) as catalyst according to method B for 4 h. Purification by FC (*n*-hexane/ethyl acetate 10:1→6:1) afforded the desired compound (85 mg, 98%); MS (ESI): 348 $(M+H)^+$; $^1H$ NMR ($CD_3COCD_3$): 3.45 (s, 3H), 3.70 (s, 3H), 3.83 (s, 3H), 6.76 (dd, *J*= 8.2, 2.5 Hz, 1H), 6.78 (dd, *J*= 7.9, 1.3 Hz, 1H), 6.83 (t, *J*= 2.5 Hz, 1H), 6.90 (ddd, *J*= 8.2, 2.5, 0.6 Hz, 1H), 6.92 (t, *J*= 2.1 Hz, 1H),7.01 (d, *J*= 7.8 Hz, 1H), 7.19 (t, *J*= 8.2 Hz, 1H), 7.30-7.34 (m, 2H), 7.39 (dt, *J*= 7.8, 1.3 Hz, 1H), 7.53-7.55 (m, 2H); $^{13}C$ NMR ($CD_3COCD_3$): 39.2, 56.6, 56.7, 114.0, 114.1, 114.9, 115.2, 121.0, 121.2, 129.1, 129.5, 129.7, 130.2, 131.6, 131.7, 139.2, 142.0, 143,6, 148.4, 162.1, 162.2, 171.2.

1.12. 3'-Hydroxy-*N*-(3-hydroxyphenyl)-*N*-methylbiphenyl-3-carboxamide (12).

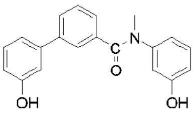 12

The title compound was prepared by reaction of 3'-methoxy-*N*-(3-methoxyphenyl)-N-methylbiphenyl-3-carboxamide (11) (75 mg, 0.22 mmol) with borontrifluoride dimethyl sulfide complex (0.28 ml, 2.64 mmol) according to method C for 8 h. Purification by FC ($CH_2Cl_2/CH_3OH$ 100:1→50:1) yielded the title compound as colorless solid (65 mg, 94%); MS (ESI): 320 $(M+H)^+$; $^1H$ NMR ($CD_3COCD_3$): 3.42 (s, 3H), 6.65-6.68 (m, 2H), 6.70 (ddd, *J*= 7.9, 2.5, 0.9 Hz, 1H), 6.82 (ddd, *J*= 7.9, 2.5, 0.9 Hz, 1H), 6.91 (ddd, *J*= 7.6, 2.0, 0.9 Hz, 1H), 6.97 (t, *J*= 2.0 Hz, 1H), 7.11 (td, *J*= 7.9, 0.9 Hz, 1H), 7.23 (t, *J*= 7.9 Hz, 1H), 7.29 (td, J= 7.6, 0.9 Hz, 1H), 7.32 (dt, *J*= 7.6, 1.6 Hz, 1H), 7.51 (ddd, J= 7.3, 1.9, 1.2 Hz, 1H), 7.57 (td, *J*= 1.9, 0.9 Hz, 1H), 8.36 (s, 1H), 8.45 (s, 1H); $^{13}C$ NMR ($CD_3COCD_3$): 39.3, 115.5, 115.6, 116.2, 116.4, 120.0, 120.2, 129.0, 129.3, 129.6, 130.0, 131.7, 139.2, 142.2, 143.6, 148.4, 159.7, 159.9, 171.2.

1.13. 2'-Methoy-*N*-(3-methoxyphenyl)-*N*-methylbiphenyl-3-carboxamide (13).

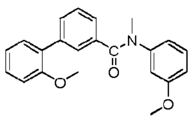 13

The title compound was prepared by reaction of 3-bromo-N-(3-methoxyphenyl)-N-methylbenzamide (9a) (80 mg, 0.25 mmol) and 2-methoxyphenylboronic acid (45 mg, 0.3 mmol) with tetrakis(triphenylphosphine) palladium (29 mg, 0.025 mmol) as catalyst according to method B for 12 h. Purification by FC (*n*-hexane/ethyl acetate 10:1→6:1) afforded the desired compound as colorless oil (79 mg, 91%); $^1H$ NMR ($CD_3COCD_3$): 3.44 (s, 3H), 3.69 (s, 3H), 3.75 (s, 3H), 6.74-6.78 (m, 3H), 6.96 (td, *J*= 7.4, 1.0 Hz, 1H), 7.02 (dd, *J*= 7.6, 1.9 Hz, 1H), 7.05 (dd, *J*= 8.0, 0.9 Hz, 1H), 7.17-7.21 (m, 1H), 7.24 (td, *J*= 8.0, 1.0 Hz, 1H), 7.28-7.32 (m, 2H), 7.43-7.45 (m, 2H); $^{13}C$ NMR ($CD_3COCD_3$): 38.2, 55.7, 55.9, 112.5, 113.0, 113.9, 120.0, 121.6, 127.7, 128.0, 129.8, 130.4, 130.6, 131.3, 131,4, 137.5, 139.0, 147.5, 157.4, 161.2, 170.5.

1.14. 2'-Hydroxy-*N*-(3-hvdroxyphenyl)-*N*-methylbiphenyl-3-carboxamide (14).

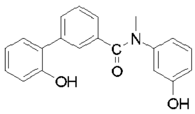 14

The title compound was prepared by reaction of 2'-methoxy-N-(3-methoxyphenyl)-N-methylbiphenyl-3-carboxamide (13) (60 mg, 0.17 mmol) with borontrifluoride dimethyl sulfide complex (0.21 ml, 2.04 mmol) according to method C for 14 h. Purification by FC ($CH_2Cl_2$/$CH_3OH$ 100:1→50:1) yielded the title compound as colorless solid (50 mg, 91%); MS (ESI): 320 $(M+H)^+$; $^1H$ NMR ($CD_3COCD_3$): 3.41 (s, 3H), 6.65-6.68 (m, 3H), 6.86 (td, *J*= 7.9, 1.3 Hz, 1H), 6.95 (dd, *J*= 7.9, 1.3 Hz, 1H), 7.03, (dd, *J*= 7.6, 1.6 Hz, 1H), 7.09 (td, *J*= 7.9, 0.6 Hz, 1H), 7.15 (ddd, *J*= 7.9, 7.0, 1.6 Hz, 1H), 7.24 (td, *J*= 7.6, 0.6 Hz, 1H), 7.29 (dt, *J*= 7.0, 1.6 Hz, 1H), 7.55-7.57 (m, 2H), 8.29, (s, 1H), 8.49 (s, 1H); $^{13}C$ NMR ($CD_3COCD_3$): 38.4, 114.4, 115.1, 117.0, 119.2, 120.8, 127.7, 128.0, 128.5, 129.6, 130.3, 130.7, 131.3, 131.4, 137.4, 139.0, 147.4, 155.0, 158.9, 170.5.

1.15. *N*-(3-Methoxyphenyl)-*N*-methylbiphenyl-3-carboxamide (15).

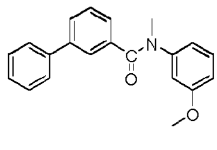

15

The title compound was prepared by reaction of 3-bromo-N-(3-methoxyphenyl)-N-methylbenzamide (9a) (80 mg, 0.25 mmol) and phenylboronic acid (36 mg, 0.3 mmol) with tetrakis(triphenylphosphine) palladium (29 mg, 0.025 mmol) as catalyst according to method B for 4 h. Purification by FC (*n*-hexane/ethyl acetate 10:1→6:1) afforded the desired compound as colorless oil (75 mg, 95%); MS (ESI): 318 $(M+H)^+$; $^1H$ NMR ($CD_3COCD_3$): 3.45 (s, 3H), 3.70 (s, 3H), 6.76 (ddd, J= 8.2, 2.5, 0.9 Hz, 1H), 6.80 (ddd, J= 7.9, 2.2, 0.9 Hz, 1H), 6.83 (t, J= 2.2 Hz, 1H), 7.20 (t, J= 8.2 Hz, 1H), 7.30-7.37 (m, 3H), 7.39-7.45 (m, 4H), 7.55 (dt, J= 7.6, 1.6 Hz, 1H), 7.57 (t, *J*= 1.6 Hz, 1H).

1.16. *N*-(3-Hydroxyphenyl)-*N*-methylbiphenyl-3-carboxamide (16).

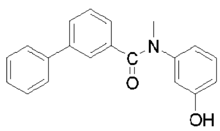

16

The title compound was prepared by reaction of *N*-(3-methoxyphenyl)-*N*-methyl biphenyl-3-carboxamide (15) (50 mg, 0.16 mmol) with borontrifluoride dimethyl sulfide complex (0.10 ml, 0.96 mmol) according to method C for 6 h. Purification by FC ($CH_2Cl_2$/$CH_3OH$ 100:1→50:1) yielded the title compound as colorless solid (40 mg, 84%); MS (ESI): 304 $(M+H)^+$; $^1H$ NMR ($CD_3COCD_3$): 3.43 (s, 3H), 6.66-6.70 (m, 3H), 7.11 (t, J= 8.2 Hz, 1H), 7.30-7.38 (m, 3H), 7.39-7.42 (m, 2H), 7.44-7.46 (m, 2H), 7.55 (dt, *J*= 7.6, 1.6 Hz, 1H), 7.58 (t, *J*= 1.6 Hz, 1H), 8.52 (s, 1H).

1.17. *N*-(3-Methoxyphenethyl)-5-(3-methoxyphenyl)-*N*-methylthiophene-2-carboxamide (17).

2-(3-Methoxyphenyl)-*N*-methylethanamine (17b).

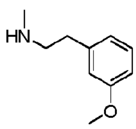

17b

To a solution of THF (0.2 ml) and methylamine (1.9 ml, 1.205 g, 22 mmol, 40% in water) at room temperature was added a solution of 3-methoxyphenethyl bromide (215 mg, 1 mmol) in THF (0.8 ml) over 20 min. The reaction mixture was kept stirring at rt overnight and evaporated. The residue was purified using FC (eluent :$CH_2Cl_2$/$CH_3OH$ (50:1-30:1)), and yielded the title compound as colorless solid (145 mg, 88%); MS (ESI): 166 $(M+H)^+$; $^1H$ NMR

($CD_3COCD_3$): 2.06-2.09 (m, 4H), 3.28 (t, J= 7.9 Hz, 2H), 3.38 (t, J= 7.9 Hz, 2H), 3.78 (s, 3H), 6.81 (dd, J= 2.4, 8.0 Hz, 1H), 6.90 (d, J= 7.6 Hz, 1H), 6.95 (s, 1H), 7.22 (t, J= 8.0 Hz, 1H); $^{13}C$ NMR ($CD_3COCD_3$): 32.7, 33.5, 51.1, 55.5, 113.4, 115.3, 121.8, 130.6, 139.6, 161.0.

5-Bromo-*N*-(3-methoxyphenethyl)-*N*-methylthiophene-2-carboxamide (17a).

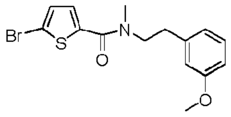

17a

The title compound was prepared from 5-bromothiophene-2-carbonyl chloride (171 mg, 0.76 mmol) and 2-(3-methoxyphenyl)-*N*-methylethanamine (17b) (125 mg, 0.76 mmol) in presence of triethylamine (0.12 ml, 1.11 mmol) according to method A. Purification by FC ($CH_2Cl_2$) yielded the desired compound as colorless oil (235 mg, 94%). The product was directly in the next step without further analysis.

*N*-(3-Methoxyphenethyl)-5-(3-methoxyphenyl)-*N*-methylthiophene-2-carboxamide (17).

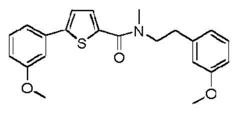

17

The title compound was prepared by reaction of 5-bromo-N-(3-methoxyphenethyl)-N-methylthiophene-2-carboxamide (17a) (78 mg, 0.22 mmol) and 3-methoxyphenylboronic acid (41 mg, 0.27 mmol) with tetrakis(triphenylphosphine) palladium (25 mg, 0.02 mmol) as catalyst according to method B for 6 h. Purification by FC (*n*-hexane/ethyl acetate 10:1→6:1) afforded the desired compound as colorless solid (65 mg, 78%); $^1H$ NMR ($CD_3COCD_3$): 2.96 (t, *J*= 7.5 Hz, 2H), 3.19 (br s, 3H), 3.76 (s, 3H), 3.79 (t, *J*= 7.5 Hz, 2H), 3.87 (s, 3H), 6.78 (ddd, *J*= 8.2, 2.5, 0.9 Hz, 1H), 6.84 (br s, 1H), 6.85 (br s, 1H), 6.94 (ddd, *J*= 8.2, 2.5, 0.9 Hz, 1H), 7.19-7.23 (m, 2H), 7.26 (ddd, *J*= 7.8, 1.5, 0.9 Hz, 1H), 7.32-7.37 (m, 2H), 7.41 (d, *J*= 3.8 Hz, 1H).

1.18. *N*-(3-Hydroxyphenethyl)-5-(3-hydroxyphenyl)-*N*-methylthiophene-2-carboxamide (18).

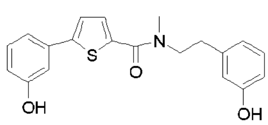

18

The title compound was prepared by reaction of N-(3-methoxyphenethyl)-5-(3-methoxyphenyl)-N-methylthiophene-2-carboxamide (17)(45 mg, 0.12 mmol) with borontrifluoride dimethyl sulfide complex (0.16 ml, 1.44 mmol) according to method C for 14 h. Purification by FC ($CH_2Cl_2/CH_3OH$ 100:1→50:1) yielded the title compound as colorless solid (35 mg, 84%); $^1H$ NMR ($CD_3COCD_3$): 2.91 (t, *J*= 7.6 Hz, 2H), 3.19 (br s, 3H), 3.76 (t, *J*= 7.6 Hz, 2H), 6.70 (ddd, *J*= 8.0, 2.5, 0.9 Hz, 1H), 6.73-6.76 (m, 2H), 6.84 (ddd, *J*= 8.0, 2.5, 0.9 Hz, 1H), 7.12 (t, *J*= 7.9 Hz, 1H), 7.15-7.18 (m, 2H), 7.26 (t, *J*= 7.9 Hz, 1H), 7.34 (s, 1H), 7.35 (s, 1H), 8.21 (s, 1H), 8.54 (s, lah); $^{13}C$ NMR ($CD_3COCD_3$): 113.4, 113.5, 114.2, 116.3, 118.0, 120.8, 123.8, 130.3, 131.1, 135.8, 158.5, 158.9.

1.19. *N*-(3-Methoxyphenethyl)-*N*-methyl-5-phenylthiophene-2-carboxamide (19).

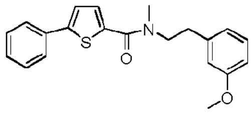

19

The title compound was prepared by reaction of 5-bromo-N-(3-methoxyphenethyl)-N-methylthiophene-2-carboxamide (17a) (75 mg, 0.21 mmol) and phenylboronic acid (31 mg, 0.25 mmol) with tetrakis(triphenylphosphine) palladium (24 mg, 0.02 mmol) as catalyst according to method B for 6 h. Purification by FC (*n*-hexane/ethyl acetate 10:1→6:1) afforded the desired compound as colorless solid (60 mg, 82%); $^1$H NMR ($CD_3COCD_3$): 2.96 (t, *J*= 7.6 Hz, 2H), 3.19 (br s, 3H), 3.76 (s, 3H), 3.79 (t, *J*= 7.6 Hz, 2H), 6.78 (ddd, *J*= 8.2, 2.5, 0.9 Hz, 1H), 6.84-6.85 (m, 2H), 7.21 (t, *J*= 8.8 Hz, 1H), 7.34-7.38 (m, 2H), 7.40 (d, *J*= 3.8 Hz, 1H), 7.42-7.46 (m, 2H), 7.69-7.71 (m, 2H); $^{13}$C NMR ($CD_3COCD_3$): 55.4, 112.8, 115.2, 121.9, 123.9, 126. 7, 129.2, 130.0, 130.3, 134.5, 160.9.

1.20. *N*-(3-Hydroxyphenethyl)-*N*-methyl-5-phenylthiophene-2-carboxamide (20).

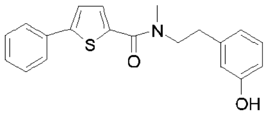

20

The title compound was prepared by reaction of N-(3-methoxyphenethyl)-N-methyl-5-phenylthiophene-2-carboxamide (19) (45 mg, 0.13 mmol) with borontrifluoride dimethyl sulfide complex (0.08 ml, 0.78 mmol) according to method C for 14 h. Purification by FC ($CH_2Cl_2$/$CH_3OH$ 100:1→50:1) yielded the title compound as colorless solid (38 mg, 88%); $^1$H NMR ($CD_3COCD_3$): 2.91 (t, J= 7.6 Hz, 2H), 3.19 (br s, 3H), 3.76 (t, *J*= 7.6 Hz, 2H), 6.70 (ddd, *J*= 8.0, 2.5, 0.9 Hz, 1H), 6.73-6.76 (m, 2H), 7.12 (t, *J*= 8.0 Hz, 1H), 7.34-7.38 (m, 2H), 7.40 (d, *J*= 3.8 Hz, 1H), 7.42-7.46 (m, 2H), 7.69-7.72 (m, 2H), 8.21 (s, 1H); $^{13}$C NMR ($CD_3COCD_3$): 114.2, 116.6, 120.8, 123.9, 126.7, 129.1, 130.0, 130.3, 134.5, 134.5, 158.5.

1.21. 5-(3-Fluorophenyl)-*N*-(3-methoxyphenethyl)-*N*-methylthiophene-2-carboxamide (21).

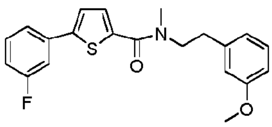

21

The title compound was prepared by reaction of 5-bromo-N-(3-methoxyphenethyl)-N-methylthiophene-2-carboxamide (17a) (71 mg, 0.2 mmol) and 3-fluorophenylboronic acid (34 mg, 0.24 mmol) with tetrakis(triphenylphosphine) palladium (23 mg, 0.02 mmol) as catalyst according to method B for 6 h. Purification by FC (*n*-hexane/ethyl acetate 10:1→6:1) afforded the desired compound as colorless solid (60 mg, 81%); MS (ESI): 370 $(M+H)^+$; $^1$H NMR ($CD_3COCD_3$): 2.96 (t, *J*= 7.5 Hz, 2H), 3.19 (br s, 3H), 3.76 (s, 3H), 3.79 (t, *J*= 7.5 Hz, 2H), 6.78 (ddd, *J*= 0.9, 2.5, 8.2 Hz, 1H), 6.83-6.85 (m, 2H), 7.13 (m, 1H), 7.21 (t, *J*= 8.2 Hz, 1H), 7.36 (br s, 1H), 7.46-7.50 (m, 3H), 7.53 (dt, *J*= 1.3, 7.8 Hz, 1 H); $^{13}$C NMR ($CD_3COCD_3$) : 55.4, 112.8, 113.1, 113.3, 115.3, 115.6, 115.8, 121.9, 122.7, 122.7, 124.9, 130.3, 131.9, 132.0, 136.9, 160.9, 165.1.

1.22. 5-(3-Fluorophenyl)-*N*-(3-hydroxyphenethyl)-*N*-methylthiophene-2-carboxamide (22).

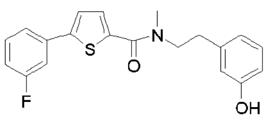

22

The title compound was prepared by reaction of 5-(3-fluorophenyl)-N-(3-methoxyphenethyl)-N-methylthiophene-2-carboxamide (21) (40 mg, 0.11 mmol) with borontrifluoride dimethyl sulfide complex (0.07 ml, 0.66 mmol) according to method C for 14 h. Purification by FC ($CH_2Cl_2/CH_3OH$ 100:1→50:1) yielded the title compound as colorless solid (30 mg, 78%); MS (ESI): 356 $(M+H)^+$; $^1H$ NMR ($CD_3COCD_3$): 2.91 (t, *J*= 7.5 Hz, 2H), 3.19 (br s, 3H), 3.76 (t, *J*= 7.5 Hz, 2H), 6.70 (ddd, *J*= 8.2, 2.5, 0.9 Hz, 1H), 6.73-6.76 (m, 2H), 7.10-7.15 (m, 2H), 7.36 (br s, 1H), 7.46-7.50 (m, 3H), 7.53 (dt, *J*= 7.8, 1.2 Hz, 1H), 8.21 (s, 1H); $^{13}C$ NMR ($CD_3COCD_3$): 113.1, 113.3, 114.2, 115.6, 115.8, 116.6, 120.71, 122.6, 122.7, 124.9, 130.3, 131.9, 132.0, 136.8, 158.6, 163.1, 165.0.

1.23. *N*-(3-Methoxybenzyl)-5-(3-methoxyphenyl)-*N*-methythiophene-2-carboxamide (23).

5-Bromo-*N*-(3-methoxybenzyl)-*N*-methylthiophene-2-carboxamide (23a).

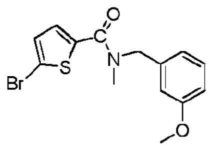

23a

The title compound was prepared by reaction of 5-bromothiophene-2-carbonyl chloride (225 mg, 1 mmol) and *N*-methyl-3-methoxybenzylamine (151 mg, 1 mmol) in presence of triethylamine (0.16 ml, 1.15 mmol) according to method A. The product was purified by FC (*n*-hexane/ethyl acetate 6:1), yielded (335 mg, 99%) as colorless oil; MS (ESI): 339 $(M+H)^+$; $^1H$ NMR ($CDCl_3$): 3.11 (br s, 3H), 3.80 (s, 3H), 4.72 (br s, 2H), 6.80 (s, 1H), 6.83-6.86 (dd, J= 8.0, 2.0 Hz, 2H), 6.96 (br s, 1H), 7.07 (br s, 1H), 7.29 (t, J= 8.0 Hz, 1H); $^{13}C$ NMR ($CDCl_3$): 55.2, 112.9, 117.2, 129.4, 129.8, 138.1, 139.5, 153.1, 160.1, 163.3.

*N*-(3-Methoxybenzyl)-5-(3-methoxyphenyl)-*N*-methylthiophene-2-carboxamide (23).

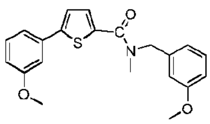

23

The title compound was prepared by reaction of 5-bromo-N-(3-methoxybenzyl)-N-methylthiophene-2-carboxamide (23a) (124 mg, 0.37 mmol) and 3-methoxyphenylboronic acid (67 mg, 0.44 mmol) with tetrakis(triphenylphosphine) palladium (43 mg, 0.04 mmol) as catalyst according to method B for 6 h. Purification by FC (*n*-hexane/ethyl acetate 6:1) afforded the desired compound as colorless solid (130 mg, 96%); MS (ESI): 368 $(M+H)^+$; $^1H$ NMR ($CD_3COCD_3$): 3.16 (br s, 3H), 3.80 (s, 3H), 3.86 (s, 3H), 4.79 (br s, 2H), 6.86-6.88 (m, 1H), 6.91-6.95 (m, 3H), 7.23 (t, J= 2.0 Hz, 1H), 7.25-7.28 (m, 1H), 7.30 (t, J= 8.0 Hz, 1H), 7.35 (t, J= 8.0 Hz, 1H), 7.41 (br s, 2H); $^{13}C$ NMR ($CD_3COCD_3$): 55.5, 55.7, 112.1, 113.5, 114.9, 119.1, 124.3, 130.6, 131.1, 135.7, 138.7, 140.1, 148.1, 161.1, 161.3.

1.24. *N*-(3-Hydroxybenzyl)-5-(3-hydroxyphenyl)-*N*-methylthiophene-2-carboxamide (24).

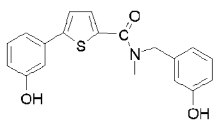

24

The title compound was prepared by reaction of N-(3-methoxybenzyl)-5-(3-methoxyphenyl)-N-methylthiophene-2-carboxamide (23) (70 mg, 0.19 mmol) with boron trifluoride dimethyl sulfide complex (0.24 ml, 2.28 mmol) according to method C for 4 h. Purification by FC ($CH_2Cl_2/CH_3OH$ 100:1→50:1) yielded the title compound as colorless

solid (59 mg, 91%); MS (ESI): 340 $(M+H)^+$; $^1H$ NMR ($CD_3COCD_3$): 3.15 (br s, 3H), 4.76 (br s, 2H), 6.77-6.79 (m, 1H), 6.80-6.83 (m, 2H), 6.85 (ddd, *J*= 8.0, 2.5, 0.9 Hz, 1H), 7.15-7.18 (m, 2H), 7.21 (t, *J*= 8.0 Hz, 1H), 7.26 (t, *J*= 7.9 Hz, 1H), 7.34 (d, *J*= 3.5 Hz, 1H), 7.39 (br s, 1H), 8.35 (s, 1H), 8.54 (s, 1H); $^{13}C$ NMR ($CD_3COCD_3$): 113.5, 115.2, 116.4, 118.0, 124.0, 130.7, 131.1, 135.7, 138.4, 140.0, 148.4, 158.8, 158.9.

1.25. *N*-(3-Methoxybenzyl)-5-(4-methoxyphenyl)-*N*-methylthiophene-2-carboxamide (25).

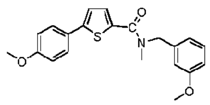

25

The title compound was prepared by reaction of 5-bromo-N-(3-methoxybenzyl)-N-methylthiophene-2-carboxamide (23a) (130 mg, 0.38 mmol) and 4-methoxyphenylboronic acid (70 mg, 0.46 mmol) with tetrakis(triphenylphosphine) palladium (44 mg, 0.04 mmol) as catalyst according to method B for 6 h. Purification by FC (*n*-hexane/ethyl acetate 6:1) afforded the desired compound as colorless solid (138 mg, 98%); MS(ESI): 368 $(M+H)^+$.; $^1H$ NMR ($CD_3COCD_3$): 3.17 (br s, 3H), 3.80 (s, 3H), 3.84 (s, 3H), 4.79 (br s, 2H), 6.87 (d, *J*= 8.0 Hz, 1H), 6.91-6.93 (m, 2H), 7.00 (d, *J*= 8.8 Hz, 2H), 7.27-7.32 (m, 2H), 7.38 (br s, 1H), 7.63 (d, *J*= 8.8 Hz, 2H); $^{13}C$ NMR ($CD_3COCD_3$): 55.5, 55.7, 113.5, 115.4, 122.9, 127.1, 128.1, 130.6, 137.6, 140.2, 148.5, 161.0, 161.1.

1.26. *N*-(3-Hydroxybenzyl)-5-(4-hydroxyphenyl)-*N*-methythiophene-2-carboxamide (26).

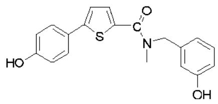

26

The title compound was prepared by reaction of N-(3-methoxybenzyl)-5-(4-methoxyphenyl)-N-methylthiophene-2-carboxamide (25) (100 mg, 0.27 mmol) with borontrifluoride dimethyl sulfide complex (0.34 ml, 3.24 mmol) according to method C for 4 h. Purification by FC ($CH_2Cl_2/CH_3OH$ 100:1→50:1) yielded the title compound as colorless solid (85 mg, 92%); MS (ESI): 340 $(M+H)^+$; $^1H$ NMR ($CD_3COCD_3$): 3.15 (br s, 3H), 4.75 (br s, 2H), 6.76-6.80 (m, 3H), 6.90 (d, *J*= 8.5 Hz, 2H), 7.19-7.23 (m, 2H), 7.36 (br s, 1H), 7.54 (d, *J*= 8.5 Hz, 2H), 8.40 (s, 1H), 8.70 (s, 1H); $^{13}C$ NMR ($CD_3COCD_3$): 115.2, 116.8, 122.5, 126.1, 128.2, 130.6, 137.1, 140.1, 149.0, 158.8, 158.9.

1.27. *N*-(3-Methoxybenzyl)-5-(2-methoxyphenyl)-*N*-methylthiophene-2-carboxamide (27).

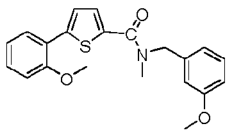

27

The title compound was prepared by reaction of 5-bromo-N-(3-methoxybenzyl)-N-methylthiophene-2-carboxamide (23a) (90 mg, 0.26 mmol) and 2-methoxyphenyl boronic acid (48 mg, 0.32 mmol) with tetrakis(triphenylphosphine) palladium (30 mg, 0.026 mmol) as catalyst according to method B for 4 h. Purification by FC (n-hexane/ethyl acetate 6:1) afforded the desired compound as colorless solid (92 mg, 93%); MS (ESI): 368 $(M+H)^+$; $^1H$ NMR ($CD_3COCD_3$): 3.16 (br s, 3H), 3.80 (s, 3H), 3.97 (s, 3H), 4.80 (br s, 2H), 6.87 (dd, *J*= 8.0, 2.0 Hz, 1H), 6.92-6.94 (m, 2H), 7.03 (td, *J*= 7.0, 1.0 Hz, 1H), 7.15 (dd, *J*= 8.0, 1.0 Hz, 1H), 7.29-7.36 (m, 2H), 7.39 (br s, 1H), 7.52 (d, *J*= 4.0 Hz, 1H), 7.76 (dd, *J*= 8.0, 1.5 Hz, 1H); $^{13}C$ NMR ($CD_3COCD_3$): 55.5, 56.0, 112.9, 113.5, 121.8, 123.1, 125.5, 128.8, 129.5, 130.2, 130.6, 132.0, 138.6, 140.3, 143.5, 156.7, 161.1.

1.28. *N*-(3-Hydroxybenzyl)-5-(2-hydroxyphenyl)-*N*-methylthiophene-2-carboxamide (28).

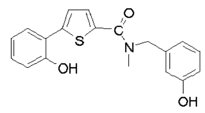

28

The title compound was prepared by reaction of N-(3-methoxybenzyl)-5-(2-methoxyphenyl)-N-methylthiophene-2-carboxamide (27) (48 mg, 0.13 mmol) with borontrifluoride dimethyl sulfide complex (0.16 ml, 1.56 mmol) according to method C for 14 h. Purification by FC ($CH_2Cl_2$/$CH_3OH$ 100:1→50:1) yielded the title compound as colorless solid (40 mg, 90%); MS (ESI): 340 $(M+H)^+$; $^1H$ NMR ($CD_3COCD_3$): 3.15 (br s, 3H), 4.76 (br s, 2H), 6.77 (dd, *J*= 8.0, 2.0 Hz, 1H), 6.81-6.84 (m, 2H), 6.92 (td, *J*= 7.6, 0.9 Hz, 1H), 7.04 (dd, *J*= 8.0, 0.9 Hz, 1H), 7.16-7.22 (m, 2H), 7.39 (br s, 1H), 7.55 (d, *J*= 3.4 Hz, 1H), 7.70 (dd, *J*= 8.0, 0.9 Hz, 1H), 8.40 (s, 1H), 9.34 (s, 1H); $^{13}C$ NMR ($CD_3COCD_3$): 115.2, 117.3, 120.9, 121.4, 125.3, 128.9, 130.0, 130.6, 138.1, 140.1, 144.5, 154.6, 158.8.

1.29. *N*-(3-Methoxybenzyl)-*N*-methyl-5-phenylthiophene-2-carboxamide (29).

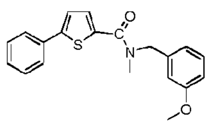

29

The title compound was prepared by reaction of 5-bromo-N-(3-methoxybenzyl)-N-methylthiophene-2-carboxamide (23a) (90 mg, 0.27 mmol) and phenylboronic acid (43 mg, 0.35 mmol) with tetrakis(triphenylphosphine) palladium (31 mg, 0.027 mmol) as catalyst according to method B for 14 h. Purification by FC (*n*-hexane/ ethyl acetate 6:1) afforded the desired compound as colorless solid (87 mg, 96%); MS (ESI): 338 $(M+H)^+$; $^1H$ NMR ($CD_3COCD_3$): 3.17 (br s, 3H), 3.80 (s, 3H), 4.80 (br s, 2H), 6.88 (ddd, *J*= 8.2, 2.5, 0.9 Hz, 1H), 6.92 (s, 1H), 6.93 (d, *J*= 7.9 Hz, 1H), 7.32 (t, *J*= 8.2 Hz, 1H), 7.36 (t, *J*= 7.6 Hz, 1H), 7.41-7.46 (m, 4H), 7.71 (d, *J*= 7.6 Hz, 2H); $^{13}C$ NMR ($CD_3COCD_3$): 55.5, 113.5, 124.1, 126.7, 129.2, 130.0, 130.6, 134.5, 140.1, 148.3, 161.1.

1.30. *N*-(3-Hdroxybenzyl)-*N*-methyl-5-phenylthiophene-2-carboxamide (30).

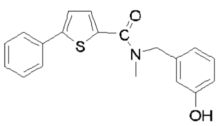

30

The title compound was prepared by reaction of N-(3-methoxybenzyl)-N-methyl-5-phenylthiophene-2-carboxamide (29) with borontrifluoride dimethyl sulfide complex (0.13 ml, 1.20 mmol) according to method C for 6 h. Purification by FC ($CH_2Cl_2$/ $CH_3OH$ 100:1→50:1) yielded the title compound as colorless solid (63 mg, 98%); MS (ESI): 346 ($M+CH_3CN$); $^1H$ NMR ($CD_3COCD_3$): 3.16 (br s, 3H), 4.76 (br s, 2H), 6.78 (ddd, *J*= 8.2, 2.5, 0.9 Hz, 1H), 6.81-6.83 (m, 2H), 7.22 (t, *J*= 7.8 Hz, 1H), 7.36 (tt, J= 8.2, 1.3 Hz, 1H), 7.40-7.46 (m, 4H), 7.71 (d, J= 7.6 Hz, 2H), 8.37 (s, 1H); $^{13}C$ NMR ($CD_3COCD_3$): 115.2, 124.0, 126.7, 129.2, 130.0, 130.7, 134.5, 138.7, 140.0, 148.3, 158.8.

1.31. 5-(4-Cyanophenyl)-*N*-(3-methoxybenzyl)-*N*-methylthiophene-2-carboxamide (31).

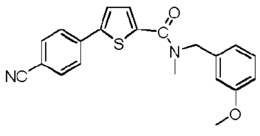

31

The title compound was prepared by reaction of 5-bromo-N-(3-methoxybenzyl)-N-methylthiophene-2-carboxamide (23a) (80 mg, 0.24 mmol) and 4-cyanophenylboronic acid (42 mg, 0.29 mmol) with tetrakis(triphenylphosphine) palladium (28 mg, 0.024 mmol) as catalyst according to method B for 14 h. Purification by FC (*n*-hexane/ethyl acetate 6:1) afforded the desired compound as colorless solid (80 mg, 92%); MS (ESI): 363 $(M+H)^+$; $^1H$ NMR ($CD_3COCD_3$): 3.18 (br s, 3H), 3.80 (s, 3H), 4.80 (br s, 2H), 6.87-6.89 (m, 1H), 6.91-6.93 (m, 2H), 7.31 (t, *J*= 7.9 Hz, 1H), 7.46 (s, 1H), 7.60 (d, *J*= 3.3 Hz, 1H), 7.84 (d, *J*= 8.8 Hz, 2H), 7.92 (d, *J*= 8.2 Hz, 2H); $^{13}C$ NMR ($CD_3COCD_3$): 55.5, 112.3, 113.6, 119.1, 126.3, 127.3, 130.7, 133.9, 138.7, 140.0, 145.7, 161.2.

1.32. 5-(4-Cyanophenyl)-*N*-(3-hydroxybenzyl)-*N*-methylthiophene-2-carboxamide (32).

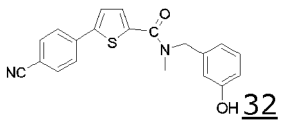

32

The title compound was prepared by reaction of 5-(4-cyanophenyl)-N-(3-methoxybenzyl)-N-methylthiophene-2-carboxamide (31) (60 mg, 0.17 mmol) with borontrifluoride dimethyl sulfide complex (0.11 ml, 1.02 mmol) according to method C for 14 h. Purification by FC ($CH_2Cl_2/CH_3OH$ 100:1$\rightarrow$50:1) yielded the title compound as yellowish solid (55 mg, 95%); MS (ESI): 349 $(M+H)^+$; $^1H$ NMR ($CD_3COCD_3$): 3.16 (br s, 3H), 4.76 (br s, 2H), 6.77-6.82 (m, 3H), 7.21 (t, *J*= 7.7 Hz, 1H), 7.46 (br s, 1H), 7.60 (d, *J*= 2.8 Hz, 1H), 7.83 (d, *J*= 8.5 Hz, 2H), 7.92 (d, *J*= 8.5 Hz, 2H), 8.37 (s, 1H); $^{13}C$ NMR ($CD_3COCD_3$): 112.3, 115.2, 115.3, 118.8, 119.1, 126.3, 127.3, 130.7, 133.9, 138.7, 139.9, 145.7, 158.8.

1.33. *N*-Benzyl-5-(3-methoxyphenyl)-*N*-methylthiophene-2-carboxamide (33). *N*-Benzyl-5-bromo-*N*-methylthiophene-2-carboxamide (33a).

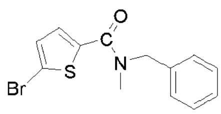

33a

The title compound was prepared by reaction of 5-bromothiophene-2-carbonyl chloride (225 mg, 1 mmol) and *N*-methylbenzylamine (121 mg, 1 mmol) in presence of triethylamine (0.16 ml, 1.15 mmol) according to method A. Purification by FC (n-hexane/ethyl acetate 6:1) afforded the desired compound as colorless oil (298 mg, 96%). MS (ESI): 311 $(M+H)^+$; $^1H$ NMR ($CD_3COCD_3$): 3.14 (br s, 3H), 4.78 (br s, 2H), 7.14 (d, J= 3.8 Hz, 1H), 7.25-7.40 (m, 6H); $^{13}C$ NMR ($CD_3COCD_3$): 117.1, 128.3, 129.6, 131.4, 138.2, 141.9.

*N*-Benzyl-5-(3-methoxyphenyl)-*N*-methylthiophene-2-carboxamide (33).

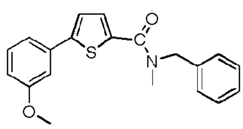

33

The title compound was prepared by reaction of by reaction of N-benzyl-5-bromo-N-methylthiophene-2-carboxamide (33a) (80 mg, 0.26 mmol) and 3-methoxyphenylboronic acid (47 mg, 0.31 mmol) with tetrakis(triphenylphosphine) palladium (30 mg, 0.026 mmol) as catalyst according to method B for 4 h. Purification by FC (*n*-hexane/ethyl acetate 6:1) afforded the desired compound as colorless solid (75 mg, 86%); MS (ESI): 338 $(M+H)^+$; $^1H$ NMR ($CD_3COCD_3$): 3.17 (br s, 3H), 3.86 (s, 3H), 4.82 (br s, 2H), 6.94 (ddd, J= 8.2, 2.5, 0.9 Hz, 1H), 7.23 (t, J= 2.0 Hz, 1H), 7.27 (ddd, J= 7.9, 1.6, 0.9 Hz, 1H), 7.29-7.32 (m, 1H), 7.34-7.41 (m, 7H); $^{13}C$ NMR ($CD_3COCD_3$): 55.7, 112.1, 114.9, 119.1, 124.3, 128.2, 129.6, 131.1, 135.7, 138.5, 148.1, 161.3.

1.34. *N*-Benzyl-5-(3-hydroxyphenyl)-*N*-methylthiophene-2-carboxamide (34).

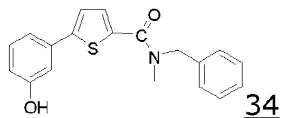

34

The title compound was prepared by reaction of N-benzyl-5-(3-methoxyphenyl)-N-methylthiophene-2-carboxamide (33) (65 mg, 0.19 mmol) with borontrifluoride dimethyl sulfide complex (0.12 ml, 1.14 mmol) according to method C for 4 h. Purification by FC ($CH_2Cl_2/CH_3OH$ 100:1) yielded the title compound as colorless solid (55 mg, 88%). MS (ESI): 324 $(M+H)^+$; $^1H$ NMR ($CD_3COCD_3$): 3.17 (br s, 3H), 4.82 (br s, 2H), 6.85 (ddd, J= 8.2, 2.2, 0.9 Hz, 1H), 7.16-7.18 (m, 2H), 7.26 (t, J= 7.8 Hz, 1H), 7.29-7.33 (m, 1H), 7.34-7.41 (m, 6H), 8.55 (s, 1H); $^{13}C$ NMR ($CD_3COCD_3$): 113.5, 116.4, 118.0, 124.0, 128.2, 129.6, 131.1, 135.7, 138.5, 148.4, 158.9.

1.35. *N*-Benzyl-5-(4-methoxyphenyl)-*N*-methylthiophene-2-carboxamide (35).

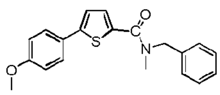

35

The title compound was prepared by reaction of N-benzyl-5-bromo-N-methylthiophene-2-carboxamide (33a) (72 mg, 0.23 mmol) and 4-methoxyphenylboronic acid (42 mg, 0.27 mmol) with tetrakis(triphenylphosphine) palladium (27 mg, 0.023 mmol) as catalyst according to method B for 5 h. Purification by FC (*n*-hexane/ethyl acetate 6:1) afforded the desired compound as colorless solid (75 mg, 96%); MS (ESI): 338 $(M+H)^+$; $^1H$ NMR ($CD_3COCD_3$): 3.16 (br s, 3H), 3.84 (s, 3H), 4.82 (br s, 2H), 7.00 (d, J= 8.8 Hz, 2H); 7.27 (d, J= 3.8 Hz, 1H), 7.29-7.32 (m, 1H), 7.35-7.41 (m, 5H), 7.63 (d, J= 8.8 Hz, 2H); $^{13}C$ NMR ($CD_3COCD_3$): 55.7, 115.4, 122.9, 127.1, 128.1, 128.2, 129.6, 137.6, 138.6, 148.5, 161.0.

1.36. *N*-Benzyl-5-(4-hydroxyphenyl)-*N*-methylthiophene-2-carboxamide (36).

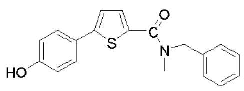

36

The title compound was prepared by reaction of N-benzyl-5-(4-methoxyphenyl)-N-methylthiophene-2-carboxamide (35) (56 mg, 0.17 mmol) with borontrifluoride dimethyl sulfide complex (0.11 ml, 1.02 mmol) according to method C for 6 h. Purification by FC ($CH_2Cl_2/CH_3OH$ 100:1) yielded the title compound as colorless solid (49 mg, 91%); MS: 324 $(M+H)^+$; $^1H$ NMR ($CD_3COCD_3$): 3.16 (br s, 3H), 4.82 (br s, 2H), 6.91 (d, J= 8.8 Hz, 2H), 7.23 (d, J= 3.8 Hz, 1H), 7.28-7.32 (m, 1H), 7.34-7.40 (m, 5H), 7.55 (d, J= 8.8 Hz, 2H), 8.64 (s, 1H); $^{13}C$ NMR ($CD_3COCD_3$): 116.8, 122.5, 126.1, 128.2, 129.6, 137.2, 138.6, 149.0, 158.9.

1.37. *N*-Benzyl-5-(2-methoxyphenyl)-*N*-methylthiophene-2-carboxamide (37).

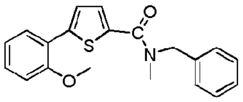 37

The title compound was prepared by reaction of N-benzyl-5-bromo-N-methylthiophene-2-carboxamide (33a) (80 mg, 0.26 mmol) and 2-methoxyphenylboronic acid (47 mg, 0.31 mmol) with tetrakis(triphenylphosphine) palladium (30 mg, 0.026 mmol) as catalyst according to method B for 14 h. Purification by FC (*n*-hexane/ ethyl acetate 6:1) afforded the desired compound as colorless solid (76 mg, 87%); MS (ESI): 338 $(M+H)^+$; $^1H$ NMR ($CD_3COCD_3$): 3.16 (br s, 3H), 3.97 (s, 3H), 4.82 (br s, 2H), 7.03 (td, J= 7.6, 1.3 Hz, 1H), 7.15 (dd, J= 8.2, 1.3 Hz, 1H), 7.29-7.41 (m. 7H), 7.52 (d, *J*= 4.0 Hz, 1H), 7.76 (dd, *J*= 7.8, 1.6 Hz, 1 H); $^{13}C$ NMR ($CD_3COCD_3$): 56.0, 112.9, 121.8, 123.1, 125.5, 128.1, 128.8, 129.5, 130.2, 132.0, 138.6, 143.5, 156.7.

1.38. *N*-Benzyl-5-(2-hydroxyphenyl)-*N*-methylthiophene-2-carboxamide (38).

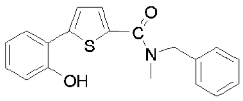 38

The title compound was prepared by reaction of N-benzyl-5-(2-methoxyphenyl)-N-methylthiophene-2-carboxamide (37) (55 mg, 0.16 mmol) with borontrifluoride dimethyl sulfide complex (0.10 ml, 0.96 mmol) according to method C for 14 h. Purification by FC ($CH_2Cl_2/CH_3OH$ 100:1) yielded the title compound as colorless solid (49 mg, 93%); MS: 324 $(M+H)^+$; $^1H$ NMR ($CD_3COCD_3$): 3.16 (br s, 3H), 4.83 (br s, 2H), 6.93 (ddd, J= 8.5, 7.3, 1.3 Hz, 1H), 7.03 (dd, J= 7.8, 1.3 Hz, 1H), 7.18 (ddd, J= 8.5, 7.3, 1.3 Hz, 1H), 7.28-7.32 (m, 1H), 7.35-7.41 (m, 5H), 7.56 (d, J= 4.0 Hz, 1H), 7.70 (dd, J= 7.8, 1.6 Hz, 1H), 9.23 (s, 1H); $^{13}C$ NMR ($CD_3COCD_3$): 117.3, 121.0, 121.5, 125.4, 128.1, 129.0, 129.6, 130.0, 138.2, 138.7, 154.6.

1.39. *N*-(3-Hydroxybenzyl)-5-(4-methoxyphenyl)-*N*-methylthiophene-2-carboxamide (39).

5-Bromo-*N*-(3-hydroxybenzyl)-*N*-methylthiophene-2-carboxamide (39a).

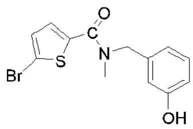 39a

The title compound was prepared by reaction of 23a (340 mg, 1mmol) and borontrifluoride dimethyl sulfide complex (0.63 ml, 6 mmol) according to method C for 5 h. Purification by FC ($CH_2Cl_2/CH_3OH$ 100:1) afforded the desired compound as colorless solid (300 mg, 93%); $^1H$ NMR ($CD_3COCD_3$): 3.13 (br s, 3H), 4.71 (br s, 2H), 6.76-6.79 (m, 3H), 7.13-7.14 (m, 1H), 7.20 (t, J= 7.7 Hz, 1H), 7.25 (br s, 1H), 8.69 (s, 1H).

*N*-(3-Hydroxybenzyl)-5-(4-methoxyphenyl)-*N*-methylthiophene-2-carboxamide (39).

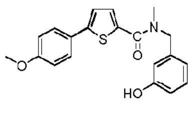 39

The title compound was prepared by reaction of 5-bromo-N-(3-hydroxybenzyl)-N-methylthiophene-2-carboxamide (39a) (49 mg, 0.15 mmol) and 4-methoxyphenylboronic acid (27 mg, 0.18 mmol) with tetrakis(triphenylphosphine)

palladium (17 mg, 0.015 mmol) as catalyst according to method B for 6 h. Purification by FC (*n*-hexane/ethyl acetate 6:1) afforded the desired compound as colorless solid (48 mg, 90%); MS (ESI): 354 $(M+H)^+$; $^{1}H$ NMR ($CD_3COCD_3$): 3.15 (br s, 3H), 3.84 (s, 3H), 4.75 (br s, 2H), 6.77 (dd, J= 8.0, 2.2 Hz, 1H), 6.80-6.83 (m, 2H), 7.00 (d, J= 8.8 Hz, 2H), 7.21 (t, J= 8.0 Hz, 1H), 7.27 (d, J= 3.5 Hz, 1H), 7.31 (br s, 1H), 7.63 (d, J= 8.8 Hz, 2H), 8.35 (s, 1H); $^{13}C$ NMR ($CD_3COCD_3$): 55.7, 115.2, 115.4, 122.9, 127.1, 128.1, 129.6, 130.6, 132.0, 133.4, 140.1, 143.9, 158.8.

1.40. 5-(3-(Dimethylamino)phenyl)-*N*-(3-hydroxybenzyl)-*N*-methylthiophene-2-carboxamide (40).

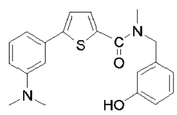

40

The title compound was prepared by reaction of 5-bromo-N-(3-hydroxybenzyl)-N-methylthiophene-2-carboxamide (39a) (49 mg, 0.15 mmol) and *N,N*-dimethyl-3-aminophenylboronic acid (30 mg, 0.18 mmol) with tetrakis(triphenylphosphine) palladium (17 mg, 0.015 mmol) as catalyst according to method B for 14 h. Purification by FC (*n*-hexane/ethyl acetate 5:1→3:1) afforded the desired compound as yellowish solid (45 mg, 82%); MS (ESI): 367 $(M+H)^+$; $^{1}H$ NMR ($CD_3COCD_3$): 3.00 (s, 6H), 3.15 (br s, 3H), 4.75 (br s, 2H), 6.75-6.82 (m, 4H), 6.98 (d, *J*= 4.0 Hz, 1H), 7.00 (s, 1H), 7.19-7.26 (m, 2H), 7.36 (s, 1H), 7.39 (br s, 1H), 8.36 (s, 1H); $^{13}C$ NMR ($CD_3COCD_3$): 40.5, 110.5, 113.5, 114.9, 115.2, 123.7, 130.5, 135.0, 138.1, 140.1, 149.6, 152.1, 157.8, 158.8.

1.41. 5-(3-Fluorophenyl)-*N*-(3-hydroxybenzyl)-*N*-methylthiophene-2-carboxamide (41).

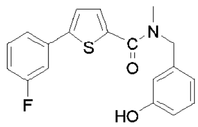

41

The title compound was prepared by reaction of 5-bromo-N-(3-hydroxybenzyl)-N-methylthiophene-2-carboxamide (39a) (49 mg, 0.15 mmol) and 3-fluorophenylboronic acid (25 mg, 0.18 mmol) with tetrakis(triphenylphosphine) palladium (17 mg, 0.015 mmol) as catalyst according to method B for 14 h. Purification by FC (*n*-hexane/ethyl acetate 6:1→3:1) afforded the desired compound as colorless solid (40 mg, 78%); MS (ESI): 342 $(M+H)^+$; $^{1}H$ NMR ($CD_3COCD_3$): 3.16 (br s, 3H), 4.76 (br s, 2H),6.77-6-82 (m, 3H), 7.11-7.15 (m, 1H), 7.21 (t, *J*= 7.7 Hz, 1H), 7.42 (br s, 1H), 7.46-7.51 (m, 3H), 7.54 (d, *J*= 8.2 Hz, 1H), 8.35 (s, 1H); $^{13}C$ NMR ($CD_3COCD_3$): 113.2, 115.3, 115.7, 115.9, 122.7, 125.1, 130.7, 131.9, 132.0, 136.8, 139.2, 140.1, 144.4, 146.5, 148.3, 150.9, 158.8, 160.9, 163.1, 165.1.

1.42. 5-(4-Fluorophenyl)-*N*-(3-hydroxybenzyl)-*N*-methylthiophene-2-carboxamide (42).

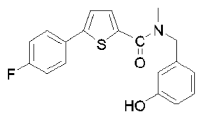

42

The title compound was prepared by reaction of 5-bromo-N-(3-hydroxybenzyl)-N-methylthiophene-2-carboxamide (39a) (49 mg, 0.15 mmol) and 4-fluorophenylboronic acid (25 mg, 0.18 mmol) with tetrakis(triphenylphosphine) palladium (17 mg, 0.015 mmol) as catalyst according to method B for 14 h. Purification by FC (*n*-hexane/ethyl acetate 5:1→3:1) afforded the desired compound as colorless solid (43 mg, 84%); MS (ESI): 342 $(M+H)^+$; $^{1}H$ NMR ($CD_3COCD_3$): 3.16 (br s, 3H), 4.75 (br s, 2H), 6.77-6.82 (m, 3H), 7.19-7.24 (m, 3H), 7.37 (d, *J*= 3.7 Hz, 1H), 7.40 (br s, 1H), 7.73-7.76 (m, 2H), 8.35 (br s, 1H); $^{13}C$ NMR ($CD_3COCD_3$): 115.3, 116.8, 116.9, 124.3, 127.8, 128.7, 128.8, 130.6, 131.0, 138.9, 140.0, 147.1, 158.8, 162.7, 164.2, 164.6.

1.43. 5-(2-Fluoro-3-methoxyphenyl)-*N*-(3-hydroxybenzyl)-*N*-methylthiophene-2-carboxamide (43).

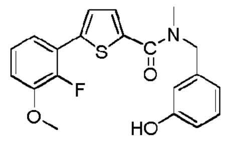

43

The title compound was prepared by reaction of 5-bromo-N-(3-hydroxybenzyl)-N-methylthiophene-2-carboxamide (39a) (49 mg, 0.15 mmol) and 2-fluoro-3-methoxyphenylboronic acid (31 mg, 0.18 mmol) with tetrakis(triphenylphosphine) palladium (17 mg, 0.015 mmol) as catalyst according to method B for 14 h. Purification by FC (*n*-hexane/ethyl acetate 5:1→3:1) afforded the desired compound as colorless solid (40 mg, 72%); MS (ESI): 372 $(M+H)^+$; $^1H$ NMR ($CD_3COCD_3$): 3.16 (br s, 3H), 3.93 (s, 3H), 4.76 (br s, 2H), 6.77-6.83 (m, 3H), 7.13-7.23 (m, 3H), 7.31 (td, *J*= 7.8, 1.6 Hz, 1H), 7.45 (br s, 1H), 7.48 (br s, 1H), 8.35 (s, 1H); $^{13}C$ NMR ($CD_3COCD_3$) : 56.7, 114.0, 114.1, 115.3, 120.5, 120.6, 122.7, 122.8, 125.6, 127.2, 130.7, 139.9, 140.7, 148.9, 149.6, 150.9, 158.8.

1.44. 5-(2-Fluoro-3-hydroxyphenyl)-*N*-(3-hydroxybenzyl)-*N*-methylthiophene-2-carboxamide (44).

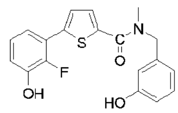

44

The title compound was prepared by reaction of 5-(2-fluoro-3-methoxyphenyl)-N-(3-hydroxybenzyl)-N-methylthiophene-2-carboxamide (43) (30 mg, 0.08 mmol) with borontrifluoride dimethyl sulfide complex (0.05 ml, 0.48 mmol) according to method C for 14 h. Purification by FC ($CH_2Cl_2/CH_3OH$ 100:1) yielded the title compound as colorless solid (25 mg, 87%); MS (ESI): 358 $(M+H)^+$; $^1H$ NMR ($CD_3COCD_3$): 3.16 (br s, 3H), 4.76 (br s, 2H), 6.77-6.99 (m, 3H), 7.01 (td, *J*= 8.0, 1.6 Hz, 1H), 7.08 (td, *J*= 8.0, 0.9 Hz, 1H), 7.21 (t, *J*= 8.0 Hz, 2H), 7.44 (br s, 1H), 7.46 (s, 1H), 8.36 (s, 1H), 8.86 (d, *J*= 1.6 Hz, 1H); $^{13}C$ NMR ($CD_3COCD_3$): 115.3, 118.4, 119.7, 122.9, 125.6, 126.6, 127.0, 127.1, 130.6, 139.6, 140.0, 141.1, 146.7, 148.3, 150.3, 158.8.

1.45. *N*-(3-Hydroxybenzyl)-5-(3-methoxyphenyl)-*N*-methylthiophene-2-carboxamide (45).

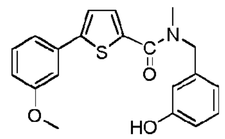

45

The title compound was prepared by reaction of 5-bromo-N-(3-hydroxybenzyl)-N-methylthiophene-2-carboxamide (39a) (33 mg, 1mmol) and 3-methoxyphenylboronic acid (18 mg, 0.12 mmol) with tetrakis(triphenylphosphine) palladium (12 mg, 0.01 mmol) as catalyst according to method B for 6 h. Purification by FC (*n*-hexane/ethyl acetate 6:1→3:1) afforded the desired compound as colorless solid (25 mg, 71%). MS (ESI): 354 $(M+H)^+$; $^1H$ NMR ($CD_3COCD_3$): 3.16 (br s, 3H), 3.86 (s, 3H), 4.76 (br s, 2H), 6.77-6.82 (m, 3H), 6.94 (ddd, *J*= 8.0, 2.5, 0.9 Hz, 1H), 7.21 (t, *J*= 8.0 Hz, 1H), 7.23 (t, *J*= 2.0 Hz, 1H), 7.27 (d, *J*= 8.0 Hz, 1H), 7.35 (t, *J*= 8.0 Hz, 1H), 7.41 (br, s, 2H), 8.36 (s, 1H); $^{13}C$ NMR ($CD_3COCD_3$): 55.7, 112.1, 114.9, 115.3, 119.1, 130.6, 131.1, 135.7, 140.0, 148.1, 158.8, 161.2.

1.46. *N*-(3-Hydroxybenzyl)-*N*-methyl-5-m-tolylthiophene-2-carboxamide (46). *N*-(3-Methoxybenzyl)-*N*-methyl-5-m-tolylthiophene-2-carboxamide (46a).

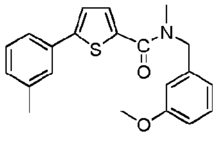

46a

The title compound was prepared by reaction of 5-bromo-N-(3-methoxybenzyl)-N-methylthiophene-2-carboxamide (23a) (53 mg, 0.15 mol) and 3-methylphenylboronic acid (24 mg, 0.18 mmol) with tetrakis(triphenylphosphine) palladium (17 mg, 0.015 mmol) as catalyst according to method B for 6 h. Purification by FC (*n*-hexane/ethyl acetate 6:1) afforded the desired compound as colorless oil (50 mg, 95%); MS (ESI): 352 $(M+H)^+$; $^1H$ NMR ($CD_3COCD_3$): 2.38 (s, 3H), 3.17 (br s, 3H), 3.80 (s, 3H), 4.80 (br s, 2H), 6.86-6.89 (m, 1H), 6.91-6.93 (m, 2H), 7.19 (d, *J*= 7.6 Hz, 1H), 7.29-7.33 (m, 2H), 7.38-7.42 (m, 2H), 7.49 (d, *J*= 7.6 Hz, 1H), 7.53 (s, 1H); $^{13}C$ NMR ($CD_3COCD_3$): 21.3, 55.5, 113.5, 123.8, 123.9, 127.3, 129.9, 130.0, 130.6, 134.4, 138.5, 139.7, 140.1, 148.5, 161.1.

*N*-(3-Hydroxybenzyl)-*N*-methyl-5-m-tolylthiophene-2-carboxamide (46).

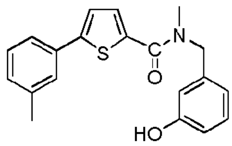

46

The title compound was prepared by reaction of N-(3-methoxybenzyl)-N-methyl-5-m-tolylthiophene-2-carboxamide (46a) (35 mg, 0.1 mmol) with borontrifluoride dimethyl sulfide complex (0.06 ml, 0.6 mmol) according to method C for 4 h. Purification by FC ($CH_2Cl_2/CH_3OH$ 100:1) yielded the title compound as colorless solid (30 mg, 89%); MS (ESI): 338 $(M+H)^+$; $^1H$ NMR ($CD_3COCD_3$): 2.37 (s, 3H), 3.15 (br s, 3H), 4.75 (br s, 2H), 6.78 (ddd, *J*= 8.2, 2.5, 0.9 Hz, 1H), 6.80-6.83 (m, 2H), 7.17-7.22 (m, 2H), 7.32 (t, *J*= 7.8 Hz, 1H), 7.37-7.43 (m, 2H), 7.49 (d, *J*= 7.8 Hz, 1H), 7.53 (s, 1H), 8.46 (s, 1H); $^{13}C$ NMR ($CD_3COCD_3$): 21.3, 115.2, 123.8, 123.9, 127.3, 129.9, 130.0, 130.7, 134.4, 138.5, 139.7, 140.0, 148.5, 158.8.

1.47. *N*,5-bis(3-Methoxyphenyl)-*N*-methylthiophene-2-carboxamide (47). 5-Bromo-*N*-(3-methoxyphenyl)-*N*-methylthiophene-2-carboxamide (47a).

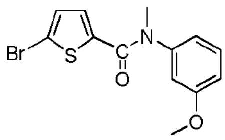

47a

The title compound was prepared by reaction of 5-bromothiophene-2-carbonyl chloride (225 mg, 1 mmol) and *N*-methyl-3-methoxyaniline (137 mg, 1 mmol) in presence of triethylamine (0.16 ml, 1.15 mmol) according to method A. Purification by FC (*n*-hexane/ethyl acetate 6:1) afforded the desired compound as colorless solid (320 mg, 98%); $^1H$ NMR ($CD_3COCD_3$): 3.34 (br s, 3H), 3.82 (s, 3H), 6.52 (d, *J*= 4.1 Hz, 1H), 6.92 (d, *J*= 4.1 Hz, 1H), 6.93 (ddd, *J*= 8.0, 1.9, 0.9 Hz, 1H), 6.99 (t, *J*= 2.2 Hz, 1H), 7.03 (ddd, *J*= 8.0, 2.5, 0.9 Hz, 1H), 7.39 (t, *J*= 8.0 Hz, 1H); $^{13}C$ NMR ($CD_3COCD_3$): 38.9, 55.9, 114.7, 115.2, 118.3, 121.2, 131.0, 131.5, 133.1, 141.3, 145.8, 161.9.

*N*,5-bis(3-Methoxyphenyl)-*N*-methylthiophene-2-carboxamide (47).

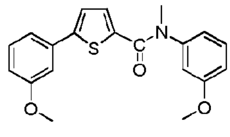

47

The title compound was prepared by reaction of 5-bromo-N-(3-methoxyphenyl)-N-methylthiophene-2-carboxamide (47a) (75 mg, 0.23 mmol) and 3-methoxyphenylboronic acid (41 mg, 0.27 mmol) with tetrakis(triphenylphosphine) palladium (27 mg, 0.023 mmol) as catalyst according to method B for 5 h. Purification by FC ($CH_2Cl_2$) afforded the desired compound as colorless solid (80 mg, 98%); $^1H$ NMR ($CD_3COCD_3$): 3.38 (s, 3H), 3.82 (s, 3H), 3.83 (s, 3H), 6.58 (d, *J*= 4.1 Hz, 1H), 6.90 (ddd, *J*= 8.2, 2.5, 0.9 Hz, 1H), 6.94 (ddd, *J*= 7.6, 1.6, 0.9 Hz, 1H), 6.98-7.01 (m, 2H), 7.12 (t, *J*= 2.0 Hz, 1H), 7.15 (ddd, *J*= 7.6, 1.6, 0.9 Hz, 1H), 7.15 (d, *J*= 4.1 Hz, 1H), 7.30 (t, *J*= 8.0 Hz, 1H), 7.38 (td, *J*= 8.0, 0.9 Hz, 1H); $^{13}C$ NMR ($CD_3COCD_3$): 39.0, 55.6, 55.9, 112.0, 114.6, 114.7, 114.8, 118.8, 121.0, 124.1, 131.0, 131.3, 133.1, 135.7, 138.9, 146.6, 148.9, 161.2, 161.8, 162.4.

1.48. *N*,5-bis(3-Hydroxyphenyl)-*N*-methylthiophene-2-carboxamide (48).

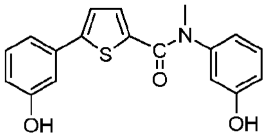

48

The title compound was prepared by reaction of *N*,5-bis(3-methoxyphenyl)-*N*-methylthiophene-2-carboxamide (47) (60 mg, 0.17 mmol) with borontrifluoride dimethyl sulfide complex (0.21 ml, 2.04 mmol) according to method C for 8 h. Purification by FC ($CH_2Cl_2/CH_3OH$ 100:1→50:1) yielded the title compound as colorless solid (50 mg, 90%); MS (ESI): 326 $(M+H)^+$; $^1H$ NMR ($CD_3COCD_3$): 3.36 (s, 3H), 6.63 (d, *J*= 3.8 Hz, 1H), 6.81 (ddd, *J*= 8.2, 2.5, 0.9 Hz, 1H), 6.83-6.86 (m, 2H), 6.91 (ddd, *J*= 8.2, 2.5, 0.9 Hz, 1H), 7.04-7.07 (m, 2H), 7.11 (d, *J*= 4.1 Hz, 1H), 7.21 (t, *J*= 7.9 Hz, 1H), 7.30 (td, *J*= 7.9, 0.6 Hz, 1H), 8.53 (s, 1H), 8.70 (s, 1H); $^{13}C$ NMR ($CD_3COCD_3$): 39.0, 113.3, 115.9, 116.1, 116.4, 117.9, 119.9, 123.8, 131.1, 131.4, 133.3, 135.7, 138.6, 146.5, 149.2, 158.8, 159.4, 162.4.

1.49. 5-(2-Methoxyphenyl)-*N*-(3-methoxyphenyl)-*N*-methylthiophene-2-carboxamide (49).

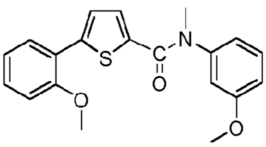

49

The title compound was prepared by reaction of 5-bromo-*N*-(3-methoxyphenyl)-*N*-methylthiophene-2-carboxamide (47a) (75 mg, 0.23 mmol) and 2-methoxyphenylboronic acid (41 mg, 0.27 mmol) with tetrakis(triphenylphosphine) palladium (27 mg, 0.023 mmol) as catalyst according to method B for 14 h. Purification by FC (*n*-hexane/ethyl acetate 10:1→6:1) afforded the desired compound as colorless solid (78 mg, 96%); MS (ESI): 354 $(M+H)^+$; $^1H$ NMR ($CD_3COCD_3$): 3.38 (s, 3H), 3.82 (s, 3H), 3.90, (s, 3H), 6.69 (d, *J*= 4.1 Hz, 1H), 6.93 (ddd, *J*= 8.0, 1.9, 0.9 Hz, 1H), 6.96-7.01 (m, 3H), 7.09 (dd, *J*= 8.0, 0.9 Hz, 1H), 7.28-7.32 (m, 2H), 7.37 (td, *J*= 8.0, 0.6 Hz, 1H), 7.65 (dd, *J*= 8.0, 1.6 Hz, 1H); $^{13}C$ NMR ($CD_3COCD_3$): 38.9, 54.9, 55.9, 112.9, 114.6, 114.7, 121.2, 121.8, 123.1, 125.2, 128.7, 130.2, 131.3, 132.0, 138.7, 144.6, 146.8, 156.7, 161.8, 162.9.

1.50. 5-(2-Hydroxyphenyl)-*N*-(3-hydroxyphenyl)-*N*-methylthiophene-2-carboxamide (50).

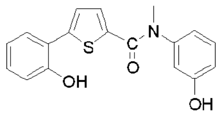

50

The title compound was prepared by reaction of 5-(2-methoxyphenyl)-*N*-(3-methoxyphenyl)-*N*-methylthiophene-2-carboxamide (49) (50 mg, 0.14 mmol) with borontrifluoride dimethyl sulfide complex (0.18 ml, 1.68 mmol) according to method C for 14 h. Purification by FC ($CH_2Cl_2/CH_3OH$ 100:1→50:1) yielded the title compound as colorless solid (40 mg, 88%); $^1H$ NMR ($CD_3COCD_3$): 3.36 (s, 3H), 6.59 (d, *J*= 4.3 Hz, 1H), 6.81 (t, *J*= 2.2 Hz, 1H), 6.84 (ddd, *J*= 7.3, 1.8, 0.9 Hz, 1H), 6.86-6.89 (m, 2H), 6.99 (dd, *J*= 8.0, 1.2 Hz, 1H), 7.15 (ddd, *J*= 8.2, 7.3, 1.2 Hz, 1H), 7.28 (t, *J*= 8.0 Hz, 1H), 7.32 (d, *J*= 4.0 Hz, 1H), 7.57 (dd, *J*= 8.0, 1.5 Hz, 1H), 8.67 (s, 1H), 9.18 (s, 1H); $^{13}C$ NMR ($CD_3COCD_3$): 39.0, 115.9, 117.3, 119.8, 120.9, 121.5, 125.4, 128.9, 130.0, 131.3, 131.9, 138.5, 145.3, 146.8, 154.7, 159.4, 163.0.

1.51. *N*-(3-Methoxyphenyl)-*N*-methyl-5-phenylthiophene-2-carboxamide (51).

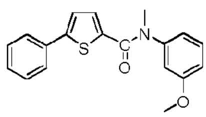

51

The title compound was prepared by reaction of 5-bromo-N-(3-methoxyphenyl)-N-methylthiophene-2-carboxamide (47a) (75 mg, 0.23 mmol) and phenylboronic acid (33 mg, 0.27 mmol) with tetrakis(triphenylphosphine) palladium (27 mg, 0.023 mmol) as catalyst according to method B for 4 h. Purification by FC (*n*-hexane/ethyl acetate 10:1→6:1) afforded the desired compound as colorless solid (70 mg, 94%); $^1H$ NMR ($CD_3COCD_3$): 3.38 (s, 3H), 3.82 (s, 3H), 6.60 (d, J= 4.0 Hz, 1H), 6.94 (ddd, J= 8.0, 2.1, 0.9 Hz, 1H), 6.99-7.01 (m, 2H), 7.16 (d, J= 4.0 Hz, 1H), 7.32 (ddt, J= 8.0, 6.3, 1.2 Hz, 1H), 7.36-7.41 (m, 3H), 7.59 (d, J= 8.0 Hz, 2H); $^{13}C$ NMR ($CD_3COCD_3$) : 39.0, 55.9, 114.5, 114.7, 121.0, 123.9, 126.6, 129.2, 130.0, 131.3, 133.20, 134.5, 138.9, 146.6, 149.0, 161.8, 162.4.

1.52. *N*-(3-Hydroxyphenyl)-*N*-methyl-5-phenylthiophene-2-carboxamide (52).

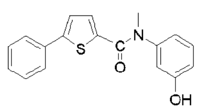

52

The title compound was prepared by reaction of N-(3-methoxyphenyl)-N-methyl-5-phenylthiophene-2-carboxamide (51) (40 mg, 0.12 mmol) with borontrifluoride dimethyl sulfide complex (0.08 ml, 0.72 mmol) according to method C for 6 h. Purification by FC ($CH_2Cl_2/CH_3OH$ 100:1→50:1) yielded the title compound as colorless solid (30 mg, 79%); $^1H$ NMR ($CD_3COCD_3$): 3.36 (s, 3H), 6.62 (d, J= 4.0 Hz, 1H), 6.83-6.86 (m, 2H), 6.92 (ddd, J= 8.2, 2.4, 0.9 Hz, 1H), 7.17 (d, J= 4.0 Hz, 1H), 7.28-7.34 (m, 2H), 7.38-7.41 (m, 2H), 7.59 (d, J= 7.0 Hz, 2H), 8.74 (s, 1H); $^{13}C$ NMR ($CD_3COCD_3$): 39.0, 115.9, 116.1, 119.9, 123.9, 126.6, 129.2, 130.0, 131.4, 133.3, 134.5, 138.9, 146.5, 149.1, 159.5, 162.4.

1.53. 5-(4-Cyanophenyl)-*N*-(3-methoxyphenyl)-*N*-methylthiophene-2-carboxamide (53).

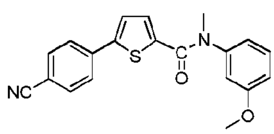

53

The title compound was prepared by reaction of 5-bromo-N-(3-methoxyphenyl)-N-methylthiophene-2-carboxamide (47a) (70 mg, 0.21 mmol) and 4-cyanophenylboronic acid (37 mg, 0.25 mmol) with tetrakis(triphenylphosphine)

palladium (24 mg, 0.021 mmol) as catalyst according to method B for 6 h. Purification by FC (*n*-hexane/ethyl acetate 10:1→6:1) afforded the desired compound as colorless solid (60 mg, 82%); MS (ESI): 349 $(M+H)^+$; $^1H$ NMR ($CD_3COCD_3$): 3.39 (s, 3H), 3.82 (s, 3H), 6.62 (d, J= 4.1 Hz, 1H), 6.95 (ddd, J= 7.6, 1.9, 1.2 Hz, 1H), 7.00-7.02 (m, 2H), 7.35 (d, *J*= 4.1 Hz, 1H), 7.37-7.40 (m, 1H), 7.79 (s, 4H); $^{13}C$ NMR ($CD_3COCD_3$): 39.0, 55.9, 112.3, 114.5, 114.8, 119.1, 121.0, 126.0, 127.1, 131.4, 133.3, 133.8, 138.6, 141.0, 146.4, 146.4, 161.8, 162.1.

1.54. 5-(4-Cyanophenyl)-*N*-(3-hydroxyphenyl)-*N*-methylthiophene-2-carboxamide (54).

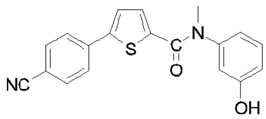
54

The title compound was prepared by reaction of 5-(4-cyanophenyl)-*N*-(3-methoxyphenyl)-*N*-methylthiophene-2-carboxamide (53) (40 mg, 0.11 mmol) with borontrifluoride dimethyl sulfide complex (0.07 ml, 0.66 mmol) according to method C for 6 h. Purification by FC ($CH_2Cl_2$/$CH_3OH$ 100:1→50:1) yielded the title compound as colorless solid (30 mg, 79%); MS (ESI): 335 $(M+H)^+$; $^1H$ NMR ($CD_3COCD_3$): 3.37 (s, 3H), 6.65 (d, *J*= 4.1 Hz, 1H), 6.84-6.87 (m, 2H), 6.92 (ddd, *J*= 8.0, 2.2, 0.9 Hz, 1H), 7.30 (t, *J*= 8.0 Hz, 1H), 7.36 (d, *J*= 4.1 Hz, 1H), 7.79 (s, 4H), 8.72 (s, 1H); $^{13}C$ NMR ($CD_3COCD_3$): 39.0, 112.2, 115.9, 116.3, 119.1, 119.9, 126.1, 127.1, 131.5, 133.3, 133.8, 138.6, 141.1, 146.3, 146.4, 159.5, 162.0.

1.55. *N*-(3-Methoxyphenyl)-5-(4-methoxyphenyl)-*N*-methylthiophene-2-carboxamide (55).

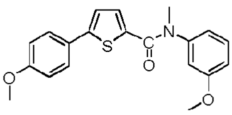
55

The title compound was prepared by reaction of 5-bromo-*N*-(3-methoxyphenyl)-*N*-methylthiophene-2-carboxamide (47a) (40 mg, 0.12 mmol) and 4-methoxyphenyl-boronic acid (22 mg, 0.14 mmol) with tetrakis(triphenylphosphine) palladium (14 mg, 0.012 mmol) as catalyst according to method B for 6 h. Purification by FC ($CH_2Cl_2$/$CH_3OH$ 200:1) afforded the desired compound as colorless solid (40 mg, 92%); MS (ESI): 354 $(M+H)^+$; $^1H$ NMR ($CD_3COCD_3$): 3.37 (s, 3H), 3.81 (s, 3H), 3.82 (s, 3H), 6.55 (d, *J*= 4.1 Hz, 1H), 6.92-6.96 (m, 3H), 6.97-7.01 (m, 2H), 7.02 (d, *J*= 4.1 Hz, 1H), 7.37 (td, *J*= 7.9, 0.6 Hz, 1H), 7.51 (d, *J*= 9.1 Hz, 2H); $^{13}C$ NMR ($CD_3COCD_3$): 39.0, 55.7, 55.9, 114.5, 115.6, 115.3, 121.0, 122.7, 127.1, 127.9, 131.3, 133.3, 137.8, 146.7, 149.3, 161.0, 161.8, 162.5.

1.56. 5-(2-Fluoro-3-methoxyphenyl)-*N*-(3-methoxyphenyl)-*N*-methylthiolphene-2-carboxamide (56).

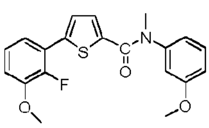
56

The title compound was prepared by reaction of 5-bromo-*N*-(3-methoxyphenyl)-*N*-methylthiophene-2-carboxamide (47a) (40 mg, 0.12 mmol) and 2-fluoro-3-methoxyphenylboronic acid (25 mg, 0.14 mmol) with tetrakis(triphenylphosphine) palladium (14 mg, 0.012 mmol) as catalyst according to method B for 14 h. Purification by FC (*n*-hexane/ethyl acetate 10:1→6:1) afforded the desired compound as colorless solid (30 mg, 66%); MS (ESI): 372 $(M+H)^+$; $^1H$ NMR ($CD_3COCD_3$): 3.38 (s, 3H), 3.82 (s, 3H), 3.90 (s, 3H), 6.65 (dd, *J*= 4.0, 1.0 Hz, 1H), 6.95 (ddd, *J*= 7.6, 1.8, 1.0 Hz, 1H), 6.99-7.01 (m, 2H), 7.09-7.20 (m, 3H), 7.23 (dd, *J*= 4.0, 1.0 Hz, 1H), 7.37 (dd, *J*= 9.1, 7.9 Hz, 1H); $^{13}C$ NMR ($CD_3COCD_3$): 39.0, 55.9, 56.7, 114.0, 114.1, 114.6, 114.8, 120.4, 121.1, 122.7, 122.8, 125.5, 126.9, 127.0, 131.3, 132.5, 140.0, 141.7, 146.5, 148.8, 149.5, 149.6, 161.8, 162.4.

1.57. 5-(2,4-Dimethoxyphenyl)-*N*-(3-methoxyphenyl)-*N*-methylthiophene-2-carboxamide (57).

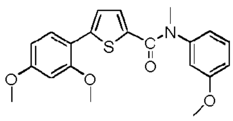

57

The title compound was prepared by reaction of 5-bromo-*N*-(3-methoxyphenyl)-*N*-methylthiophene-2-carboxamide (47a) (40 mg, 0.12 mmol) and 2,4-dimethoxyphenylboronic acid (27 mg, 0.14 mmol) with tetrakis(triphenylphosphine) palladium (14 mg, 0.012 mmol) as catalyst according to method B for 14 h. Purification by FC (*n*-hexane/ethyl acetate 10:1→6:1) afforded the desired compound as colorless solid (35 mg, 74%); MS (ESI): 384 $(M+H)^+$; $^1H$ NMR ($CD_3COCD_3$): 3.37 (s, 3H), 3.82 (s, 3H), 3.83 (s, 3H), 3.89 (s, 3H), 6.57 (ddd, *J*= 8.5, 2.4 Hz, 1H), 6.63-6.64 (m, 2H), 6.92 (ddd, *J*=7.9, 2.1, 0.9 Hz, 1H), 6.97 (t, *J*= 2.1 Hz, 1H), 7.00 (ddd, *J*= 8.2 2.7, 0.9 Hz, 1H), 7.15 (d, *J*= 4.3 Hz, 1H), 7.37 (t, *J*= 8.1 Hz, 1H), 7.55 (d, *J*= 8.5 Hz, 1H); $^{13}C$ NMR ($CD_3COCD_3$): 38.9, 55.8, 55.9, 99.6, 106.8, 114.5, 114.7, 116.1, 121.2, 123.9, 129.6, 131.2, 132.0, 137.3, 145.1, 146.9, 157.9, 161.7, 162.0, 163.0.

1.58. 5-(3-(Dimethylamino)phenyl)-*N*-(3-methoxyphenyl)-*N*-methylthiophene-2-carboxamide (58).

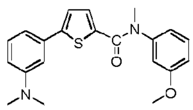

58

The title compound was prepared by reaction of 5-bromo-N-(3-methoxyphenyl)-N-methylthiophene-2-carboxamide (47a) (49 mg, 0.15 mmol) and 3-(dimethylamino)phenylboronic acid (30 mg, 0.18 mmol) with tetrakis(triphenylphosphine) palladium (17 mg, 0.015 mmol) as catalyst according to method B for 14 h. Purification by FC (*n*-hexane/ethyl acetate 10:1→6:1) afforded the desired compound as yellowish solid (26 mg, 47%); MS (ESI): 367 $(M+H)^+$; $^1H$ NMR ($CD_3COCD_3$): 2.96 (s, 6H), 3.38 (s, 3H), 3.82 (s, 3H), 6.56 (d, J= 4.0 Hz, 1H), 6.72 (dd, J= 8.2, 2.4 Hz, 1H), 6.86 (d, J= 7.6 Hz, 1H), 6.89 (t, J= 2.0 Hz, 1H), 6.94 (d, *J*= 7.6 Hz, 1H), 6.98-7.01 (m, 2H), 7.10 (d, J= 4.0 Hz, 1H), 7.19 (t, J= 7.9 Hz, 1H), 7.37 (t, J= 7.9 Hz, 1H); $^{13}C$ NMR ($CD_3COCD_3$): 39.0, 40.5, 55.9, 110.3, 113.5, 114.6, 114.8, 121.0, 123.5, 130.5, 131.3, 133.0, 135.0, 138.3, 146.7, 150.4, 152.0, 161.8, 162.5.

1.59. 5-(3-Fluorophenyl)-*N*-(3-methoxyphenyl)-*N*-methylthiophene-2-carboxamide (59).

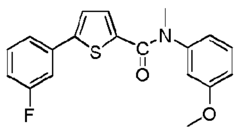

59

The title compound was prepared by reaction of 5-bromo-*N*-(3-methoxyphenyl)-*N*-methylthiophene-2-carboxamide (47a) (49 mg, 0.15 mmol) and 3-fluorophenylboronic acid (25 mg, 0.18 mmol) with tetrakis(triphenylphosphine) palladium (17 mg, 0.015 mmol) as catalyst according to method B for 8 h. Purification by FC (*n*-hexane/ethyl acetate 10:1→6:1) afforded the desired compound as colorless solid (40 mg, 78%); MS (ESI): 342 $(M+H)^+$; $^1H$ NMR ($CD_3COCD_3$): 3.38 (s, 3H), 3.82 (s, 3H), 6.59 (d, *J*= 4.1 Hz, 1H), 6.94-6.96 (m, 1H), 6.99-7.02 (m, 2H), 7.08-7.12 (m, 1H), 7.23 (d, *J*= 3.8 Hz, 1H), 7.34-7.46 (m, 4H); $^{13}C$ NMR ($CD_3COCD_3$): 39.0, 55.9, 113.0, 113.2, 114.5, 114.8, 115.7, 115.9, 121.0, 122.6, 124.9, 131.4, 131.9, 132.0, 133.1, 136.7, 136.8, 139.7, 146.5, 147.3, 161.8, 162.2, 163.0, 165.0.

1.60. 5-(3,4-Difluorophenyl)-*N*-(3-methoxyphenyl)-*N*-methylthiophene-2-carboxamide (60).

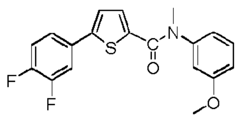

60

The title compound was prepared by reaction of 5-bromo-*N*-(3-methoxyphenyl)-*N*-methylthiophene-2-carboxamide (47a) (49 mg, 0.15 mmol) and 3,4-difluorophenylboronic acid (30 mg, 0.19 mmol) with tetrakis(triphenylphosphine) palladium (17 mg, 0.015 mmol) as catalyst according to method B for 14 h. Purification by FC (*n*-hexane/ethyl acetate 10:1→6:1) afforded the desired compound as colorless solid (35 mg, 65%); MS (ESI): 360 $(M+H)^+$; $^1H$ NMR ($CD_3COCD_3$): 3.38 (s, 3H), 3.82 (s, 3H), 6.57 (d, *J*= 4.1 Hz, 1H), 6.95 (ddd, *J*= 7.6, 1.8, 0.9 Hz, 1H), 6.99-7.02 (m, 2H), 7.18 (d, *J*= 4.1 Hz, 1H), 7.33-7.43 (m, 3H), 7.56 (ddd, *J*= 12.0, 7.6, 2.2 Hz, 1H); $^{13}C$ NMR ($CD_3COCD_3$): 39.0, 55.9, 114.5, 114.8, 115.5, 118.9, 119.1, 121.0, 123.4, 123.4, 123.5, 131.4, 132.1, 133.1, 139.9, 146.4, 146.5, 149.9, 150.0, 150.2, 150.3, 151.8, 151.9, 152.2, 152.3, 154.4, 161.8, 162.2.

1.61. 5-(3-Fluoro-4-methoxyphenyl)-*N*-(3-methoxyl)henyl)-*N*-methylthiolphene-2-carboxamide (61).

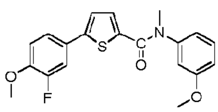

61

The title compound was prepared by reaction of 5-bromo-*N*-(3-methoxyphenyl)-*N*-methylthiophene-2-carboxamide (47a) (40 mg, 0.12 mmol) and 3-fluoro-4-methoxyphenylboronic acid (25 mg, 0.14 mmol) with tetrakis(triphenylphosphine) palladium (14 mg, 0.015 mmol) as catalyst according to method B for 6 h. Purification by FC (*n*-hexane/ethyl acetate 10:1→6:1) afforded the desired compound as colorless solid (40 mg, 72%); MS (ESI): 372 $(M+H)^+$; $^1H$ NMR ($CD_3COCD_3$): 3.38 (s, 3H), 3.82 (s, 3H), 3.91 (s, 3H), 6.55 (d, *J*= 4.0 Hz, 1H), 6.94 (ddd, *J*= 7.9, 1.9, 0.9 Hz, 1H), 6.99-7.01 (m, 2H), 7.08 (d, *J*= 4.0 Hz, 1H), 7.15 (t, *J*= 8.5 Hz, 1H), 7.33-7.39 (m, 3H); $^{13}C$ NMR ($CD_3COCD_3$): 39.0, 55.9, 56.6, 114.0, 114.2, 114.6, 114.7, 115.0, 115.1, 121.0, 122.9, 123.0, 123.6, 127.6, 127.7, 131.3, 133.2, 138.6, 146.6, 147.8, 148.8, 152.2, 154.2, 161.8, 162.3.

1.62. 5-(4-Fluoro-3-methoxyphenyl)-*N*-(3-methoxypheny)-*N*-methylthiophene-2-carboxamide (62).

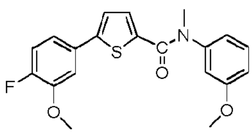

62

The title compound was prepared by reaction of 5-bromo-*N*-(3-methoxyphenyl)-*N*-methylthiophene-2-carboxamide (47a) (40 mg, 0.12 mmol) and 4-fluoro-3-methoxyphenylboronic acid (25 mg, 0.14 mmol) with tetrakis(triphenylphosphine) palladium (14 mg, 0.015 mmol) as catalyst according to method B for 8 h. Purification by FC (*n*-hexane/ethyl acetate 10:1→6:1) afforded the desired compound as colorless solid (43 mg, 96%); MS (ESI): 372 $(M+H)^+$; $^1H$ NMR ($CD_3COCD_3$): 3.38 (s, 3H), 3.82 (s, 3H), 3.95 (s, 3H), 6.52 (d, *J*= 4.0 Hz, 1H), 6.94 (ddd, *J*= 7.2, 2.2, 0.9 Hz, 1H), 6.98-7.01 (m, 2H), 7.11-7.17 (m, 3H), 7.32 (dd, *J*= 8.0, 2.1 Hz, 1H), 7.38 (td, *J*= 8.0, 0.9 Hz, 1H); $^{13}C$ NMR ($CD_3COCD_3$): 39.0, 55.9, 56.6, 112.2, 114.6, 117.1, 117.3, 119.2, 121.0, 124.2, 131.3, 131.4, 133.0, 139.1, 146.6, 148.1, 149.1, 152.2, 154.2, 161.8, 162.4.

1.63. *N*-(3-methoxyphenyl)-*N*-methyl-5-(3-(methylthio)phenyl)thiophene-2-carboxamide (63).

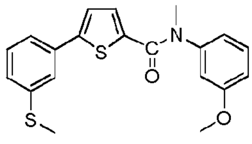

63

The title compound was prepared by reaction of 5-bromo-*N*-(3-methoxyphenyl)-*N*-methylthiophene-2-carboxamide (47a) (33 mg, 0.1 mmol) and 3-(thiomethyl)phenylboronic acid (23 mg, 0.14 mmol) with tetrakis(triphenylphosphine) palladium (12 mg, 0.01 mmol) as catalyst according to method B for 8 h. Purification by FC (*n*-hexane/ethyl acetate 10:1→7:1) afforded the desired compound as yellowish solid (22 mg, 59%); $^1$H NMR ($CD_3COCD_3$): 2.52 (s, 3H), 3.38 (s, 3H), 3.82 (s, 3H), 6.59 (d, *J*= 4.0 Hz, 1H), 6.94 (ddd, *J*= 8.0, 1.9, 0.9 Hz, 1H), 6.98-7.02 (m, 2H), 7.18 (d, *J*= 4.0 Hz, 1H), 7.23 (dt, *J*= 6.9, 1.9 Hz, 1H), 7.30-7.35 (m, 2H), 7.38 (td, *J*= 8.0, 0.9 Hz, 1H), 7.44-7.45 (m, 1H); $^{13}$C NMR ($CD_3COCD_3$): 15.3, 29.0, 55.9, 114.6, 114.7, 121.0, 123.2, 123.9, 124.3, 126.8, 130.4, 131.4, 133.1, 135.1, 139.2, 140.9, 146.6, 148.4, 161.8, 162.3.

1.64. *N*-(3-methoxyphenyl)-*N*-methyl-5-*m*-tolylthiophene-2-carboxamide (64).

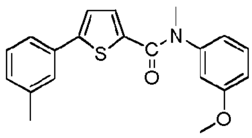

64

The title compound was prepared by reaction of 5-bromo-*N*-(3-methoxyphenyl)-*N*-methylthiophene-2-carboxamide (47a) (33 mg, 0.1 mmol) and 3-methylphenylboronic acid (19 mg, 0.14 mmol) with tetrakis(triphenylphosphine) palladium (12 mg, 0.01 mmol) as catalyst according to method B for 8 h. Purification by FC (*n*-hexane/ethyl acetate 10:1→6:1) afforded the desired compound as colorless solid (26 mg, 77%); MS (ESI): 338 (M+H)$^+$; $^1$H NMR ($CD_3COCD_3$): 2.34 (s, 3H), 3.38 (s, 3H), 3.81 (s, 3H), 6.57 (d, *J*= 4.0 Hz, 1H), 6.94 (ddd, *J*= 7.9, 2.2, 0.9 Hz, 1H), 6.98-7.01 (m, 2H), 7.13 (d, *J*= 4.0 Hz, 1H), 7.14-7.16 (m, 1H), 7.27 (t, *J*= 7.9 Hz, 1H), 7.36-7.39 (m, 2H), 7.41-7.42 (m, 1H); $^{13}$C NMR ($CD_3COCD_3$) : 21.3, 39.0, 55.9, 114.5, 114.6, 121.0, 123.7, 123.8, 127.2, 129.9, 130.0, 131.3, 133.1, 134.4, 138.8, 139.6, 146.7, 149.2, 161.8, 162.5.

1.65. 5-(2,6-Difluoro-3-methoxyphenyl)-*N*-(3-methoxyphenyl)-*N*-methylthiophene-2-carboxamide (65).

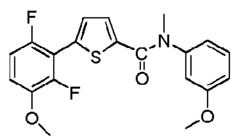

65

The title compound was prepared by reaction of 5-bromo-*N*-(3-methoxyphenyl)-*N*-methylthiophene-2-carboxamide (47a) (33 mg, 0.1 mmol) and 2,6-difluoro-3-methoxyphenyl boronic acid (26 mg, 0.14 mmol) with tetrakis(triphenylphosphine) palladium (12 mg, 0.01 mmol) as catalyst according to method B for 14 h. Purification by FC (*n*-hexane/ethyl acetate 10:1) afforded the desired compound as colorless solid (10 mg, 26%); MS (ESI): 390 (M+H)$^+$; $^1$H NMR ($CD_3COCD_3$): 3.40 (s, 3H), 3.82 (s, 3H), 3.90 (s, 3H), 6,71 (dt, *J*= 4.1, 0.9 Hz, 1H), 6.95 (ddd, *J*= 7.6, 1.9, 0.9 Hz, 1H), 6.99-7.01 (m, 2H), 7.05 (ddd, *J*= 11.4, 9.1, 2.2 Hz, 1H), 7.16 (td, *J*= 9.1, 5.0 Hz, 1H), 7.23 (dt, *J*= 4.1, 1.1 Hz, 1H), 7.36-7.39 (m, 1H); $^{13}$C NMR ($CD_3COCD_3$) : 39.0, 55.9, 57.1, 111.5, 111.7, 114.0, 114.1, 114.6, 114.9, 121.1, 129.9 131.4, 131.9, 134.4, 141.0, 145.9, 146.0, 146.4, 148.8, 149.1, 149.2, 149.9, 150.8, 152.8, 154.7, 161.8, 162.3.

1.66. 5-(2-Fluoro-3-methylphenyl)-*N*-(3-methoxyphenyl)-*N*-methylthiophene-2-carboxamide (66).

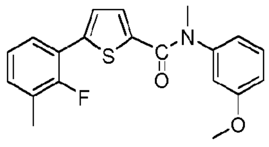

66

The title compound was prepared by reaction of 5-bromo-*N*-(3-methoxyphenyl)-*N*-methylthiophene-2-carboxamide (47a) (49 mg, 0.15 mmol) and 2-fluoro-3-methylphenylboronic acid (28 mg, 0.18 mmol) with tetrakis(triphenylphosphine) palladium (17 mg, 0.015 mmol) as catalyst according to method B for 14 h. Purification by FC (*n*-hexane/ethyl acetate 10:1) afforded the desired compound as colorless solid (50 mg, 94%); $^{1}H$ NMR ($CD_3COCD_3$): 2.29 (d, *J*= 2.5 Hz, 3H), 3.39 (s, 3H), 3.81 (s, 3H), 6.63 (dd, *J*= 4.1, 0.9 Hz, 1H), 6.94 (ddd, *J*= 7.9, 1.9, 0.9 Hz, 1H), 6.98-7.01 (m, 2H), 7.11 (t, *J*= 7.9 Hz, 1H), 7.22 (dd, *J*= 4.1, 0.9 Hz, 1H), 7.23-7.25 (m, 1H), 7.36-7.39 (m, 1H), 7.45-7.48 (m, 1H). $^{13}C$ NMR ($CD_3COCD_3$): 14.6, 39.0, 55.9, 114.5, 114.7, 121.0, 121.8, 121.9, 123.9, 125.3, 126.6, 126.7, 126.7, 126.8, 127.1, 127.2, 131.3, 132.2, 132.3, 132.5, 139.7, 139.8, 142.1, 146.6, 157.4, 159.3, 161.8, 162.4.

1.67. 5-(3-Methoxyphenyl)-*N*-methy-*N*-phenylthiophene-2-carboxamide (67). 5-Bromo-*N*-methy-*N*-phenylthiophene-2-carboxamide (67a).

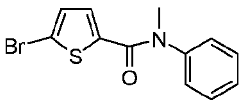

67a

The title compound was prepared by reaction of 5-bromothiophene-2-carbonyl chloride (113 mg, 0.5 mmol) and *N*-methylaniline (54 mg, 0.5 mmol) in presence of triethylamine (0.08 ml, 0.58 mmol) according to method A. Purification by FC ($CH_2Cl_2$) yielded the title compound as colorless solid (146 mg, 99%). MS (ESI): 296 $(M+H)^+$; $^{1}H$ NMR ($CD_3COCD_3$): 3.38 (s, 3H), 6.43 (d, *J*= 4.1 Hz, 1H), 6.90 (d, *J*= 4.1 Hz, 1H), 7.27-7.40 (m, 2H), 7.44-7.52 (m, 3H); $^{13}C$ NMR ($CD_3COCD_3$): 39.0, 118.3, 129.2, 129.4, 130.9, 131.0, 133.1, 141.4, 144.8, 161.3.

5-(3-Methoxyphenyl)-*N*-methyl-*N*-phenylthiophene-2-carboxamide (67).

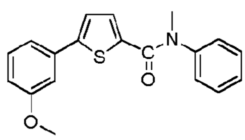

67

The title compound was prepared by reaction of 5-bromo-*N*-methyl-*N*-phenylthiophene-2-carboxamide (67a) (74 mg, 0.25 mmol) and 3-methoxyphenylboronic acid (45 mg, 0.3 mmol) with tetrakis(triphenylphosphine) palladium (29 mg, 0.025 mmol) as catalyst according to method B for 4 h. Purification by FC (*n*-hexane/ethyl acetate 10:1→6:1) afforded the desired compound as colorless solid (65 mg, 76%); MS (ESI): 324 $(M+H)^+$; $^{1}H$ NMR ($CD_3COCD_3$): 3.39 (s, 3H), 3.83 (s, 3H), 6.50 (d, *J*= 4.1 Hz, 1H), 6.90 (ddd, *J*= 8.2, 2.5, 0.9 Hz, 1H), 7.10 (t, *J*= 2.2 Hz, 1H), 7.12-7.14 (m, 2H), 7.30 (t, *J*= 8.2 Hz, 1H), 7.38-7.40 (m, 2H), 7.42-7.45 (m, 1H), 7.47-7.51 (m, 2H); $^{13}C$ NMR ($CD_3COCD_3$): 39.0, 55.6, 112.0, 114.8, 119.0, 124.0, 128.9, 129.0, 130.7, 131.0, 133.1, 135.7, 138.9, 145.6, 148.9, 161.2, 162.4.

1.68. 5-(3-Hydroxyphenyl)-*N*-methyl-*N*-phenylthiophene-2-carboxamide 68.

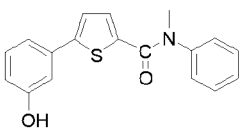

68

The title compound was prepared by reaction of 5-(3-methoxyphenyl)-*N*-methyl-*N*-phenylthiophene-2-carboxamide (67) (45 mg, 0.14 mmol) with borontrifluoride dimethyl sulfide complex (0.09 ml, 0.84 mmol) according to method C for 6 h. Purification by FC ($CH_2Cl_2/CH_3OH$ 100:1→50:1) yielded the title compound as colorless solid (38 mg, 88%); MS (ESI): 310 $(M+H)^+$; $^1$H NMR ($CD_3COCD_3$): 3.39 (s, 3H), 6.52 (d, *J*= 4.1 Hz, 1H), 6.81 (ddd, *J*= 8.0, 2.5, 0.9 Hz, 1H), 7.02-7.06 (m, 2H), 7.08 (d, *J*= 4.1 Hz, 1H), 7.21 (t, *J*= 8.0 Hz, 1H), 7.37-7.40 (m, 2H), 7.41-7.45 (m, 1H), 7.47-7.51 (m, 2H), 8.50 (s, 1H). $^{13}$C NMR ($CD_3COCD_3$): 39.0, 113.3, 116.4, 117.9, 123.7, 128.9, 129.0, 130.7, 131.1, 133.2, 135.7, 138.6, 145.5, 149.2, 158.8, 162.5.

1.69. *N*-Methyl-*N*,5-diphenylthiophene-2-carboxamide (69).

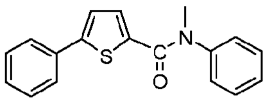

69

The title compound was prepared by reaction of 5-bromo-*N*-methyl-*N*-phenylthiophene-2-carboxamide (67a) (57 mg, 0.19 mmol) and phenylboronic acid (35 mg, 0.23 mmol) with tetrakis(triphenylphosphine) palladium (22 mg, 0.019 mmol) as catalyst according to method B for 4 h. Purification by FC (*n*-hexane/ethyl acetate 10:1→6:1) afforded the desired compound as colourless solid (50 mg, 90%); MS (ESI): 294 $(M+H)^+$; $^1$H NMR ($CD_3COCD_3$): 3.39 (s, 3H), 6.52 (d, *J*= 4.1 Hz, 1H), 7.14 (d, *J*= 4.1 Hz, 1H), 7.30-7.34 (m, 1H), 7.37-7.41 (m, 4H), 7.42-7.45 (m, 1H), 7.47-7.51 (m, 2H), 7.56-7.58 (m, 2H); $^{13}$C NMR ($CD_3COCD_3$): 39.0, 123.8, 126.6, 128.9, 129.0, 129.2, 130.0, 130.7, 133.3, 134.4, 138.9, 145.6, 149.1, 162.5.

1.70. 5-(3-Methoxyphenyl)-*N*-methyl-*N*-*m*-tolylthiophene-2-carboxamide (70). 5-Bromo-*N*-methyl-*N*-*m*-tolylthiophene-2-carboxamide (70a).

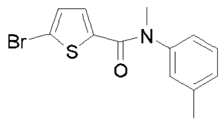

70a

The title compound was prepared by reaction of 5-bromothiophene-2-carbonyl chloride (225 mg, 1 mmol) and *N*-methyl-*m*-toluidine (121 mg, 1 mmol) in presence of triethylamine (0.16 ml, 1.15 mmol) according to method A. Purification by FC ($CH_2Cl_2$) yielded the desired compound as colourless solid (300 mg, 97%); MS (ESI): 309 $(M+H)^+$; $^1$H NMR ($CD_3COCD_3$): 2.37 (s, 3H), 3.34 (s, 3H), 6.46 (d, *J*= 4.1 Hz, 1H), 6.90 (d, *J*= 4.1 Hz, 1H), 7.14-7.16 (m, 1H), 7.22 (s, 1H), 7.28-7.29 (m, 1H), 7.37 (t, *J*= 7.7 Hz, 1H); $^{13}$C NMR ($CD_3COCD_3$): 21.2, 39.0, 118.3, 126.1, 129.6, 130.1, 130.6, 131.0, 133.1, 141.0, 141.4, 144.7, 161.2.

5-(3-Methoxyphenyl)-*N*-methyl-*N*-m-tolylthiophene-2-carboxamide (70).

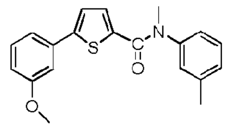

70

The title compound was prepared by reaction of 5-bromo-*N*-methyl-*N*-m-tolylthio-phene-2-carboxamide (70a) (78 mg, 0.25 mmol) and 3-methoxyphenylboronic acid (45 mg, 0.3 mmol) with tetrakis(triphenylphosphine) palladium (29 mg, 0.025 mmol) as catalyst according to method B for 4 h. Purification by FC (*n*-hexane/ethyl acetate 25:1→10:1) afforded the desired compound as colorless solid (65 mg, 77%); MS (ESI): 338 $(M+H)^+$; $^1$H NMR ($CD_3COCD_3$): 2.36 (s, 3H), 3.37 (s, 3H), 3.83 (s, 3H), 6.51 (d, *J*= 4.1 Hz, 1H), 6.90 (ddd, *J*= 8.2, 2.5, 0.9 Hz, 1H), 7.10-7.11 (m, 1H), 7.13-7.17

(m, 3H), 7.23 (s, 1H), 7.26 (d, *J*= 7.6 Hz, 1H), 7.30 (t, *J*= 7.6 Hz, 1H), 7.36 (t, *J*= 7.7 Hz, 1H); $^{13}C$ NMR ($CD_3COCD_3$): 21.2, 39.1, 55.6, 112.0, 114.8, 119.0, 124.0, 126.0, 129.5, 129.6, 130.5, 131.0, 133.1, 135.7, 139.1, 140.7, 145.4, 148.9, 161.2, 162.4.

1.71. 5-(3-Hydroxyphenyl)-*N*-methyl-*N*-m-tolylthiophene-2-carboxamide (71).

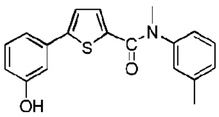

71

The title compound was prepared by reaction of 5-(3-methoxyphenyl)-*N*-methyl-*N*-*m*-tolylthiophene-2-carboxamide (70) (40 mg, 0.12 mmol) with borontrifluoride dimethyl sulfide complex (0.08 ml, 0.72 mmol) according to method C for 14 h. Purification by FC ($CH_2Cl_2/CH_3OH$ 100:1→50:1) yielded the title compound as colorless solid (30 mg, 79%); MS (ESI): 324 $(M+H)^+$; $^1H$ NMR ($CD_3COCD_3$): 2.36 (s, 3H), 3.37 (s, 3H), 6.52 (d, *J*= 4.1 Hz, 1H), 6.81 (ddd, *J*= 8.0, 2.5, 0.9 Hz, 1H), 7.03-7.06 (m, 2H), 7.08 (d, *J*= 4.1 Hz, 1H), 7.16 (d, *J*= 8.0 Hz, 1H), 7.20 (d, *J*= 7.6 Hz, 1H), 7.22 (s, 1H), 7.26 (d, *J*= 7.6 Hz, 1H), 7.36 (t, *J*= 8.0 Hz, 1H), 8.51 (s, 1H); $^{13}C$ NMR ($CD_3COCD_3$): 21.2, 39.1, 113.4, 116.4, 117.9, 123.7, 126.0, 129.5, 129.6, 130.5, 131.1, 133.2, 135.7, 138.8, 140.7, 145.4, 149.2, 158.8, 162.4.

1.72. 5-(2-Fluoro-3-methoxyphenyl)-*N*-methyl-*N*-(*m*-tolylthiophene)-2-carboxamide (72).

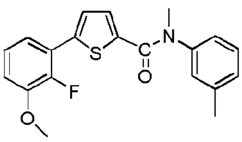

72

The title compound was prepared by reaction of 5-bromo-*N*-methyl-*N*-m-tolylthio-phene-2-carboxamide (70a) (46 mg, 0.15 mmol) and 2-fluoro-3-methoxyphenyl-boronic acid (31 mg, 0.18 mmol) with tetrakis(triphenylphosphine) palladium (17 mg, 0.015 mmol) as catalyst according to method B for 14 h. Purification by FC (*n*-hexane/ethyl acetate 10:1) afforded the desired compound as colourless solid (45 mg, 85%); MS (ESI): 356 $(M+H)^+$; $^1H$ NMR ($CD_3COCD_3$): 2.36 (s, 3H), 3.38 (s, 3H), 3.90 (s, 3H), 6.58 (dd, *J*= 4.0, 1.0 Hz, 1H), 7.08-7.19 (m, 4H), 7.21 (dd, *J*= 4.1, 1.0 Hz, 1H), 7.23-7.27 (m, 2H), 7.36 (t, *J*= 7.7 Hz, 1H); $^{13}C$ NMR ($CD_3COCD_3$): 21.2, 39.1, 56.7, 114.0, 118.8, 120.4, 122.7, 122.8, 125.4, 126.0, 126.9, 129.5, 129.7, 130.5, 132.6, 140.1, 140.8, 141.7, 145.3, 148.8, 149.5, 150.8, 162.3.

1.73. 5-(2-Fluoro-3-methoxyphenyl)-*N*-(4-methoxyphenyl)-*N*-methylthiophene-2-carboxamide (73).

5-Bromo-*N*-(4-methoxyphenyl)-*N*-methylthiophene-2-carboxamide (73a).

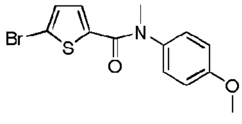

73a

The title compound was prepared by reaction of 5-bromothiophene-2-carbonyl chloride (113 mg, 0.5 mmol) and *N*-methyl-4-methoxyaniline (69 mg, 0.5 mmol) in presence of triethylamine (0.08 ml, 0.58 mmol) according to method A. Purification by FC (*n*-hexane/ethyl acetate 6:1) yielded the product as colorless solid (160 mg, 98%). MS (ESI): 326 $(M+H)^+$; $^1H$ NMR ($CD_3COCD_3$): 3.31 (s, 3H), 3.86 (s, 3H), 6.55 (d, *J*= 4.1 Hz, 1H), 6.93 (d, *J*= 4.1 Hz, 1H), 7.04 (d, *J*= 9.1 Hz, 2H), 7.30 (d, *J*= 9.1 Hz, 2H); $^{13}C$ NMR ($CD_3COCD_3$): 39.1, 55.9, 116.0, 118.4, 130.5, 130.9, 133.3, 137.3, 141.1, 160.8, 161.3.

5-(2-Fluoro-3-methoxyphenyl)-*N*-(4-methoxyphenyl)-*N*-methylthiophene-2-carboxamide (73).

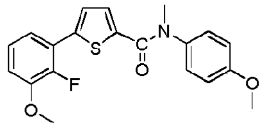

73

The title compound was prepared by reaction of 5-bromo-*N*-(4-methoxyphenyl)-*N*-methylthiophene-2-carboxamide (73a) (49 mg, 0.15 mmol) and 2-fluoro-3-methoxyphenylboronic acid (31 mg, 0.18 mmol) with tetrakis(triphenylphosphine) palladium (17 mg, 0.015 mmol) as catalyst according to method B for 14 h. Purification by FC (*n*-hexane/ethyl acetate 10:1→5:1) afforded the desired compound as colorless solid (40 mg, 72%); MS (ESI): 372 $(M+H)^+$; $^1H$ NMR ($CD_3COCD_3$): 3.35 (s, 3H), 3.85 (s, 3H), 3.90 (s, 3H), 6.62 (dd, *J*= 4.1, 1.3 Hz, 1H), 7.03 (d, *J*= 8.8 Hz, 2H), 7.08-7.18 (m, 3H), 7.23 (dd, *J*= 4.1, 0.9 Hz, 1H), 7.31 (d, *J*= 8.8 Hz, 2H); $^{13}C$ NMR ($CD_3COCD_3$): 39,2, 55.9, 56,7, 114.0, 115.8, 120.4, 120.5, 122.7, 122.8, 125.4, 125.5, 126.9, 127.0, 130.2, 132.6, 138.0, 140.1, 141.7, 141.8, 148.8, 149.5, 149.6, 150.8, 160.5, 162.4.

1.74. *N*-(2-fluorophenyl)-*N*-methyl-5-*m*-tolylthiophene-2-carboxamide (74) 5-Bromo-*N*-(2-fluorophenyl)-*N*-methylthiophene-2-carboxamide (74a).

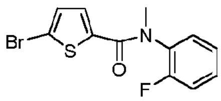

74a

The title compound was prepared by reaction of 5-bromothiophene-2-carbonyl chloride (225 mg, 1 mmol) and 2-fluoro-*N*-methylaniline (140 mg, 1 mmol) in presence of triethylamine (0.16 ml, 1.16 mmol) according to method A. Purification by FC (*n*-hexane/ethyl acetate 6:1) yielded the product as colorless oil (300 mg, 94%). MS (ESI): 314 $(M+H)^+$; $^1H$ NMR ($CD_3COCD_3$): 3.34 (s, 3H), 6.61 (d, *J*= 4.1 Hz, 1H), 6.96 (d, *J*= 4.1 Hz, 1H), 7.29-7.37 (m, 2H), 7.51-7.57 (m, 2H); $^{13}C$ NMR ($CD_3COCD_3$): 38.2, 117.8, 118.0, 118.4, 126.6, 131.2, 131.5, 131.7, 131.8, 132.1, 132.2, 132.8, 140.4, 158.3, 160.3, 161.8.

*N*-(2-fluorophenyl)-*N*-methyl-5-*m*-tolylthiophene-2-carboxamide (74).

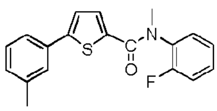

74

The title compound was prepared by reaction of 5-bromo-*N*-(2-fluorophenyl)-*N*-methylthiophene-2-carboxamide (74a) (47 mg, 0,15 mmol) and *m*-tolylboronic acid (25 mg, 0.18 mmol) with tetrakis(triphenylphosphine) palladium (17 mg, 0.015 mmol) as catalyst according to method B for 14 h. Purification by FC (*n*-hexane/ ethyl acetate 10:1→5:1) afforded the desired compound as colorless solid (42 mg, 86%); $^1H$ NMR ($CD_3COCD_3$): 2.34 (s, 3H), 3.27 (s, 3H), 6.69 (d, *J*= 4.1 Hz, 1H), 7.14-7.16 (m, 2H), 7.27 (t, *J*= 7.9 Hz, 1H), 7.28-7.37 (m, 3H), 7.41 (s, 1H), 7.48-7.53 (m, 1H), 7.55 (td, *J*= 7.9,1.9 Hz, 1H); $^{13}C$ NMR ($CD_3COCD_3$): 21.3, 38.2, 116.8, 117.7, 117.9, 123.8, 126.4, 126.5, 127.3, 129.9, 130.1, 131.2, 131.3, 131.4, 132.8, 132.9, 133.0, 134.2, 137.8, 139.7, 149.6, 158.3, 160.3, 162.9.

1.75. 5-(3-Methoxyphenyl)-*N*-methyl-*N*-(3-(trifluoromethyl)phenyl)thiophene-2-carboxamide (75).

5-Bromo-*N*-methyl-*N*-(3-(trifluoromethyl)phenyl)thiophene-2-carboxamide (75a).

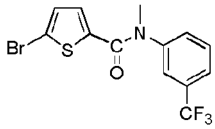

75a

The title compound was prepared by reaction of 5-bromothiophene-2-carbonyl chloride (225 mg, 1 mmol) and *N*-methyl-3-(trifluoromethyl)aniline (175 mg, 1 mmol) in presence of triethylamine (0.16 ml, 1.15 mmol) according to method A. Purification by FC (*n*-hexane/ethyl acetate 6:1) yielded the product as colorless solid (360 mg, 99%). MS (ESI): 366 $(M+H)^+$; $^1H$ NMR ($CD_3COCD_3$): 3.43 (s, 3H), 6.52 (d, *J*= 4.1 Hz, 1H), 6.95 (d, *J*= 4.1 Hz, 1H), 7.70-7.75 (m, 2H), 7.78-7.82 (m, 2H); $^{13}C$ NMR ($CD_3COCD_3$): 39.0, 118.4, 123.6, 125.7, 125.8, 125.9, 126.0, 131.2, 131.9, 132.4, 132.6, 133.1, 133.2, 140.9, 145.7, 161.5.

5-(3-Methoxyphenyl)-*N*-methyl-*N*-(3-(trifluoromethyl)phenyl)thiophene-2-carboxamide (75).

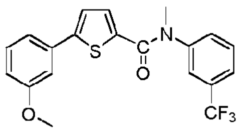

75

The title compound was prepared by reaction of 5-bromo-*N*-methyl-*N*-(3-(trifluoromethyl)phenyl)thiophene-2-carboxamide (75a) (36 mg, 0.1 mmol) and 3-methoxyphenylboronic acid (20 mg, 0.13 mmol) with tetrakis(triphenylphosphine) palladium (12 mg, 0.01 mmol) as catalyst according to method B for 6 h. Purification by FC (*n*-hexane/ethyl acetate 10:1→5:1) afforded the desired compound as colorless solid (35 mg, 90%); MS (ESI): 392 $(M+H)^+$; $^1H$ NMR ($CD_3COCD_3$): 3.47 (s, 3H), 3.83 (s, 3H), 6.57 (d, *J*= 4.1 Hz, 1H), 6.91 (ddd, *J*= 8.2, 2.5, 0.9 Hz, 1H), 7.11 (t, *J*= 2.2 Hz, 1H), 7.14 (ddd, *J*= 7.6, 1.6, 0.9 Hz, 1H), 7.19 (d, *J*= 4.1 Hz, 1H), 7.31 (t, *J*= 8.2 Hz, 1H), 7.69-7.78 (m, 3H), 7.81-7.82 (m, lah); $^{13}C$ NMR ($CD_3COCD_3$): 39.0, 55.6, 112.0, 115.0, 119.0, 124.1, 125.3, 125.4, 125.8, 125.9, 131.1, 131.7, 132.2, 132.5, 133.0, 133.5, 135.5, 138.2, 146.3, 149.2, 161.2, 162.6.

1.76. *N*-(Biphenyl-3-yl)-5-(3-methoxyphenyl)-*N*-methylthiophene-2-carboxamide (76).

*N*-methylbiphenyl-3-amine (76b).

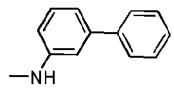

76b

3-Bromo-*N*-methylaniline (93 mg, 0.5 mmol), phenyl boronic acid (78 mg, 0.6 mmol), sodium carbonate (2 ml, 2N aqueous) and tetrakis(triphenylphosphine) palladium (58 mg, 0.05 mmol) in a DME (2 ml) solution was stirred at 80 °C for 6 hours under nitrogen. The reaction mixture was cooled to rt. The aqueous layer was extracted with dichloromethane. The combined organic layers were washed with brine, dried over sodium sulphate, filtered and concentrated to dryness. The product was purified by FC with hexane/ethyl acetate (15:1) as eluent, yielded as colorless oil (60 mg, 66%); MS (ESI): 184 $(M+H)^+$; $^1H$ NMR ($CD_3COCD_3$): 2.84 (s, 3H), 5.02 (br s, 1H), 6.60 (ddd, *J*= 8.1, 2.1, 0.9 Hz, 1H), 6.85-6.87 (m, 2H), 7.19 (t, *J*= 8.1 Hz, 1H), 7.30-7.33 (m, 1H), 7.42 (t, *J*= 7.6 Hz, 2H), 7.59-7.61 (m, 2H); $^{13}C$ NMR ($CD_3COCD_3$) : 30.5, 111.2, 112.0, 115.8, 127.7, 127.8, 129.5, 130.2, 142.7,142.9, 151.5.

*N*-(biphenyl-3-yl)-5-bromo-*N*-methylthiophene-2-carboxamide (76a).

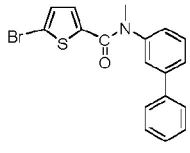

76a

The title compound was prepared from 5-bromothiophene-2-carbonyl chloride (62 mg, 0.27 mmol) and compound *N*-methylbiphenyl-3-amine (76b) (50 mg, 0.27 mmol), yielded as colorless solid (100 mg, 98%); MS (ESI): 372 $(M+H)^+$; $^1H$ NMR ($CD_3COCD_3$): 3.43 (s, 3H), 6.57 (d, *J*= 4.1 Hz, 1H), 6.92 (d, *J*= 4.1 Hz, 1H), 7.36-7.41 (m, 2H), 7.47 (t, *J*= 7.9 Hz, 2H), 7.59 (t, *J*= 7.9 Hz, 1H), 7.67-7.70 (m, 2H), 7.72 (t, *J*= 2.0 Hz, 1H), 7.76 (ddd, *J*= 7.9, 2.0, 0.9 Hz, 1H); $^{13}C$ NMR ($CD_3COCD_3$): 39.0, 118.3, 127.6, 127.7, 127.8, 127.9, 128.8, 129.8, 131.0, 131.3, 133.3, 140.5, 141.2, 143.7, 145.3, 161.3.

*N*-(Biphenyl-3-yl)-5-(3-methoxyphenyl)-*N*-methylthiophene-2-carboxamide (76).

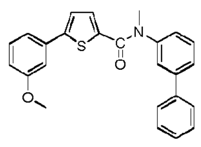

76

The title compound was prepared by reaction of *N*-(biphenyl-3-yl)-5-bromo-*N*-methylthiophene-2-carboxamide (76a) (37 mg, 0.1 mmol) and 3-methoxyphenylboronic acid (18 mg, 0.12 mmol) with tetrakis(triphenylphosphine) palladium (12 mg, 0.01 mmol) as catalyst according to method B for 14 h. Purification by FC (*n*-hexane/ethyl acetate 8:1) afforded the desired compound as colorless solid (38 mg, 95%); MS (ESI): 400 $(M+H)^+$; $^1H$ NMR ($CD_3COCD_3$): 3.46 (s, 3H), 3.81 (s, 3H), 6.65 (d, *J*= 4.0 Hz, 1H), 6.89 (dd, *J*= 8.0, 2.1 Hz, 1H), 7.09 (s, 1H), 7.12 (d, *J*= 7.6 Hz, 1H), 7.15 (d, *J*= 4.0 Hz, 1H), 7.28 (t, *J*= 8.0 Hz, 1H), 7.36-7.39 (m, 2H), 7.46 (t, *J*= 7.8 Hz, 2H), 7.57 (t, *J*= 7.8 Hz, 1H), 7.68 (d, *J*= 8.0 Hz, 2H), 7.71-7.74 (m, 2H); $^{13}C$ NMR ($CD_3COCD_3$): 39.0, 55.6, 112.0, 114.9, 119.0, 124.1, 127.3, 127.5, 127.7, 127.8, 128.7, 129.8, 131.0, 131.2, 133.3, 135.7, 138.8, 140.7, 143.6, 146.1, 149.0, 161.2, 162.5.

1.77. 5-(2-Fluoro-3-methoxyphenyl)-*N*-(3-methoxyphenyl)thiophene-2-carboxamide (77).

5-Bromo-*N*-(3-methoxyphenyl)thiophene-2-carboxamide (77a).

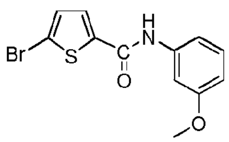

77a

The title compound was prepared by reaction of 5-bromothiophene-2-carbonyl chloride (113 mg, 0.5 mmol) and 3-methoxylaniline (62 mg, 0.5 mmol) in presence of triethylamine (0.08 ml, 0.58 mmol) according to method A. Purification by FC (n-hexane/ethyl acetate 10:1→6:1) yielded the product as colorless solid (150 mg, 96%); MS (ESI): 313 $(M+H)^+$; $^1H$ NMR ($CD_3COCD_3$): 3.79 (s, 3H), 6.70 (d, *J*= 8.0 Hz, 1H), 7.22-7.25 (m, 2H), 7.28 (d, *J*= 8.0 Hz, 1H), 7.47 (s, 1H), 7.71 (d, *J*= 4.1 Hz, 1H), 9.49 (br s, 1H); $^{13}C$ NMR ($CD_3COCD_3$): 55.5, 106.8, 110.4, 113.2, 118.8, 129.7, 130.4, 132.2, 140.8, 143.4, 159.7, 161.1.

5-(2-Fluoro-3-methoxyphenyl)-*N*-(3-methoxyphenynl)thiophene-2-carboxamide (77).

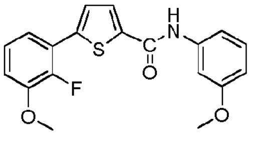

77

The title compound was prepared by reaction of 5-bromo-*N*-(3-methoxyphenyl)thiophene-2-carboxamide (77a) (62 mg, 0.2 mmol) and 2-fluoro-3-methoxyphenylboronic acid (38 mg, 0.24 mmol) with tetrakis(triphenylphosphine) palladium (23 mg, 0.02 mmol) as catalyst according to method B for 14 h. Purification by FC (*n*-hexane/ethyl acetate 6:1) afforded the desired compound as colorless solid (60 mg, 84%); MS (ESI): 358 $(M+H)^+$; $^1H$ NMR ($CD_3COCD_3$): 3.80 (s, 3H), 3.94 (s, 3H), 6.70 (ddd, *J*= 8.2, 2.5, 0.9 Hz, 1H), 7.17 (td, *J*= 8.2, 1.6 Hz, 1H), 7.21 (td, *J*= 7.6, 0.9 Hz, 1H), 7.24 (t, *J*= 8.2 Hz, 1H), 7.32-7.36 (m, 2H), 7.53 (t, *J*= 2.2 Hz, 1H), 7.57 (dd, *J*= 4.1, 1.0, Hz, 1H), 7.91 (dd, *J*= 4.1, 1.0 Hz, 1H), 9.52 (br s, 1H); $^{13}C$ NMR ($CD_3COCD_3$): 55.5, 56.8, 106.8, 110.3, 113.2, 114.3, 118.6, 120.6, 122.7, 122.8, 125.6, 127.9, 128.0, 129.5, 130.3, 141.1, 141.3, 141.4, 142.6, 149.0, 149.6, 149.7, 150.9, 160.6, 161.1.

1.78. 5-(2-Fluoro-3-methoxyphenyl)-*N*-(3-methoxyphenyl)-*N*-phenylthiophene-2-carboxamide (78).

5-Bromo-*N*-(3-methoxyphenyl)-*N*-phenylthiophene-2-carboxamide (78a).

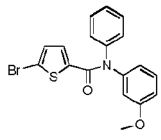

78a

The title compound was prepared by reaction of 5-bromothiophene-2-carbonyl chloride (225 mg, 1 mmol) and 3-methoxy-*N*-phenylaniline (199 mg, 1 mmol) in presence of triethylamine (0.08 ml, 0.58 mmol) according to method A. Purification by FC (*n*-hexane/ethyl acetate 6:1) yielded the product as colorless oil (220 mg, 57%); MS (ESI): 388 $(M+H)^+$; $^1H$ NMR ($CD_3COCD_3$): 3.79 (s, 3H), 6.67 (d, *J*= 4.1 Hz, 1H), 6.93 (ddd, *J*= 8.5, 2.0, 0.9 Hz, 1H), 6.96 (ddd, *J*= 7.9, 2.0, 0.9 Hz, 1H), 6.99 (d, *J*= 4.1 Hz, 1H), 7.01 (t, *J*= 2.0 Hz, 1H), 7.32-7.36 (m, 2H), 7.39-7.45 (m, 4H); $^{13}C$ NMR ($CD_3COCD_3$): 55.8, 113.9, 115.0, 119.1, 121.2, 128.2, 128.9, 130.2, 131.0, 131.2, 134.0, 141.6, 144.2, 145.2, 161.5.

5-(2-Fluoro-3-methoxyphenyl)-*N*-(3-methoxyphenyl)-*N*-phenylthiophene-2-carboxamide (78).

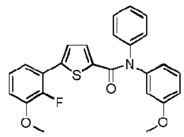

78

The title compound was prepared by reaction of 5-bromo-*N*-(3-methoxyphenyl)-*N*-phenylthiophene-2-carboxamide (78a) (39 mg, 0.1 mmol) and 2-fluoro-3-methoxyphenylboronic acid (20 mg, 0.12 mmol) with tetrakis(triphenylphosphine) palladium (12 mg, 0.01 mmol) as catalyst according to method B for 14 h. Purification by FC (*n*-hexane/ethyl acetate 6:1) afforded the desired compound as colorless solid (30 mg, 69%); MS (ESI): 434 $(M+H)^+$; $^1H$ NMR ($CD_3COCD_3$): 3.78 (s, 3H), 3.91 (s, 3H), 6.82 (dd, *J*= 4.1, 1.0 Hz, 1H), 6.92 (ddd, *J*= 8.5, 2.5, 0.9 Hz, 1H), 6.96 (ddd, *J*= 8.0, 2.0, 1.0 Hz, 1H), 7.01 (t, *J*= 2.0 Hz, 1H), 7.13 (dt, *J*= 8.0, 2.0 Hz, 1H), 7.16 (dt, *J*= 8.0, 1.0 Hz, 1H), 7.20-7.23 (m, 1H), 7.29 (dd, *J*= 4.1, 1.0 Hz, 1H), 7.31-7.35 (m, 2H), 7.39-7.45 (m, 4H); $^{13}C$ NMR ($CD_3COCD_3$): 55.8, 56.7, 113.6, 114.2, 115.0, 120.4, 120.5, 121.2, 125.5, 125.6, 127.0, 127.1, 127.9, 128.9, 130.1, 130.9, 133.4, 140.2, 140.3, 140.4, 142.4, 144.7, 145.7, 148.9, 149.5, 149.6, 150.9, 161.5, 162.7.

1.79. *N*-(3-Methoxyphenyl)-*N*-methyl-2-(3-methylphenyl)-1,3-thiazole-5-carboxamide (79).

2-Bromo-*N*-(3-methoxyphenyl)-*N*-methvl-1,3-thiazole-5-carboxamide (79a).

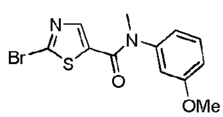

79a

A solution of 2-bromo-1,3-thiazole-5-carboxylic acid (416 mg, 2 mmol), thionyl chloride (0.58 ml, 4 mmol), 5 drops of DMF in dry toluene (10 ml) was heated at 110°C for 4 hours. After cooling, the solvent was removed under reduced pressure. This residue was diluted in dry $CH_2Cl_2$ and 3-methoxy-*N*-methylaniline (0.26 ml, 2 mmol) was added followed by triethylamine (0.28 ml, 2 mmol) according to method A and the solution was stirred at rt overnight under $N_2$ atmosphere. Purification on silica gel (*n*-hexane/ ethyl acetate 7:3) afforded the desired product as pale brown solid (393 mg, 60%); MS (ESI): 328 $(M+H)^+$; mp: 106°C; IR ($cm^{-1}$): 3098, 3059, 3014, 2966, 2943, 2838, 1633, 1582; $^1H$ NMR ($CD_3COCD_3$): 3.67 (s, 3H), 3.85 (s, 3H), 6.99 (ddd, *J* = 0.9, 1.95, 7.7 Hz, 1H), 7.05 (t, *J* = 2.3 Hz, 1H), 7.09 (ddd, *J* = 0.85, 2.5, 8.4 Hz, 1H), 7.18 (s, 1H), 7.42 - 7.46 (m, 1H); $^{13}C$ NMR ($CD_3COCD_3$): 38.6, 56.0, 114.9, 115.8, 121.3, 131.8, 136.8, 144.9, 145.7, 155.6, 159.9, 162.1.

*N*-(3-Methoxyphenyl)-*N*-methyl-2-(3-methylphenyl)-1,3-thiazole-5-carboxamide (79).

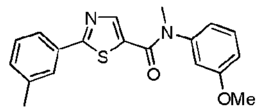

79

A solution of 2-bromo-*N*-(3-methoxyphenyl)-*N*-methyl-1,3-thiazole-5-carboxamide (79a) (200 mg, 0.61 mmol), *m*-tolyl boronic acid (105 mg, 0.77 mmol), caesium carbonate (596 mg, 1.83 mmol) and tetrakis(triphenylphosphine) palladium (14 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified on silica gel (*n*-hexane/ethyl acetate 7:3) and afforded the desired product as beige solid (170 mg, 82%); MS (ESI): 339 $(M+H)^+$; mp: 88°C; IR ($cm^{-1}$): 3065, 3005, 2924, 2841, 1626, 1588; $^1H$ NMR ($CD_3COCD_3$): 2.36 (s, 3H), 3.40 (s, 3H), 3.83 (s, 3H), 6.98 - 7.00 (m, 1H), 7.04 - 7.06 (m, 2H), 7.28-7.30 (m, 2H), 7.32 - 7.35 (m, 1H), 7.42 (dt, *J* = 0.7, 7.75 Hz, 1H), 7.64 - 6.66 (m, 1H), 7.70 - 7.71 (m, 1H); $^{13}C$ NMR ($CD_3COCD_3$): 21.2, 38.7, 55.9, 114.7, 115.2, 121.1, 124.5, 127.7, 129.9, 131.6, 132.4, 134.0, 134.7, 139.8, 145.8, 147.6, 161.2, 161.9, 171.6.

1.80. 2-(2-Fluoro-3-methoxyphenyl)-*N*-(3-hvdroxybenzyl)-*N*-methyl-1,3-thiazole-5-carboxamide (80).

2-Bromo-*N*-(3-methoxybenzyl)-*N*-methyl-1,3-thiazole-5-carboxamide (80b).

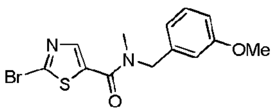

80b

A solution of 2-bromo-1,3-thiazole-5-carboxylic acid (416 mg, 2 mmol), thionyl chloride (0.58 ml, 4 mmol), 5 drops of DMF in dry toluene (10 ml) was heated at 110°C for 4 h. After cooling, the solvent was removed under reduced pressure. This residue was diluted in dry $CH_2Cl_2$ and 3-methoxy-*N*-methylbenzylamine (0.30 ml, 2 mmol) was added followed by triethylamine (0.28 ml, 2 mmol) according method A. The solution was stirred at rt overnight under $N_2$ atmosphere. Purification on silica gel (*n*-hexane/ ethyl acetate 7:3) afforded the desired product as yellow oil (530 mg, 78%); MS (ESI): 342 $(M+H)^+$; IR ($cm^{-1}$): 2934, 2835, 1611; $^1H$ NMR ($CD_3COCD_3$): 2.98-3.34 (m, 3H), 3.79 (s, 3H), 4.68-4.82 (m, 2H), 6.86 - 6.93 (m, 3H), 7.63 - 8.14 (m, 1H); $^{13}C$ NMR ($CD_3COCD_3$): 37.1, 52.4, 55.5, 113.7, 114.5, 119.4, 120.9, 130.7, 139.5, 143.2, 154.7, 161.1, 161.5.

2-Bromo-*N*-(3-hydroxybenzyl)-*N*-methyl-1,3-thiazole-5-carboxamide (80a).

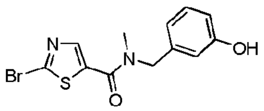

80a

The title compound was prepared by reaction of 2-bromo-*N*-(3-methoxybenzyl)-*N*-methyl-1,3-thiazole-5-carboxamide (80b) (530 mg, 1.55 mmol) with borontrifluoride dimethyl sulphide complex (0.98 ml, 9.32 mmol) according to method C for 2 h at 0°C and 1 h at rt. The product was purified on MP-LC (n-hexane/ethyl acetate 100:0 to 80:20, 15 min, 80:20 to 60:40, 35 min) and afforded the desired product as pale red oil (236 mg, 47%); MS (ESI): 328 $(M+H)^+$; IR $(cm^{-1})$: 3286, 3101, 2969, 2928, 2871, 1731, 1702, 1603; $^1H$ NMR $(CD_3COCD_3)$: 2.99-3.34 (m, 3H), 4.61-4.82 (m, 2H), 6.72 - 6.92 (m, 3H), 7.60-8.13 (m, 1H), 8.40 - 8.55 (brs, 1H).

2-(2-Fluoro-3-methoxyphenyl)-*N*-(3-hydroxybenzyl)-*N*-methyl-1,3-thiazole-5-carboxamide (80).

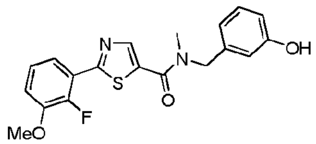

80

A solution of 2-bromo-*N*-(3-hydroxybenzyl)-*N*-methyl-1,3-thiazole-5-carboxamide (80a) (104 mg, 0.32 mmol), 2-fluoro-3-methoxyphenyl boronic acid (81 mg, 0.48 mmol), caesium carbonate (311 mg, 0.95 mmol) and tetrakis(triphenylphosphine) palladium (7 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified by MP-LC (*n*-hexane/ethyl acetate 100:0 to 60:40, 60 min). Trituration of the residue in a little amount of cold acetonitrile afforded the desired product as beige solid (83 mg, 70%); MS (ESI): 373 $(M+H)^+$; mp: 140°C; IR $(cm^{-1})$: 3220, 3029, 2993, 2931, 2838, 1737, 1591; $^1H$ NMR $(CD_3COCD_3)$: 2.82 (s, 1H), 3.2 (br s, 3H), 3.97 (s, 3H), 4.76 (br s, 2H), 6.77 - 6.85 (m, 3H), 7.22 (t, *J* = 7.55 Hz, 1H), 7.26 - 7.32 (m, 2H), 7.82 - 7.87 (br s, 1H), 8.33 - 8.40 (br s, 1H); $^{13}C$ NMR $(CD_3COCD_3)$: 56.9, 104.4, 115.3, 115.4, 116.12, 116.14, 120.1, 122.26, 122.35, 125.57, 125.60, 139.7, 149.3, 149.4, 150.0, 152.0, 158.8, 162.9.

1.81. *N*-(3-Methoxyphenyl)-*N*-methyl-5-(3-methylphenyl)-1,3-thiazole-2-carboxamide (81).

5-(3-Methylphenyl)-1,3-thiazole (81b).

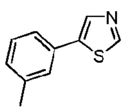

81b

A solution of 5-bromo-1,3-thiazole (1 g, 6.10 mmol), *m*-tolyl boronic acid (1.244 g, 9.14 mmol), caesium carbonate (5.962 g, 18.30 mmol) and tetrakis(triphenylphosphine) palladium (140 mg, 0.02 eq) in oxygen free DME/EtOH/water (1:1:1) (6 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified on silica gel (*n*-hexane/ethyl acetate 7:3) and afforded the desired product as colourless oil (1.06 g, 99%); MS (ESI): 176 $(M+H)^+$; IR $(cm^{-1})$: 3086, 3026, 2957, 2922, 2862, 1605; $^1H$ NMR $(CD_3COCD_3)$: 2.37 (s, 3H), 7.17 - 7.20 (m, 1H), 7.32 (t, *J* = 7.7 Hz, 1H), 7.45 - 7.48 (m, 1H), 7.50 - 7.52 (m, 1H), 8.19 (s, 1H), 8.93 (s, 1H); $^{13}C$ NMR $(CD_3COCD_3)$: 21.4, 124.8, 128.3, 129.99, 130.01, 132.1, 139.8, 140.1, 153.2.

Lithium 5-(3-methylphenyl)-1,3-thiazole-2-carboxylate (81a).

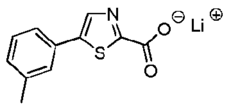

81a

To a solution of 5-(3-methylphenyl)-1,3-thiazole (81b) (1.05 g, 6 mmol) in dry THF (50 ml) was added drop wise *n*-BuLi (2.88 ml, 7.21 mmol, 2.5M in hexane) at-78°C under $N_2$ atmosphere. The reaction mixture was kept at -78°C for 1 h and warmed up to -5°C over 3 h. The reaction mixture was cooled at -78°C and dry carbon dioxide gas was bubbled through the solution for 3 h. The reaction mixture was warmed up to rt keeping $CO_2$ gas bubbling overnight. Solvent was removed under reduced pressure (bath temperature of rotavapor at 20°C). The residue was triturated with diethylether and filtered to afford the desired compound as a pale brown solid (977 mg, 72%); MS (ESI): 220 $(M+H)^+$; IR ($cm^{-1}$): 3301, 3095, 3032, 2960, 2922, 2868, 1737, 1618; $^1H$ NMR ($CD_3SOCD_3$): 2.34 (s, 3H), 7.16 (d, *J* = 7.2 Hz, 1H), 7.31 (t, *J* = 7.45 Hz, 1H), 7.46 (d, *J* = 7.2 Hz, 1H), 7.50 (s, 1H), 8.12 (s, 1H); $^{13}C$ NMR ($CD_3SOCD_3$): 20.9, 123.6, 126.9, 128.99, 129.06, 131.2, 138.5, 141.0, 161.7, 170.3.

*N*-(3-Methoxyphenyl)-*N*-methyl-5-(3-methylphenyl)-1,3-thiazole-2-carboxamide (81).

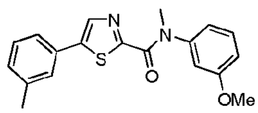

81

To a solution of lithium 5-(3-methylphenyl)-1,3-thiazole-2-carboxylate (81a) (450 mg, 2 mmol) in $CH_2Cl_2$ (15 ml) was added drop wise oxalyl chloride (0.34 ml, 4 mmol) followed by few drops of DMF at 0°C under $N_2$ atmosphere. The reaction mixture was stirred at 0°C for 10 min and at rt for 3 h. Then, the solvent was removed under reduced pressure (bath temperature of rotavapor at 20°C). The residue was diluted in dry $CH_2Cl_2$ and 3-methoxy-*N*-methylaniline (0.26 ml, 2 mmol) was added followed by triethylamine (0.28 ml, 2 mmol). The solution was stirred at rt overnight under $N_2$ atmosphere. The solvent was removed under reduced pressure (bath temperature of rotavapor at 20°C). Aqueous $Na_2CO_3$ 2N (15 ml) was added followed by ethyl acetate (15 ml). The aqueous layer was extracted with ethyl acetate. The organic layer was washed twice with $Na_2CO_3$ 2N (15 ml), once with water (15 ml), dried over $MgSO_4$, filtered and concentrated under reduced pressure. Purification on silica gel (*n*-hexane/ethyl acetate 7:3) afforded the desired product as yellow solid (520 mg, 77%); MS (ESI): 339 $(M+H)^+$; mp: 85°C; IR ($cm^{-1}$) : 3095, 3014, 2975, 2919, 2838, 1746, 1633, 1588; $^1H$ NMR ($CD_3COCD_3$): 2.36 (s, 3H), 3.54 (s, 2H), 3.78 (s, 3H), 6.87 (ddd, *J* = 0.6, 2.5, 8.25 Hz, 2H), 6.92 (t, *J* = 2.1 Hz, 1H), 7.20 - 7.22 (m, 1H), 7.25 - 7.29 (m, 1H), 7.30-7.34 (m, 1H), 7.43 - 7.47 (m, 1H), 7.48 - 7.50 (m, 1H), 7.91 - 7.96 (m, 1H); $^{13}C$ NMR ($CD_3COCD_3$): 21.3, 39.5, 55.7, 113.6, 113.8, 120.1, 124.87, 124.93, 128.3, 128.4, 130.1, 130.55, 130.62, 131.5, 139.7, 139.9, 144.3, 146.6, 161.2.

1.82. *N*-Cyclopropyl-*N*-(3-methoxyphenyl)-5-(3-methylphenyl)thiophene-2-carboxamide (82).

5-Bromo-*N*-cyclopropyl-*N*-(3-methoxyphenyl)thiophene-2-carboxamide (82a).

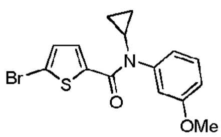

82a

In a sealed tube was introduced 1-bromo-3-methoxybenzene (374 mg, 2 mmol), sodium *tert*-butoxide (288 mg, 3 mmol), 2,2'-bis(diphenylphosphino)-1,1'-binaphthyl (38 mg, 0.06 mmol), cyclopropylamine (183 mg, 3.2 mmol), tris(dibenzylideneacetone)dipalladium(0) (18 mg, 0.02 mmol) and dry toluene (2-3 ml). The tube was flushed few seconds

with $N_2$, closed and wrapped with aluminum foil. The reaction mixture was heated at 80°C overnight. After cooling, diethyl ether was added to quench the reaction and the solution was filtered on celite. The solvent was removed under reduced pressure and the product was used in the next step without further purification.

To a solution of freshly prepared 5-bromothiophene-2-carbonyl chloride (2 mmol) (prepared from 5-bromothiophene-2-carboxylic acid (414 mg, 2 mmol), thionyl chloride (0.58 ml, 4 mmol) in dry toluene (10 ml) and catalytic amount of DMF) was added the freshly synthesized *N*-cyclopropyl-3-methoxyaniline (2 mmol) diluted in little amount of dry $CH_2Cl_2$, followed by triethylamine (0.28 ml, 2 mmol) according to method A for 24 h. Purification on silica gel (*n*-hexane/ethyl acetate 7:3) afforded the desired product as yellow solid (343 mg, 49%); MS (ESI): 353 $(M+H)^+$; mp: 60°C; IR ($cm^{-1}$): 3086, 3014, 2946, 2841, 1740, 1630, 1597; $^1H$ NMR ($CD_3COCD_3$): 0.58 - 0.62 (m, 2H), 0.78 - 0.82 (m, 2H), 3.28 - 3.32 (m, 1H), 3.82 (s, 3H), 6.66 (d, *J* = 4.1 Hz, 1H), 6.86 (ddd, *J* = 0.95, 1.9, 7.85 Hz, 1H), 6.91 (t, *J* = 2.3 Hz, 1H), 6.95 (d, *J* = 4.1 Hz, 1H), 7.03 (ddd, *J* = 0.9, 2.6, 8.45 Hz, 1H), 7.36 - 7.39 (m, 1H); $^{13}C$ NMR ($CD_3COCD_3$): 7.8, 33.1, 55.9, 115.1, 116.0, 118.5, 122.5, 131.1, 132.9, 142.0, 143.3, 161.6, 162.6.

*N*-Cyclopropyl-*N*-(3-methoxyphenyl)-5-(3-methylphenyl)thiophene-2-carboxamide (82).

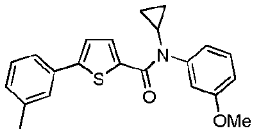

82

A solution of 5-bromo-*N*-cyclopropyl-*N*-(3-methoxyphenyl)thiophene-2-carboxamide (82a) (205 mg, 0.58 mmol), *m*-tolyl boronic acid (118 mg, 0.87 mmol), caesium carbonate (570 mg, 1.75 mmol) and tetrakis(triphenylphosphine) palladium (14 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified on MP-LC (*n*-hexane/ethyl acetate 100:0 to 80:20, 10 min, 80:20 to 60:50, 45 min) and afforded the desired product as yellow solid (181 mg, 86%); MS (ESI): 364 $(M+H)^+$; mp: 68°C; IR ($cm^{-1}$): 3092, 3014, 2972, 2922, 2835, 1737, 1702, 1627, 1600; $^1H$ NMR ($CD_3COCD_3$): 0.61 - 0.64 (m, 2H), 0.79 - 0.83 (m, 2H), 2.33 (s, 3H), 3.31 - 3.35 (m, 1H), 3.81 (s, 3H), 6.73 (d, *J* = 4 Hz, 1H), 6.88 (ddd, *J* = 0.9, 1.95, 7.7 Hz, 1H), 6.92 (t, *J* = 2.3 Hz, 1H), 6.99 (ddd, *J* = 0.9, 2.55, 8.45 Hz, 1H), 7.13 - 7.14 (m, 1H), 7.15 (d, *J* = 4 Hz, 1H), 7.25 - 7.28 (m, 1H), 7.34 - 7.38 (m, 1H), 7. 37 - 7.39 (m, 1H), 7.42-7.43 (m, 1H); $^{13}C$ NMR ($CD_3COCD_3$): 8.1, 21.3, 33.2, 55.8, 114.5, 115.8, 122.3, 123.7, 123.8, 127.2, 129.8, 129.9, 130.9, 132.9, 134.4, 139.5, 139.6, 144.0, 149.3, 161.5, 163.7.

1.83. *N*-Cyclopropyl-5-(2-fluoro-3-methoxyphenyl)-*N*-(3-hydroxybenzyl)thiophene-2-carboxamide (83).

5-Bromo-*N*-cyclopropyl-*N*-(3-methoxybenzyl)thiophene-2-carboxamide (83b).

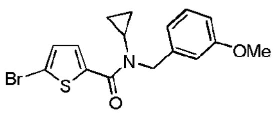

83b

In a sealed tube was introduced 1-(bromomethyl)-3-methoxybenzene (402 mg, 2 mmol), sodium tert-butoxide (288 mg, 3 mmol), 2,2'-bis(diphenylphosphino)-1,1'-binaphthyl (38 mg, 0.06 mmol), cyclopropylamine (183 mg, 3.2 mmol), tris(dibenzylideneacetone)dipalladium(0) (18 mg, 0.02 mmol) and dry toluene (2-3 ml). The tube was flushed few seconds with $N_2$, closed and wrapped with aluminum foil. The reaction mixture was heated at 80°C overnight. After cooling, diethyl ether was added to quench the reaction and the solution was filtered on celite. The solvent was removed under reduced pressure and the product was used for the next step without further purification.

To a solution of freshly prepared 5-bromothiophene-2-carbonyl chloride (2 mmol) (prepared from 5-bromothiophene-2-carboxylic acid (414 mg, 2 mmol), thionyl chloride (0.58 ml, 4 mmol) in dry toluene (10 ml) and catalytic amount of DMF) was added the freshly synthesized *N*-(3-methoxybenzyl)cyclopropanamine (2 mmol) diluted in little amount of dry $CH_2Cl_2$, followed by triethylamine (0.28 ml, 2 mmol) according to method A for 24 h. Purification on silica gel

($n$-hexane/ethyl acetate 7:3) afforded the desired product as yellow oil (302 mg, 41%); MS (ESI): 367 $(M+H)^+$; IR ($cm^{-1}$): 3089, 3008, 2946, 2838, 1737, 1600; $^1H$ NMR ($CD_3COCD_3$): 0.78 - 0.81 (m, 2H), 0.88 - 0.92 (m, 2H), 2.98 - 3.04 (m, 1H), 3.77 (s, 3H), 4.74 (s, 2H), 6.82 - 6.85 (m, 1H), 6.88 - 6.91 (m, 2H), 7.18 (d, $J$ = 4.05 Hz, 1H), 7.23 - 7.27 (m, 1H), 7.61 (d, $J$ = 4.05 Hz, 1H); $^{13}C$ NMR ($CD_3COCD_3$): 11.3, 32.0, 51.9, 55.4, 113.2, 114.1, 117.9, 120.5, 130.4, 131.5, 132.3, 141.0, 142.5, 160.9, 164.1.

5-Bromo-*N*-cyclopropyl-*N*-(3-hydroxybenzyl)thiophene-2-carboxamide (83a).

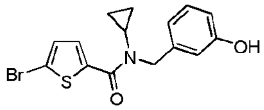

83a

The title compound was prepared by reaction of 5-bromo-N-cyclopropyl-N-(3-methoxybenzyl)thiophene-2-carboxamide (83b) (278 mg, 0.76 mmol) with borontrifluoride dimethyl sulphide complex (0.48 ml, 4.56 mmol) according to method C for 2 h at 0°C and overnight at rt. The product as brown solid (270 mg, 100%) was analytically pure and used in the next step without further purification; MS (ESI): 353 $(M+H)^+$; mp: 128°C; IR ($cm^{-1}$): 3268, 3098, 3002, 2975, 2928, 2429, 1740, 1603, 1570; $^1H$ NMR ($CD_3COCD_3$): 0.77 - 0.81 (m, 2H), 0.87 - 0.92 (m, 2H), 2.96 - 3.02 (m, 1H), 4.70 (s, 2H), 6.73 (ddd, $J$ = 0.85, 2.65, 7.8 Hz, 1H), 6.78 - 6.80 (m, 2H), 7.13 - 7.17 (m, 1H), 7.18 (d, $J$ = 4.05 Hz, 1H), 7.60 (d, $J$ = 4.05 Hz, 1H), 8.28 (br s, 1H); $^{13}C$ NMR ($CD_3COCD_3$): 11.3, 31.9, 51.9, 114.9, 115.1, 117.9, 119.5, 130.4, 131.5, 132.3, 140.9, 142.5, 158.5, 164.1.

*N*-Cyclopropyl-5-(2-fluoro-3-methoxyphenyl)-*N*-(3-hydroxybenzyl)thiophene-2-carboxamide (83).

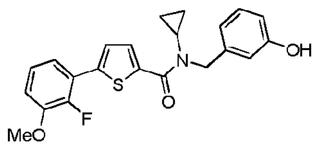

83

A solution of 5-bromo-*N*-cyclopropyl-*N*-(3-hydroxybenzyl)thiophene-2-carboxamide (83a) (104 mg, 0.32 mmol), 2-fluoro-3-methoxyphenyl boronic acid (81 mg, 0.48 mmol), cesium carbonate (311 mg, 0.95 mmol) and tetrakis(triphenylphosphine) palladium (7 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified by HPLC preparative (acetonitrile/water 40:60 to 100:0, solvent containing 1ml of TFA for 1l of solvent) and afforded the desired product as beige solid (41 mg, 37%); MS (ESI): 398 $(M+H)^+$; mp: 150°C; IR ($cm^{-1}$): 3253, 3008, 2972, 2937, 2841, 2426, 1740, 1600, 1570; $^1H$ NMR ($CD_3COCD_3$): 0.77 - 0.80 (m, 2H), 0.87 - 0.92 (m, 2H), 3.00 - 3.06 (m, 1H), 3.94 (s, 3H), 4.74 (s, 2H), 6.73 - 6.76 (m, 1H), 6.81 - 6.84 (m, 2H), 7.13 - 7.22 (m, 3H), 7.32 - 7.35 (m, 1H), 7.52 (dd, $J$ = 1.0, 4.05 Hz, 1H), 7.78 (m, 1H); $^{13}C$ NMR ($CD_3COCD_3$): 11.2, 32.1, 51.9, 56.7, 114.07, 114.08, 114.9, 115.1, 119.5, 120.57, 120.58, 122.8, 122.9, 125.5, 125.6, 127.2, 127.3, 130.4, 131.8, 140.67, 140.71, 141.1, 141.29, 141.31, 148.9, 149.6, 149.7, 150.9, 158.5, 165.1.

1.84. *N*-(3-Methoxyphenyl)-*N*-methyl-5-(3-methylphenyl)thiophene-2-sulfonamide (84).

5-Bromo-*N*-(3-methoxyphenyl)-*N*-methylthiophene-2-sulfonamide (84a).

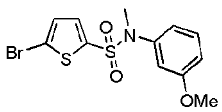

84a

To a solution of 3-methoxy-*N*-methylaniline (137 mg, 1 mmol) in $CH_2Cl_2$ (3 ml) was added NaOH 50% (1 ml) under stirring followed by tetrabutyl ammonium hydrogen sulphate (51 mg, 0.15 mmol). After few minutes, 5-bromothiophene-2-sulfonyl chloride (262 mg, 1 mmol) was added to the reaction mixture. The solution was stirred at rt for 5 h, then water (10 ml) was added to quench the reaction followed by ethyl acetate (10 ml). The aqueous layer was extracted twice with ethyl acetate. The organic layer was dried over $MgSO_4$, filtered and concentrated under reduced pressure. The product was purified on silica gel (*n*-hexane/ethyl acetate 8:2) and afforded the desired compound as yellowish oil (277 mg, 77%); IR ($cm^{-1}$): 3101, 2938, 2835, 1734, 1602, 1354; $^1H$ NMR ($CD_3COCD_3$): 3.27 (s, 3H), 3.77 (s, 3H), 6.78 - 6.81 (m, 2H), 6.90 - 6.92 (m, 1H), 7.26 - 7.30 (m, 2H), 7.31 (d, *J* = 4.1 Hz, 1H); $^{13}C$ NMR ($CD_3COCD_3$): 38.7, 55.8, 113.5, 114.2, 119.3, 120.1, 130.5, 132.2, 134.3, 138.9, 143.2, 161.0.

*N*-(3-Methoxyphenyl)-*N*-methyl-5-(3-methylphenyl)thiophene-2-sulfonamide (84).

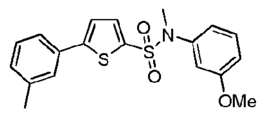

84

A solution of 5-bromo-*N*-(3-methoxyphenyl)-*N*-methylthiophene-2-sulfonamide (84) (142 mg, 0.39 mmol), *m*-tolyl boronic acid (80 mg, 0.59 mmol), caesium carbonate (385 mg, 1.18 mmol) and tetrakis(triphenylphosphine) palladium (10 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified on silica gel (*n*-hexane/ethyl acetate 8:2) and afforded the desired product as yellow oil (147 mg, 99%); MS (ESI): 374 $(M+H)^+$; IR ($cm^{-1}$): 3101, 3008, 2925, 2838, 1708, 1702, 1602; $^1H$ NMR ($CD_3COCD_3$): 2.38 (s, 3H), 3.29 (s, 3H), 3.75 (s, 3H), 6.80 (dd, *J* = 0.9, 2.15 Hz, 1H), 6.81 - 6.82 (m, 1H), 6.89 (ddd, *J* = 0.8, 2.5, 8.4 Hz, 1H), 7.23 - 7.28 (m, 2H), 7.33 - 7.36 (m, 1H), 7.39 (d, *J* = 4.0 Hz, 1H), 7.48 - 7.49 (m, 1H), 7.50 (d, *J* = 4.0 Hz, 1H), 7.53-7.54 (m, 1H); $^{13}C$ NMR ($CD_3COCD_3$): 21.3, 38.8, 55.7, 113.5, 114.0, 119.3, 124.1, 124.3, 127.6, 130.1, 130.4, 130.8, 133.5, 134.7, 135.9, 140.0, 143.6, 152.0, 160.9.

1.85. 5-(2-Fluoro-3-methoxyphenyl)-*N*-(3-hydroxybenzyl)-*N*-methylthiophene-2-sulfonamide (85).

5-Bromo-*N*-(3-methoxybenzyl)-*N*-methylthiophene-2-sulfonamide (85b).

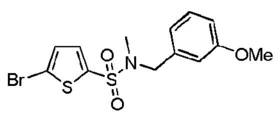

85b

To a solution of 3-methoxy-*N*-methylbenzylamine (302 mg, 2 mmol) in $CH_2Cl_2$ (6 ml) was added NaOH 50% (2 ml) under stirring followed by tetrabutyl ammonium hydrogen sulphate (102 mg, 0.30 mmol). After few minutes, 5-bromothiophene-2-sulfonyl chloride (524 mg, 2 mmol) was added to the reaction mixture. The solution was stirred at rt for 5 h, then water (10 ml) was added to quench the reaction followed by ethyl acetate (10 ml). The aqueous layer was extracted twice with ethyl acetate. The organic layer was dried over $MgSO_4$, filtered and concentrated under reduced pressure. The product was purified on silica gel (*n*-hexane/ethyl acetate 8:2) afforded the desired compound as yellowish oil (620 mg, 82%); IR ($cm^{-1}$): 3101, 2937, 2835, 1740, 1600, 1345; $^1H$ NMR ($CD_3COCD_3$): 2.70 (s, 3H), 3.80 (s, 3H), 4.21 (s, 2H), 6.88 - 6.91 (m, 1H), 6.92 - 6.95 (m, 2H), 7.28 - 7.31 (m, 1H), 7.38 (d, *J* = 4.05 Hz, 1H), 7.52 (d, *J* = 4.05 Hz, 1H); $^{13}C$ NMR ($CD_3COCD_3$): 35.0, 54.7, 55.5, 114.2, 114.7, 119.7, 121.3, 130.6, 132.5, 133.7, 138.3, 140.0, 161.0.

5-Bromo-*N*-(3-hydroxybenzyl)-*N*-methylthiophene-2-sulfonamide (85a).

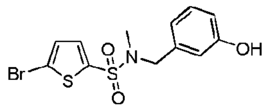

85a

The title compound was prepared by reaction of 5-bromo-N-(3-methoxybenzyl)-N-methylthiophene-2-sulfonamide (85a) (528 mg, 1.40 mmol) with borontrifluoride dimethyl sulphide complex (0.88 ml, 8.42 mmol) according to method C overnight at rt. The solvent was removed under reduced pressure and the residue was triturated in water. The precipitate formed was filtered to give the desired product as yellow solid (445 mg, 88%); mp: 106°C; IR ($cm^{-1}$): 3438, 3104, 3026, 2981, 2931, 1600, 1337; $^1H$ NMR ($CD_3COCD_3$): 2.69 (s, 3H), 4.16 (s, 2H), 6.80 (ddd, J = 0.8, 2.55, 8.95 Hz, 1H), 6.81 - 6.84 (m, 1H), 6.86 - 6.88 (m, 1H), 7.18 - 7.21 (m, 1H), 7.38 (d, *J* = 3.95 Hz, 1H), 7.51 (d, *J* = 3.95 Hz, 1H), 8.38 (s, 1H); $^{13}C$ NMR ($CD_3COCD_3$): 35.0, 54.7, 115.8, 116.0, 119.6, 120.3, 130.6, 132.4, 133.6, 138.3, 140.0, 158.7.

5-(2-Fluoro-3-methoxyphenyl)-*N*-(3-hydroxybenzyl)-*N*-methylthiophene-2-sulfonamide (85).

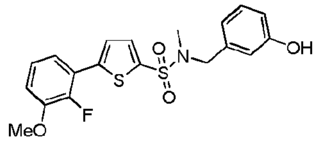

85

A solution of 5-bromo-*N*-(3-hydroxybenzyl)-*N*-methylthiophene-2-sulfonamide (85a) (200 mg, 0.55 mmol), 2-fluoro-3-methoxyphenyl boronic acid (141 mg, 0.83 mmol), caesium carbonate (540 mg, 1.66 mmol) and tetrakis(triphenylphosphine) palladium (13 mg, 0.02 eq) in oxygen free DME/EtOH/water (1:1:1) (3 mL) was heated under microwave irradiation at 150°C (150 W, 5 bar) for 15 min. The reaction mixture was cooled to rt and quenched with ethyl acetate. The aqueous layer was extracted with ethyl acetate. The combined organic layers were washed with brine, dried over magnesium sulphate, filtered and concentrated under reduced pressure. The residue was purified by HPLC preparative (acetonitrile/water 40:60 to 100:0, solvent containing 1ml of TFA for 1l of solvent) and afforded the desired product as colourless oil (42 mg, 19%); MS (ESI): 408 $(M+H)^+$; IR ($cm^{-1}$): 3438, 3107, 3029, 2981, 2928, 2856, 1725, 1600, 1337; $^1H$ NMR ($CD_3COCD_3$): 2.72 (s, 3H), 3.95 (s, 3H), 4.19 (s, 2H), 6.80 (ddd, *J* = 0.85, 2.5, 8.25 Hz, 1H), 6.83 - 6.85 (m, 1H), 6.89 - 6.90 (m, 1H), 7.17 - 7.27 (m, 3H), 7.37 - 7.40 (m, 1H), 7.67 (dd, J = 0.55, 4.05 Hz, 1H), 7.71 (dd, J = 1.25, 4.0 Hz, 1H); $^{13}C$ NMR ($CD_3COCD_3$): 35.1, 54.7, 56.8, 114.8, 115.7, 115.9, 120.3, 120.5, 121.9, 125.8, 127.7, 130.5, 133.3, 138.5, 143.7, 148.9, 149.6, 150.9, 158.6.

Example 2. Biological Methods.

[2,4,6,7-$^3$H]-E1 and [2,4,6,7-$^3$H]-E2 were purchased from Perkin Elmer, Boston. Quickszint Flow 302 scintillator fluid was bought from Zinsser Analytic, Frankfurt. Other chemicals were purchased from Sigma, Roth or Merck.

2.1 17β-HSD2 and 17β-HSD1 enzyme preparation from human placental enzyme. 17β-HSD2 and 17β-HSD1 were obtained from human placenta according to previously described procedures (Kruchten, P. et al., Mol. Cell. Endocrinol., 301: 154-159 (2009)). Fresh human placenta was homogenized. Cytosolic and microsomal fractions were separated by centrifugation at 1000 g, 10000 g and 150000 g. 17β-HSD2 was obtained directly from the microsomal fraction. For the partial purification of 17β-HSD1, the cytosolic fraction was precipitated with ammonium sulfate. Aliquots containing 17β-HSD1 or 17β-HSD2 were stored frozen.

2.2 Inhibition of 17β-HSD2 in cell-free assay. Inhibitory activities were evaluated by an established method with minor modifications (Kruchten, P. et al., Mol. Cell. Endocrinol., 301:154-159 (2009)). Briefly, the enzyme preparation was incubated with $NAD^+$ [1500 μM] in the presence of potential inhibitors at 37 °C in a phosphate buffer (50 mM) supplemented with 20% of glycerol and EDTA 1mM. Inhibitor stock solutions were prepared in DMSO. Final concentration of DMSO was adjusted to 1% in all samples. The enzymatic reaction was started by addition of a mixture of unlabelled- and [$^3$H]- E2 (final concentration: 500 nM, 0.11 μCi). After 20 min at 37°C, the incubation was stopped with $HgCl_2$ and the mixture was extracted with ether. After evaporation, the steroids were dissolved in acetonitrile. E1 and E2 were separated using acetonitrile/water (45:55) as mobile phase in a C18 RP chromatography column

(Nucleodur C18, 3μm, Macherey-Nagel, Düren) connected to a HPLC-system (Agilent 1100 Series, Agilent Technologies, Waldbronn). Detection and quantification of the steroids were performed using a radioflow detector (Berthold Technologies, Bad Wildbad). The conversion rate was calculated according to the following equation: $\%conversion = \frac{\%E1}{\%E1 + \%E2} \bullet 100$. Each value was calculated from at least three independent experiments.

2.3 Inhibition of 17β-HSD1 in cell-free assay. The 17β-HSD1 inhibition assay was performed similarly to the 17β-HSD2 test. The cytosolic fraction was incubated with NADH [500 μM], test compound and a mixture of unlabelled- and [$^3$H]-E1 (final concentration: 500 nM, 0.15 μCi) for 10 min at 37°C. Further treatment of the samples and HPLC separation was carried out as mentioned above for 17β-HSD2.

2.4 Inhibition of 17β-HSD2 in a cellular assay. Cellular 17β-HSD2 activity is measured using the breast cancer cell-line MDA-MB-23 (17β-HSD1 activity negligible). [$^3$H-E2] (200 nM) is used as substrate, and is incubated with the inhibitor for 3.5 h at 37°C. After ether extraction, substrate and product are separated by HPLC and detected with a radioflow detector following the method previously described. Potency is evaluated as percentage of inhibition (inhibitor concentration: 1 μM) as well as $IC_{50}$ values as previously mentioned in the 17β-HSD2 cell-free assay.

2.5 ER affinity assay. The binding affinity of selected compounds to the ERα and ERβ was determined according to Zimmermann et al. (Zimmermann, J. et al., J. Steroid Biochem. Mol. Biol., 94:57-66 (2005)) using recombinant human proteins. Briefly, 0.25 pM of ERα or ERβ, respectively, were incubated with [$^3$H]-E2 (10 nM) and test compound for 1 h at room temperature. The potential inhibitors were dissolved in DMSO (5% final concentration). Evaluation of non-specific-binding was performed with diethylstilbestrol (10 μM). After incubation, ligand-receptor complexes were selectively bound to hydroxyapatite (5 g/ 60 ml TE-buffer). The formed complex was separated, washed and resuspended in ethanol. For radiodetection, scintillator cocktail (Quickszint 212, Zinsser Analytic, Frankfurt) was added and the samples were measured in a liquid scintillation counter (Rack Beta Primo 1209, Wallac, Turku). From these results the percentage of [$^3$H]-E2 displacement by the compounds was calculated. The plot of % displacement versus compound concentration resulted in sigmoidal binding curves. The compound concentration to displace 50% of the receptor bound [$^3$H]-E2 were determined. Unlabelled E2 was used as a reference. For determination of the relative binding affinity (RBA) the ratio was calculated according to the following equation: $RBA[\%] = \frac{IC_{50}(E2)}{IC_{50}(compound)} \bullet 100$. This results in an RBA value of 100% for E2. After the assay was established and validated a modification was made to increase throughput. Compounds were tested at concentration of $1000 \bullet IC_{50}(E2)$. Results were reported as RBA ranges. Compounds with less than 50% displacement of [$^3$H]-E2 at a concentration of $1000 \bullet IC_{50}(E2)$ were classified as RBA <0.1%.

The results obtained with the title compounds 1-78 are shown in tables 1 to 3.

Table 1: Inhibition of human 17β-HSD2 and 17β-HSD1 by compounds 1-78 on cell-free protein.

| compound | $IC_{50}$ [nM][a] | | selectivity factor[d] |
|---|---|---|---|
| | 17β-HSD2[b] | 17β-HSD1[c] | |
| 1 | 494 | >100000 | >202 |
| 2 | 594 | 13009 | 22 |
| 3 | 265 | >100000 | >375 |
| 4 | 261 | 64532 | 247 |
| 5 | 11%[e] | n.i.[e] | |
| 6 | 638 | 9099 | 14 |
| 7 | 13%[e] | n.i.[e] | |
| 8 | 481 | 3801 | 8 |
| 9 | 1169 | 98840 | 85 |
| 10 | 28%[e] | 9%[e] | |
| 11 | 517 | >60000 | >115 |
| 12 | 35%[e] | 13%[e] | |
| 13 | 40%[e] | n.i.[e] | |

(continued)

| compound | IC50 [nM][a] | | selectivity factor[d] |
|---|---|---|---|
| | 17β-HSD2[b] | 17β-HSD1[c] | |
| 14 | 18%[e] | n.i.[e] | |
| 15 | 36%[e] | n.i.[e] | |
| 16 | 28%[e] | n.i.[e] | |
| 17 | 31%[e] | n.i.[e] | |
| 18 | 37%[e] | 23%[e] | |
| 19 | 18%[e] | 11%[e] | |
| 20 | 11%[e] | n.i.[e] | |
| 21 | n.i.[e] | n.i.[e] | |
| 22 | 17%[e] | 12%[e] | |
| 23 | 370 | >100000 | >250 |
| 24 | 394 | >5449 | 14 |
| 25 | 496 | >120000 | >240 |
| 26 | 335 | 5105 | 15 |
| 27 | 48%[e] | n.i.[e] | |
| 28 | 148 | 2982 | 20 |
| 29 | 49%[e] | n.i.[e] | |
| 30 | 185 | 3915 | 21 |
| 31 | n.i.[e] | n.i.[e] | |
| 32 | 789 | 43396 | 55 |
| 33 | 221 | >100000 | >450 |
| 34 | 42%[e] | 10%[e] | |
| 35 | 370 | >100000 | >265 |
| 36 | 36%[e] | n.i.[e] | |
| 37 | 1127 | 55741 | 49 |
| 38 | 520 | >100000 | >190 |
| 39 | 161 | >40000 | >250 |
| 40 | 246 | 18597 | 76 |
| 41 | 326 | 3150 | 10 |
| 42 | 430 | 18040 | 42 |
| 43 | 61 | 4451 | 74 |
| 44 | 40 | 202 | 5 |
| 45 | 278 | 23345 | 84 |
| 46 | 163 | 10645 | 65 |
| 47 | 68 | 7593 | 113 |
| 48 | 33%[e] | 33%[e] | |
| 49 | 492 | 14746 | 30 |
| 50 | 410 | 1304 | 3 |
| 51 | 207 | 4337 | 21 |
| 52 | 722 | 1346 | 2 |
| 53 | 48%[e] | n.i.[e] | |
| 54 | 27%[e] | n.i.[e] | |
| 55 | 147 | 6216 | 42 |
| 56 | 62 | >50.000 | >800 |
| 57 | 313 | 1926 | 6 |
| 58 | 169 | 10573 | 62 |
| 59 | 515 | 9927 | 19 |
| 60 | 49%[e] | n.i.[e] | |
| 61 | 242 | >40000 | >165 |

(continued)

| compound | $IC_{50}$ [nM][a] | | selectivity factor[d] |
|---|---|---|---|
| | 17β-HSD2[b] | 17β-HSD1[c] | |
| 62 | 726 | >100000 | >135 |
| 63 | 242 | 5306 | 22 |
| 64 | 58 | 6751 | 116 |
| 65 | 184 | 4812 | 26 |
| 66 | 130 | 5425 | 42 |
| 67 | 450 | 13338 | 30 |
| 68 | 31%[e] | 12%[e] | |
| 69 | 48%[e] | 14%[e] | |
| 70 | 207 | 11454 | 55 |
| 71 | 644 | 6800 | 11 |
| 72 | 62 | 8209 | 133 |
| 73 | 49%[e] | 18%[e] | |
| 74 | 390 | 10221 | 26 |
| 75 | 721 | 12259 | 17 |
| 76 | 137 | 1109 | 8 |
| 77 | n.i.[e] | n.i.[e] | |
| 78 | n.i.[e] | 13%[e] | |
| 79 | 54%[e] | 5%[e] | |
| 80 | 39%[e] | 15%[e] | |
| 81 | 75% (288nM) | 18% (28494 nM) | 99 |
| 82 | 11%[e] | 18%[e] | |
| 83 | 61% (664 nM) | 15% (95624 nM) | 144 |
| 84 | 20%[e] | 0%[e] | |
| 85 | 47%[e] | 23%[e] | |

[a]Mean value of three determinations, except for 79-82 (two data) and 83 (one data), SD less than 20%; [b]Human placenta, microsomal fraction, substrate E2, 500 nM, cofactor $NAD^+$, 1500 μM; [c]Human placenta, cytosolic fraction, substrate E1, 500 nM, cofactor NADH, 500 μM; [d]$IC_{50}$(17β-HSD2)/$IC_{50}$(17β-HSD1); [e]% inhibition at inhibitor concentration of 1 μM; n.i.= no inhibition

Table 2: Inhibition of 17β-HSD2 in MDA-MB-231 cellular assay.

| compound | % Inhibition @ 1 μM [a] |
|---|---|
| | MDA-MB-231 cells [b] |
| 28 | 75% |
| 30 | 60% |
| 33 | 39% |
| 39 | 88% |
| 44 | 99% |
| 47 | 76% |
| 51 | 67% |
| 70 | 50% |
| 56 | 70% |

[a] MDA-MB-231 cells, substrate [$^3$H]-E2: 200 nM; [b] % inhibition tested at 1 μM concentration

Table 3: Estrogen receptor (ERα and ERβ) relative binding affinity

| compound | ERα RBA (%)[a] | ERβ RBA (%)[a] |
|---|---|---|
| 1 | <0.1 | <0.1 |
| 2 | <0.1 | <0.1 |
| 3 | <0.1 | <0.1 |
| 4 | <0.1 | <0.1 |
| 6 | <0.1 | <0.1 |
| 11 | <0.1 | <0.1 |
| 23 | <0.1 | <0.1 |
| 24 | <0.1 | <0.1 |
| 25 | <0.1 | <0.1 |
| 26 | <0.1 | <0.1 |
| 28 | <0.1 | <0.1 |
| 30 | <0.1 | <0.1 |
| 33 | <0.1 | <0.1 |
| 35 | <0.1 | <0.1 |
| 39 | <0.1 | <0.1 |
| 40 | <0.1 | <0.1 |
| 42 | <0.1 | <0.1 |
| 43 | <0.1 | <0.1 |
| 45 | <0.1 | <0.1 |
| 46 | <0.1 | <0.1 |
| 47 | <0.1 | <0.1 |
| 51 | <0.1 | <0.1 |
| 55 | <0.1 | <0.1 |
| 56 | <0.1 | <0.1 |
| 58 | <0.1 | <0.1 |
| 61 | <0.1 | <0.1 |
| 63 | <0.1 | <0.1 |
| 64 | <0.1 | <0.1 |
| 65 | <0.1 | <0.1 |
| 66 | <0.1 | <0.1 |
| 67 | <0.1 | <0.1 |
| 70 | <0.1 | <0.1 |
| 71 | <0.1 | <0.1 |
| 72 | <0.1 | <0.1 |
| 74 | <0.1 | <0.1 |
| 75 | <0.1 | <0.1 |

[a]RBA: Relative Binding Affinity, E2 = 100%

## Claims

**1.** A compound having 17beta-hydroxysteroid dehydrogenase type 2 (17β-HSD2) inhibitory activity of the formula (IV)

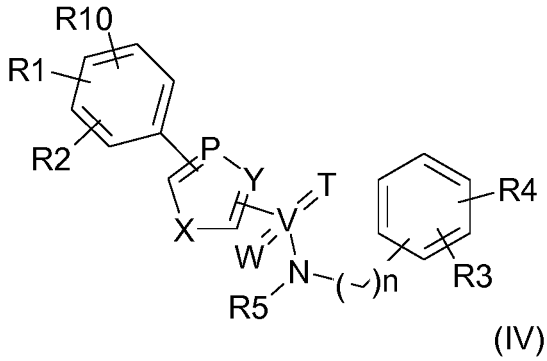

(IV) ,

wherein

R1 and R3 are independently selected from H; -OH; straight-chain, branched-chain or cyclic alkoxy that may be saturated, unsaturated or partially unsaturated; acyloxy; straight-chain, branched-chain or cyclic alkyl that may be saturated, unsaturated or partially unsaturated; $-N(R8)_2$; -SR8; halogen; haloalkyl and phenyl;
R2 and R4 are independently selected from H; -OR8; -COR8; -COOR8; -CONHR8; -OCOR8; -SR8; $-SON(R8)_2$; $-SO_2N(R8)_2$; $-N(R8)_2$; -NHCOR8; -NHSOR8; $-NHSO_2R8$; -halogen; $-NO_2$; -CN; $-SO_2R8$; $-CH_2N(R8)_2$; $-CH_2OR8$; aryl optionally substituted with up to three R8; and straight-chain, branched-chain or cyclic alkyl that may be saturated, unsaturated or partially unsaturated and may optionally be substituted with up to three R8;
R5 is $C_{1-6}$ alkyl;
R8 is selected from H; straight-chain, branched-chain or cyclic alkyl that may be saturated, unsaturated or partially unsaturated and may optionally be substituted with up to three R9; -OH; $-NO_2$; $C_{1-6}$ alkoxy; $C_{1-6}$ alkyl; halogen; haloalkyl; $-NH_2$; -CN; -NHCOR9; -CONHR9; $-NHSO_2R9$; $-SO_2NHR9$; a 5- or 6-membered aromatic or heteroaromatic aryl ring optionally substituted with up to three R9; a homoaromatic ring that might be condensed with a 5- or 6-membered, aliphatic or aromatic heterocyclic ring and that (condensed) homoaromatic ring might optionally be substituted with up to three R9;
R9 is selected from -OH; straight-chain, branched-chain or cyclic alkoxy that may be saturated, unsaturated or partially unsaturated; halogen; haloalkyl; $C_{1-6}$ alkyl; $-NH_2$; $-NO_2$; -CN and phenyl;
R10 is selected from H, halogen, haloalkyl, $C_{1-6}$ alkyl;
n is an integer selected from 0 to 2;
X, Y and P are independently selected from CH, N, N(H), S and O;
V is C or S; W is O or S; and
T, if V is S, represents O or is absent, or, if V is C, is absent;

or a salt thereof,
for use in the treatment and/or prophylaxis of sex steroid deficient-diseases.

2. The compound for use in the treatment and/or prophylaxis of sex steroid deficient-diseases of claim 1, wherein the compound has the formula (IVb)

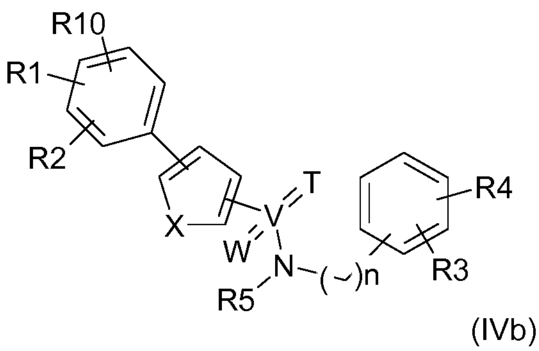

(IVb) ,

wherein the variables R1, R2, R3, R4, R5, R10, n, X, V, W and T, have the same meaning as in claim 1, or a salt thereof.

**3.** The compound for use in the treatment and/or prophylaxis of sex steroid deficient-diseases of claim 1, wherein the compound has the formula (Va)

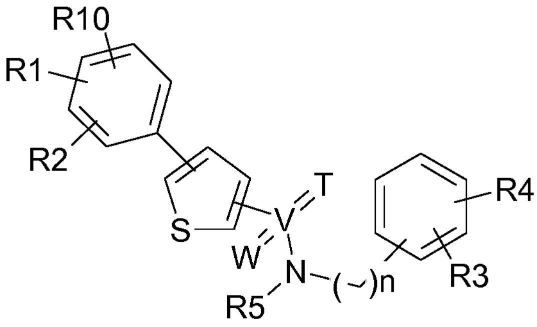

(Va)

wherein the variables R1, R2, R3, R4, R5, R10, n, V, W and T have the same meaning as in claim 1, or a salt thereof, preferably the compound has the formula (VIa)

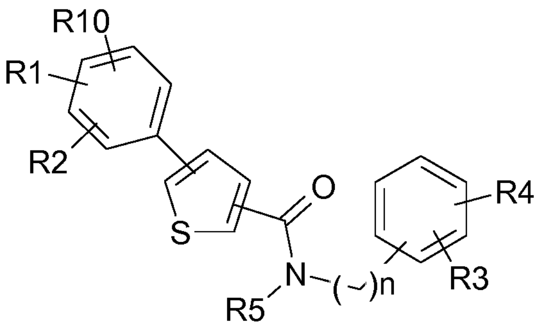

(VIa)

wherein the variables R1, R2, R3, R4, R5, R10 and n have the same meaning as in claim 1, or a salt thereof.

**4.** The compound for use in the treatment and/or prophylaxis of sex steroid deficient-diseases according to any one of claims 1 to 3, wherein the variables, if present, have the following meaning:

R1 and R3 are independently selected from H; -OH; straight-chain, branched-chain or cyclic alkoxy that may be saturated, unsaturated or partially unsaturated; straight-chain, branched-chain or cyclic alkyl that may be saturated, unsaturated or partially unsaturated; $-N(R8)_2$; halogen; haloalkyl and phenyl;
R2 and R4 are independently selected from H; -OR8; -COR8; -COOR8; -CONHR8; -OCOR8; $-SO_2N(R8)_2$; $-N(R8)_2$; -NHCOR8; $-NHSO_2R8$; halogen; $-CH_2N(R8)_2$; $-CH_2OR8$; aryl optionally substituted with up to three R8; $C_{1-6}$ alkyl optionally substituted with up to three R8; and halo $C_{1-6}$ alkyl;
R5 is $C_{1-6}$ alkyl;
R8 is selected from H; $C_{1-6}$ alkyl optionally substituted with up to three R9; halogen; halo $C_{1-6}$ alkyl; -OH; $C_{1-6}$ alkoxy; $-NH_2$; and 5- or 6-membered aromatic or heteroaromatic ring optionally substituted with up to three R9;
R9 is selected from -OH; straight-chain, branched-chain or cyclic alkoxy that may be saturated, unsaturated or partially unsaturated; halogen; haloalkyl; $C_{1-4}$ alkyl; $-NH_2$ and phenyl;
X, Y and P are independently selected from CH, N, N(H) and S; and/or
n is 0 or 1.

**5.** The compound for use in the treatment and/or prophylaxis of sex steroid deficient-diseases of claim 4, wherein R5 is methyl, isopropyl or cyclopropyl and/or R8 is phenyl or benzyl.

**6.** The compound for use in the treatment and/or prophylaxis of sex steroid deficient-diseases of claim 1, wherein the

compound

(i) has the formula (VIIa)

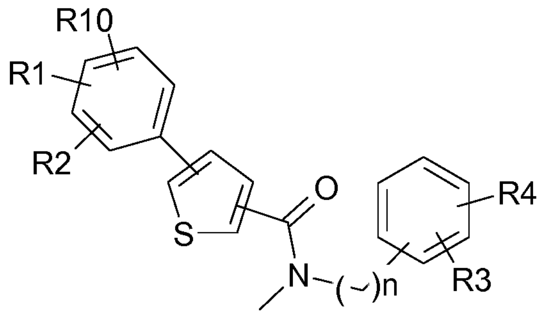

(VIIa)

wherein the variables R1 and R3 are independently selected from H, -OH, -OMe, -Me, -$NMe_2$ and phenyl; R2, R4 and R10 are independently selected from H and halogen; and n is 0 or 1; or a salt thereof, or
(ii) has the formula (VIIIa)

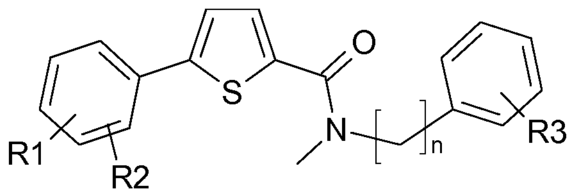

(VIIIa)

wherein R1 and R3 are independently selected from -OH, -$OCH_3$ and -$CH_3$; R2 is H, fluoro or methyl; and n is 0 or 1; or a salt thereof.

**7.** The compound for use in the treatment and/or prophylaxis of sex steroid deficient-diseases of claim 1, wherein the compound is selected from

N-(3-methoxyphenethyl)-5-(3-methoxyphenyl)-N-methylthiophene-2-carboxamide (17),
N-(3-hydroxyphenethyl)-5-(3-hydroxyphenyl)-N-methylthiophene-2-carboxamide (18),
*N*-(3-methoxyphenethyl)-*N*-methyl-5-phenylthiophene-2-carboxamide (19),
*N*-(3-hydroxyphenethyl)-*N*-methyl-5-phenylthiophene-2-carboxamide (20),
5-(3-fluorophenyl)-*N*-(3-methoxyphenethyl)-*N*-methylthiophene-2-carboxamide (21),
5-(3-fluorophenyl)-*N*-(3-hydroxyphenethyl)-*N*-methylthiophene-2-carboxamide (22),
*N*-(3-methoxybenzyl)-5-(3-methoxyphenyl)-*N*-methylthiophene-2-carboxamide (23),
*N*-(3-hydroxybenzyl)-5-(3-hydroxyphenyl)-*N*-methylthiophene-2-carboxamide (24),
*N*-(3-methoxybenzyl)-5-(4-methoxyphenyl)-*N*-methylthiophene-2-carboxamide (25),
*N*-(3-hydroxybenzyl)-5-(4-hydroxyphenyl)-*N*-methylthiophene-2-carboxamide (26),
*N*-(3-methoxybenzyl)-5-(2-methoxyphenyl)-*N*-methylthiophene-2-carboxamide (27),
*N*-(3-hydroxybenzyl)-5-(2-hydroxyphenyl)-*N*-methylthiophene-2-carboxamide (28),
*N*-(3-methoxybenzyl)-*N*-methyl-5-phenylthiophene-2-carboxamide (29),
*N*-(3-hydroxybenzyl)-*N*-methyl-5-phenylthiophene-2-carboxamide (30),
5-(4-cyanophenyl)-*N*-(3-methoxybenzyl)-*N*-methylthiophene-2-carboxamide (31),
5-(4-cyanophenyl)-*N*-(3-hydroxybenzyl)-*N*-methylthiophene-2-carboxamide (32),
*N*-benzyl-5-(3-methoxyphenyl)-*N*-methylthiophene-2-carboxamide (33),
*N*-benzyl-5-(3-hydroxyphenyl)-*N*-methylthiophene-2-carboxamide (34),
*N*-benzyl-5-(4-methoxyphenyl)-*N*-methylthiophene-2-carboxamide (35),
*N*-benzyl-5-(4-hydroxyphenyl)-*N*-methylthiophene-2-carboxamide (36),

*N*-benzyl-5-(2-methoxyphenyl)-*N*-methylthiophene-2-carboxamide (37),
*N*-benzyl-5-(2-hydroxyphenyl)-*N*-methylthiophene-2-carboxamide (38),
*N*-(3-hydroxybenzyl)-5-(4-methoxyphenyl)-*N*-methylthiophene-2-carboxamide (39),
5-(3-(dimethylamino)phenyl)-*N*-(3-hydroxybenzyl)-*N*-methylthiophene-2-carboxamide (40),
5-(3-fluorophenyl)-*N*-(3-hydroxybenzyl)-*N*-methylthiophene-2-carboxamide (41),
5-(4-fluorophenyl)-*N*-(3-hydroxybenzyl)-*N*-methylthiophene-2-carboxamide (42),
5-(2-fluoro-3-methoxyphenyl)-*N*-(3-hydroxybenzyl)-*N*-methylthiophene-2-carboxamide (43),
5-(2-fluoro-3-hydroxyphenyl)-*N*-(3-hydroxybenzyl)-*N*-methylthiophene-2-carboxamide (44),
*N*-(3-hydroxybenzyl)-5-(3-methoxyphenyl)-*N*-methylthiophene-2-carboxamide (45),
*N*-(3-hydroxybenzyl)-*N*-methyl-5-m-tolylthiophene-2-carboxamide (46),
*N*,5-bis(3-methoxyphenyl)-*N*-methylthiophene-2-carboxamide (47),
*N*,5-bis(3-hydroxyphenyl)-*N*-methylthiophene-2-carboxamide (48),
5-(2-methoxyphenyl)-*N*-(3-methoxyphenyl)-*N*-methylthiophene-2-carboxamide (49),
5-(2-hydroxyphenyl)-*N*-(3-hydroxyphenyl)-*N*-methylthiophene-2-carboxamide (50),
*N*-(3-methoxyphenyl)-*N*-methyl-5-phenylthiophene-2-carboxamide (51),
*N*-(3-hydroxyphenyl)-*N*-methyl-5-phenylthiophene-2-carboxamide (52),
5-(4-cyanophenyl)-*N*-(3-methoxyphenyl)-*N*-methylthiophene-2-carboxamide (53),
5-(4-cyanophenyl)-*N*-(3-hydroxyphenyl)-*N*-methylthiophene-2-carboxamide (54),
*N*-(3-methoxyphenyl)-5-(4-methoxyphenyl)-*N*-methylthiophene-2-carboxamide (55),
5-(2-fluoro-3-methoxyphenyl)-*N*-(3-methoxyphenyl)-*N*-methylthiophene-2-carboxamide (56),
5-(2,4-dimethoxyphenyl)-*N*-(3-methoxyphenyl)-*N*-methylthiophene-2-carboxamide (57),
5-(3-(dimethylamino)phenyl)-*N*-(3-methoxyphenyl)-*N*-methylthiophene-2-carboxamide (58),
5-(3-fluorophenyl)-*N*-(3-methoxyphenyl)-*N*-methylthiophene-2-carboxamide (59),
5-(3,4-difluorophenyl)-*N*-(3-methoxyphenyl)-*N*-methylthiophene-2-carboxamide (60),
5-(3-fluoro-4-methoxyphenyl)-*N*-(3-methoxyphenyl)-*N*-methylthiophene-2-carboxamide (61),
5-(4-fluoro-3-methoxyphenyl)-*N*-(3-methoxyphenyl)-*N*-methylthiophene-2-carboxamide (62),
*N*-(3-methoxyphenyl)-*N*-methyl-5-(3-(methylthio)phenyl)thiophene-2-carboxamide (63),
*N*-(3-methoxyphenyl)-*N*-methyl-5-m-tolylthiophene-2-carboxamide (64),
5-(2,6-difluoro-3-methoxyphenyl)-*N*-(3-methoxyphenyl)-*N*-methylthiophene-2-carboxamide (65),
5-(2-fluoro-3-methylphenyl)-*N*-(3-methoxyphenyl)-*N*-methylthiophene-2-carboxamide (66),
5-(3-methoxyphenyl)-*N*-methyl-*N*-phenylthiophene-2-carboxamide (67),
5-(3-hydroxyphenyl)-*N*-methyl-*N*-phenylthiophene-2-carboxamide (68),
*N*-methyl-*N*,5-diphenylthiophene-2-carboxamide (69),
5-(3-methoxyphenyl)-*N*-methyl-*N*-m-tolylthiophene-2-carboxamide (70),
5-(3-hydroxyphenyl)-*N*-methyl-*N*-m-tolylthiophene-2-carboxamide (71),
5-(2-fluoro-3-methoxyphenyl)-*N*-methyl-*N*-(*m*-tolylthiophene)-2-carboxamide (72),
5-(2-fluoro-3-methoxyphenyl)-*N*-(4-methoxyphenyl)-*N*-methylthiophene-2-carboxamide (73),
*N*-(2-fluorophenyl)-*N*-methyl-5-m-tolylthiophene-2-carboxamide (74),
5-(3-methoxyphenyl)-*N*-methyl-*N*-(3-(trifluoromethyl)phenyl)thiophene-2-carboxamide (75),
*N*-(biphenyl-3-yl)-5-(3-methoxyphenyl)-*N*-methylthiophene-2-carboxamide (76),
5-(2-fluoro-3-methoxyphenyl)-*N*-(3-methoxyphenyl)thiophene-2-carboxamide (77) and
5-(2-fluoro-3-methoxyphenyl)-*N*-(3-methoxyphenyl)-*N*-phenylthiophene-2-carboxamide (78),

preferably the compound is selected from compounds (28), (30), (39), (43), (44), (46), (47), (55), (56), (58), (64), (65), (72) and (76), or a salt thereof.

**8.** The compound for use in the treatment and/or prophylaxis of sex steroid deficient-diseases according to any one of claims 1 to 7, which is for the treatment and prophylaxis of diseases caused by a misbalance of OB/OC activity like osteoporosis and osteopenia, bone repair, post-menopausal symptoms, vaginal atrophy, colon cancer, neuronal diseases like Alzheimer and Parkinson's disease, depression, anxiety, hypercholesterolemia, cardiovascular diseases, hair loss, rheumatic diseases and inflammatory diseases, preferably is for the treatment and prophylaxis of osteoporosis.

**9.** A compound having 17β-HSD2 inhibitory activity of the formula (Va)

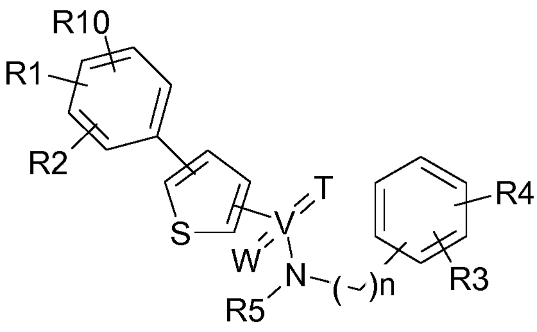

(Va)

wherein

R1 and R3 are independently selected from H; -OH; straight-chain, branched-chain or cyclic alkoxy that may be saturated, unsaturated or partially unsaturated; acyloxy; straight-chain, branched-chain or cyclic alkyl that may be saturated, unsaturated or partially unsaturated; $-N(R8)_2$; -SR8; halogen; haloalkyl and phenyl;
R2 and R4 are independently selected from H; -OR8; -COR8; -COOR8; -CONHR8; -OCOR8; -SR8; $-SON(R8)_2$; $-SO_2N(R8)_2$; $-N(R8)_2$; -NHCOR8; -NHSOR8; $-NHSO_2R8$; -halogen; $-NO_2$; -CN; $-SO_2R8$; $-CH_2N(R8)_2$; $-CH_2OR8$; aryl optionally substituted with up to three R8; and straight-chain, branched-chain or cyclic alkyl that may be saturated, unsaturated or partially unsaturated and may optionally be substituted with up to three R8;
R5 is $C_{1-6}$ alkyl;
R8 is selected from H; straight-chain, branched-chain or cyclic alkyl that may be saturated, unsaturated or partially unsaturated and may optionally be substituted with up to three R9; -OH; $-NO_2$; $C_{1-6}$ alkoxy; $C_{1-6}$ alkyl; halogen; haloalkyl; $-NH_2$; -CN; -NHCOR9; -CONHR9; $-NHSO_2R9$; $-SO_2NHR9$; a 5- or 6-membered aromatic or heteroaromatic aryl ring optionally substituted with up to three R9; a homoaromatic ring that might be condensed with a 5- or 6-membered, aliphatic or aromatic heterocyclic ring and that (condensed) homoaromatic ring might optionally be substituted with up to three R9;
R9 is selected from -OH; straight-chain, branched-chain or cyclic alkoxy that may be saturated, unsaturated or partially unsaturated; halogen; haloalkyl; $C_{1-6}$ alkyl; $-NH_2$; $-NO_2$; -CN and phenyl;
R10 is selected from H, halogen, haloalkyl, $C_{1-6}$ alkyl;
n is 1 or 2;
V is C or S; W is O or S; and
T, if V is S, represents O or is absent, or, if V is C, is absent;

or a salt thereof.

**10.** The compound of claim 9, which has the formula (VIa)

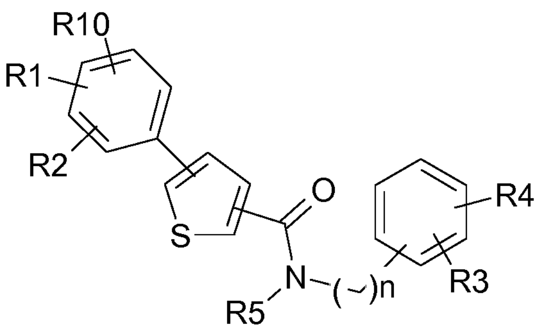

(VIa)

wherein the variables R1, R2, R3, R4, R5, R10 and n have the same meaning as in claim 9, or a salt thereof.

**11.** The compound of claim 9 or 10, wherein the variables, if present, have the following meaning:

R1 and R3 are independently selected from H; -OH; straight-chain, branched-chain or cyclic alkoxy that may be saturated, unsaturated or partially unsaturated; straight-chain, branched-chain or cyclic alkyl that may be saturated, unsaturated or partially unsaturated; $-N(R8)_2$; halogen; haloalkyl and phenyl;
R2 and R4 are independently selected from H; -OR8; -COR8; -COOR8; -CONHR8; -OCOR8; $-SO_2N(R8)_2$; $-N(R8)_2$; -NHCOR8; $-NHSO_2R8$; halogen; $-CH_2N(R8)_2$; $-CH_2OR8$; aryl optionally substituted with up to three R8; $C_{1-6}$ alkyl optionally substituted with up to three R8; and halo $C_{1-6}$ alkyl;
R5 is $C_{1-6}$ alkyl;
R8 is selected from H; $C_{1-6}$ alkyl optionally substituted with up to three R9; halogen; halo $C_{1-6}$ alkyl; -OH; $C_{1-6}$ alkoxy; $-NH_2$; and 5- or 6-membered aromatic or heteroaromatic ring optionally substituted with up to three R9;
R9 is selected from -OH; straight-chain, branched-chain or cyclic alkoxy that may be saturated, unsaturated or partially unsaturated; halogen; haloalkyl; $C_{1-4}$ alkyl; $-NH_2$ and phenyl; and/or
n is 1.

**12.** The compound of claim 11, wherein R5 is methyl, isopropyl or cyclopropyl and/or R8 is phenyl or benzyl.

**13.** The compound of claim 9, which

(i) has the formula (VIIa)

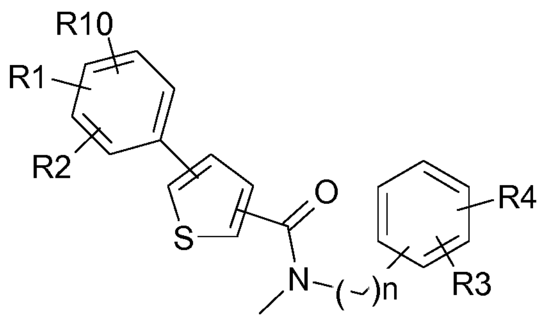

(VIIa)

wherein the variables R1 and R3 are independently selected from H, -OH, -OMe, -Me, $-NMe_2$ and phenyl; R2, R4 and R10 are independently selected from H and halogen; and n is 1; or a salt thereof, or
(ii) has the formula (VIIIa)

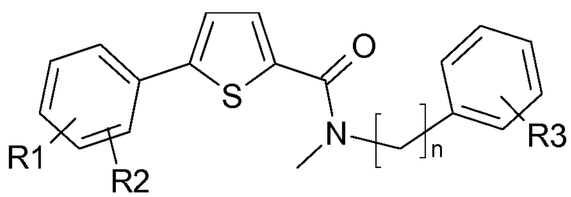

(VIIIa)

wherein R1 and R3 are independently selected from -OH, $-OCH_3$ and $-CH_3$; R2 is H, fluoro or methyl; and n is 1; or a salt thereof.

**14.** The compound of claim 9, which is selected from

N-(3-methoxyphenethyl)-5-(3-methoxyphenyl)-N-methylthiophene-2-carboxamide (17),
N-(3-hydroxyphenethyl)-5-(3-hydroxyphenyl)-N-methylthiophene-2-carboxamide (18),
*N*-(3-methoxyphenethyl)-*N*-methyl-5-phenylthiophene-2-carboxamide (19),
*N*-(3-hydroxyphenethyl)-*N*-methyl-5-phenylthiophene-2-carboxamide (20),

5-(3-fluorophenyl)-*N*-(3-methoxyphenethyl)-*N*-methylthiophene-2-carboxamide (21),
5-(3-fluorophenyl)-*N*-(3-hydroxyphenethyl)-*N*-methylthiophene-2-carboxamide (22),
*N*-(3-methoxybenzyl)-5-(3-methoxyphenyl)-*N*-methylthiophene-2-carboxamide (23),
*N*-(3-hydroxybenzyl)-5-(3-hydroxyphenyl)-*N*-methylthiophene-2-carboxamide (24),
*N*-(3-methoxybenzyl)-5-(4-methoxyphenyl)-*N*-methylthiophene-2-carboxamide (25),
*N*-(3-hydroxybenzyl)-5-(4-hydroxyphenyl)-*N*-methylthiophene-2-carboxamide (26),
*N*-(3-methoxybenzyl)-5-(2-methoxyphenyl)-*N*-methylthiophene-2-carboxamide (27),
*N*-(3-hydroxybenzyl)-5-(2-hydroxyphenyl)-*N*-methylthiophene-2-carboxamide (28),
*N*-(3-methoxybenzyl)-*N*-methyl-5-phenylthiophene-2-carboxamide (29),
*N*-(3-hydroxybenzyl)-*N*-methyl-5-phenylthiophene-2-carboxamide (30),
5-(4-cyanophenyl)-*N*-(3-methoxybenzyl)-*N*-methylthiophene-2-carboxamide (31),
5-(4-cyanophenyl)-*N*-(3-hydroxybenzyl)-*N*-methylthiophene-2-carboxamide (32),
*N*-benzyl-5-(3-methoxyphenyl)-*N*-methylthiophene-2-carboxamide (33),
*N*-benzyl-5-(3-hydroxyphenyl)-*N*-methylthiophene-2-carboxamide (34),
*N*-benzyl-5-(4-methoxyphenyl)-*N*-methylthiophene-2-carboxamide (35),
*N*-benzyl-5-(4-hydroxyphenyl)-*N*-methylthiophene-2-carboxamide (36),
*N*-benzyl-5-(2-methoxyphenyl)-*N*-methylthiophene-2-carboxamide (37),
*N*-benzyl-5-(2-hydroxyphenyl)-*N*-methylthiophene-2-carboxamide (38),
*N*-(3-hydroxybenzyl)-5-(4-methoxyphenyl)-*N*-methylthiophene-2-carboxamide (39),
5-(3-(dimethylamino)phenyl)-*N*-(3-hydroxybenzyl)-*N*-methylthiophene-2-carboxamide (40),
5-(3-fluorophenyl)-*N*-(3-hydroxybenzyl)-*N*-methylthiophene-2-carboxamide (41),
5-(4-fluorophenyl)-*N*-(3-hydroxybenzyl)-*N*-methylthiophene-2-carboxamide (42),
5-(2-fluoro-3-methoxyphenyl)-*N*-(3-hydroxybenzyl)-*N*-methylthiophene-2-carboxamide (43),
5-(2-fluoro-3-hydroxyphenyl)-*N*-(3-hydroxybenzyl)-*N*-methylthiophene-2-carboxamide (44),
*N*-(3-hydroxybenzyl)-5-(3-methoxyphenyl)-*N*-methylthiophene-2-carboxamide (45), and
*N*-(3-hydroxybenzyl)-*N*-methyl-5-m-tolylthiophene-2-carboxamide (46),

preferably the compound is selected from compounds (28), (30), (39), (43), (44) and (46), or a salt thereof.

**15.** A compound having 17β-HSD2 inhibitory activity

*N*,5-bis(3-methoxyphenyl)-*N*-methylthiophene-2-carboxamide (47),
*N*,5-bis(3-hydroxyphenyl)-*N*-methylthiophene-2-carboxamide (48),
5-(2-methoxyphenyl)-*N*-(3-methoxyphenyl)-*N*-methylthiophene-2-carboxamide (49),
5-(2-hydroxyphenyl)-*N*-(3-hydroxyphenyl)-*N*-methylthiophene-2-carboxamide (50),
*N*-(3-methoxyphenyl)-*N*-methyl-5-phenylthiophene-2-carboxamide (51),
*N*-(3-hydroxyphenyl)-*N*-methyl-5-phenylthiophene-2-carboxamide (52),
5-(4-cyanophenyl)-*N*-(3-methoxyphenyl)-*N*-methylthiophene-2-carboxamide (53),
5-(4-cyanophenyl)-*N*-(3-hydroxyphenyl)-*N*-methylthiophene-2-carboxamide (54),
*N*-(3-methoxyphenyl)-5-(4-methoxyphenyl)-*N*-methylthiophene-2-carboxamide (55),
5-(2-fluoro-3-methoxyphenyl)-*N*-(3-methoxyphenyl)-*N*-methylthiophene-2-carboxamide (56),
5-(2,4-dimethoxyphenyl)-*N*-(3-methoxyphenyl)-*N*-methylthiophene-2-carboxamide (57),
5-(3-(dimethylamino)phenyl)-*N*-(3-methoxyphenyl)-*N*-methylthiophene-2-carboxamide (58),
5-(3-fluorophenyl)-*N*-(3-methoxyphenyl)-*N*-methylthiophene-2-carboxamide (59),
5-(3,4-difluorophenyl)-*N*-(3-methoxyphenyl)-*N*-methylthiophene-2-carboxamide (60),
5-(3-fluoro-4-methoxyphenyl)-*N*-(3-methoxyphenyl)-*N*-methylthiophene-2-carboxamide (61),
5-(4-fluoro-3-methoxyphenyl)-*N*-(3-methoxyphenyl)-*N*-methylthiophene-2-carboxamide (62),
*N*-(3-methoxyphenyl)-*N*-methyl-5-(3-(methylthio)phenyl)thiophene-2-carboxamide (63),
*N*-(3-methoxyphenyl)-*N*-methyl-5-m-tolylthiophene-2-carboxamide (64),
5-(2,6-difluoro-3-methoxyphenyl)-*N*-(3-methoxyphenyl)-*N*-methylthiophene-2-carboxamide (65),
5-(2-fluoro-3-methylphenyl)-*N*-(3-methoxyphenyl)-*N*-methylthiophene-2-carboxamide (66),
5-(3-methoxyphenyl)-*N*-methyl-*N*-phenylthiophene-2-carboxamide (67),
5-(3-hydroxyphenyl)-*N*-methyl-*N*-phenylthiophene-2-carboxamide (68),
*N*-methyl-*N*,5-diphenylthiophene-2-carboxamide (69), 5-(3-methoxyphenyl)-*N*-methyl-*N*-m-tolylthiophene-2-carboxamide (70),
5-(3-hydroxyphenyl)-*N*-methyl-*N*-m-tolylthiophene-2-carboxamide (71),
5-(2-fluoro-3-methoxyphenyl)-*N*-methyl-*N*-(m-tolylthiophene)-2-carboxamide (72),
5-(2-fluoro-3-methoxyphenyl)-*N*-(4-methoxyphenyl)-*N*-methylthiophene-2-carboxamide (73),

*N*-(2-fluorophenyl)-*N*-methyl-5-m-tolylthiophene-2-carboxamide (74),
5-(3-methoxyphenyl)-*N*-methyl-*N*-(3-(trifluoromethyl)phenyl)thiophene-2-carboxamide (75),
*N*-(biphenyl-3-yl)-5-(3-methoxyphenyl)-*N*-methylthiophene-2-carboxamide (76),
5-(2-fluoro-3-methoxyphenyl)-*N*-(3-methoxyphenyl)thiophene-2-carboxamide (77) and
5-(2-fluoro-3-methoxyphenyl)-*N*-(3-methoxyphenyl)-*N*-phenylthiophene-2-carboxamide (78),

or a salt thereof.

**16.** A process for producing a compound formula (Va) or a salt thereof according to any one of claims 9 to 14, which comprises condensing an activated acyl or sulfonyl compound of the formula (B) with a secondary amine of the formula (C), followed by a cross-coupling reaction with a compound of the formula (A)

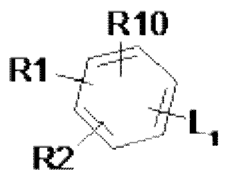

A

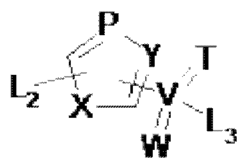

B

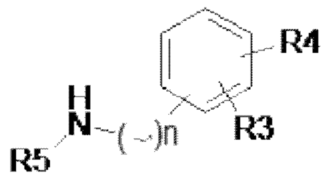

C

wherein all the variables are as defined in claim 9, and L1, L2 and L3 are leaving groups.

**17.** A pharmaceutical composition or medicament comprising at least one compound having 17β-HSD2 inhibitory activity according to any one of claims 9 to 15 or a pharmaceutically acceptable salt thereof, and optionally a suitable carrier or excipient.

**18.** The pharmaceutical composition or medicament of claim 17, which is for the treatment and prophylaxis of sex steroid deficient diseases including diseases caused by a misbalance of OB/OC activity like osteoporosis and osteopenia, bone repair, post-menopausal symptoms, vaginal atrophy, colon cancer, neuronal diseases like Alzheimer and Parkinson's disease, depression, anxiety, hypercholesterolemia, cardiovascular diseases, hair loss, rheumatic diseases and inflammatory diseases, preferably for the treatment and prophylaxis of osteoporosis.

**19.** Use of a compound having 17β-HSD2 inhibitory activity according to any one of claims 9 to 15 or a pharmaceutically acceptable salt thereof for preparing a medicament for the treatment and prophylaxis of sex steroid deficient diseases.

**20.** A compound for use in the treatment and/or of prophylaxis of sex steroid deficient diseases of claim 1, wherein the compound has the formula (IVb)

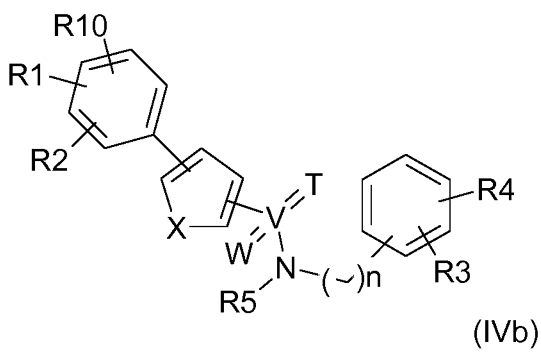

(IVb),

wherein

R1 and R3 are independently selected from H; -OH; straight-chain, branched-chain or cyclic alkoxy that may be saturated, unsaturated or partially unsaturated; acyloxy; straight-chain, branched-chain or cyclic alkyl that

may be saturated, unsaturated or partially unsaturated; $-N(R8)_2$; -SR8; halogen; haloalkyl and phenyl;
R2 and R4 are independently selected from H; -OR8; -COR8; -COOR8; -CONHR8; -OCOR8; -SR8; $-SON(R8)_2$; $-SO_2N(R8)_2$; $-N(R8)_2$; -NHCOR8; -NHSOR8; $-NHSO_2R8$; -halogen; $-NO_2$; -CN; $-SO_2R8$; $-CH_2N(R8)_2$; $-CH_2OR8$; aryl optionally substituted with up to three R8; and straight-chain, branched-chain or cyclic alkyl that may be saturated, unsaturated or partially unsaturated and may optionally be substituted with up to three R8;
R5 is $C_{1-6}$ alkyl;
R8 is selected from H; straight-chain, branched-chain or cyclic alkyl that may be saturated, unsaturated or partially unsaturated and may optionally be substituted with up to three R9; -OH; $-NO_2$; $C_{1-6}$ alkoxy; $C_{1-6}$ alkyl; halogen; haloalkyl; $-NH_2$; -CN; -NHCOR9; -CONHR9; $-NHSO_2R9$; $-SO_2NHR9$; a 5- or 6-membered aromatic or heteroaromatic aryl ring optionally substituted with up to three R9; a homoaromatic ring that might be condensed with a 5- or 6-membered, aliphatic or aromatic heterocyclic ring and that (condensed) homoaromatic ring might optionally be substituted with up to three R9;
R9 is selected from -OH; straight-chain, branched-chain or cyclic alkoxy that may be saturated, unsaturated or partially unsaturated; halogen; haloalkyl; $C_{1-6}$ alkyl; $-NH_2$; $-NO_2$; -CN and phenyl;
R10 is selected from H, halogen, haloalkyl, $C_{1-6}$ alkyl;
n is an integer selected from 0 to 2;
X is O;
V is C or S; W is O or S; and
T, if V is S, represents O or is absent, or, if V is C, is absent;

or a salt thereof,
which is for use in the treatment and/or prophylaxis of inflammatory diseases.

**21.** The compound of the formula (IVb) for use in the treatment and/or prophylaxis of inflammatory diseases of claim 20, wherein the variables, if present, have the following meaning:

R1 and R3 are independently selected from H; -OH; straight-chain, branched-chain or cyclic alkoxy that may be saturated, unsaturated or partially unsaturated; straight-chain, branched-chain or cyclic alkyl that may be saturated, unsaturated or partially unsaturated; $-N(R8)_2$; halogen; haloalkyl and phenyl;
R2 and R4 are independently selected from H; -OR8; -COR8; -COOR8; -CONHR8; -OCOR8; $-SO_2N(R8)_2$; $-N(R8)_2$; -NHCOR8; $-NHSO_2R8$; halogen; $-CH_2N(R8)_2$; $-CH_2OR8$; aryl optionally substituted with up to three R8; $C_{1-6}$ alkyl optionally substituted with up to three R8; and halo $C_{1-6}$ alkyl;
R5 is $C_{1-6}$ alkyl;
R8 is selected from H; $C_{1-6}$ alkyl optionally substituted with up to three R9; halogen; halo $C_{1-6}$ alkyl; -OH; $C_{1-6}$ alkoxy; $-NH_2$; and 5- or 6-membered aromatic or heteroaromatic ring optionally substituted with up to three R9;
R9 is selected from -OH; straight-chain, branched-chain or cyclic alkoxy that may be saturated, unsaturated or partially unsaturated; halogen; haloalkyl; $C_{1-4}$ alkyl; $-NH_2$ and phenyl; and/or
n is 0 or 1.

**22.** The compound of the formula (IVb) for use in the treatment and/or prophylaxis of inflammatory diseases of claim 21, wherein R5 is methyl, isopropyl or cyclopropyl and/or R8 is phenyl or benzyl.

**Patentansprüche**

**1.** Verbindung mit 17beta-Hydroxysteroid dehydrogenase-Typ 2 (17β-HSD2) inhibitorischer Aktivität mit der Formel (IV)

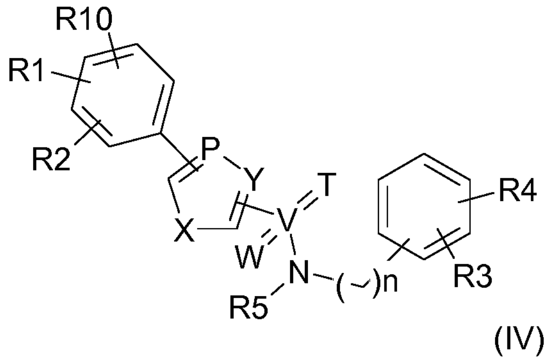

(IV),

worin

R1 und R3 unabhängig voneinander aus H; -OH; geradkettigem, verzweigtem oder cyclischem Alkoxy, welches gesättigt, ungesättigt oder partiell ungesättigt sein kann; Acyloxy; geradkettigem, verzweigtem oder cyclischem Alkyl, welches gesättigt, ungesättigt oder partiell ungesättigt sein kann; $-N(R8)_2$; -SR8; - Halogen; Haloalkyl und Phenyl, ausgewählt sind;
R2 und R4 unabhängig voneinander aus H; -OR8; -COR8; -COOR8; -CONHR8; - OCOR8; -SR8; $-SON(R8)_2$; $-SO_2N(R8)_2$; $-N(R8)_2$; -NHCOR8; -NHSOR8; $-NHSO_2R8$; -Halogen; $-NO_2$; -CN; $-SO_2R8$; $-CH_2N(R8)_2$; $-CH_2OR8$; Aryl optional mit bis zu drei R8 substituiert; und geradkettigem, verzweigtem, oder cyclischem Alkyl, welches gesättigt, ungesättigt oder partiell ungesättigt, und optional mit bis zu drei R8 substituiert, ausgewählt sind;
R5 $C_{1-6}$ Alkyl ist;
R8 aus H; geradkettigem, verzweigtem, oder cyclischem Alkyl, welches gesättigt, ungesättigt oder partiell ungesättigt und optional mit bis zu drei R9 substituiert sein kann; -OH; $-NO_2$; $C_{1-6}$ Alkoxy; $C_{1-6}$ Alkyl; Halogen; Haloalkyl; $-NH_2$; -CN; - NHCOR9; -CONHR9; $-NHSO_2R9$; $-SO_2NHR9$; einem 5- or 6-gliedrigen aromatischen oder heteroaromatischen Arylring, optional mit bis zu drei R9 substituiert; einem homoaromatischen Ring der mit einem 5- or 6-gliedrigen, aliphatischen oder aromatischen heterocyclischen Ring kondensiert sein kann, und dieser (kondensierte) homoaromatische Ring kann optional mit bis zu drei R9 substituiert sein;
R9 aus -OH; geradkettigem, verzweigtem oder cyclischem Alkoxy, welches gesättigt, ungesättigt oder partiell ungesättigt sein kann; Halogen; Haloalkyl; $C_{1-6}$ Alkyl; $-NH_2$; $-NO_2$; -CN und Phenyl ausgewählt ist;
R10 aus H, Halogen, Haloalkyl, $C_{1-6}$ Alkyl ausgewählt ist;
n ein Integer aus 0 bis 2 ausgewählt ist;
X, Y und P unabhängig voneinander aus CH, N, N(H), S und O ausgewählt sind; V C oder S ist; W O oder S ist; und T, falls V S ist, entweder O repräsentiert oder abwesend ist, oder, falls V C ist, abwesend ist;

oder ein Salz davon,
zur Verwendung in der Behandlung und/oder Vorbeugung von Sexualhormon-defizienten Erkrankungen.

**2.** Verbindung zur Verwendung in der Behandlung und/oder Vorbeugung von Sexualhormon-defizienten Erkrankungen nach Anspruch 1, worin die Verbindung die Formel (IVb) hat

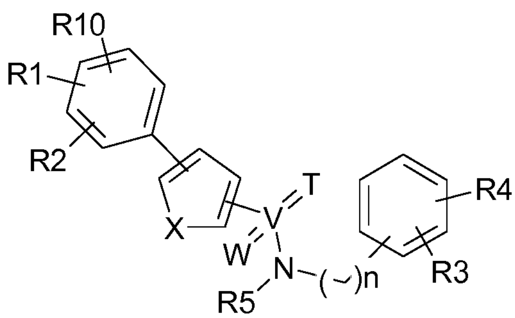

(IVb),

worin die Variablen R1, R2, R3, R4, R5, R10, n, X, V, W und T, wie im Anspruch 1 definiert sind, oder ein Salz davon.

3. Verbindung zur Verwendung in der Behandlung und/oder Vorbeugung von Sexualhormon-defizienten Erkrankungen nach Anspruch 1, worin die Verbindung die Formel (Va) hat

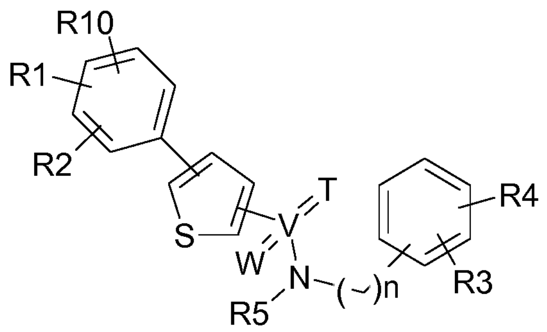

(Va)

worin die Variablen R1, R2, R3, R4, R5, R10, n, X, V, W und T, wie im Anspruch 1 definiert sind, oder ein Salz davon, vorzugsweise die Verbindung die Formel (VIa) hat.

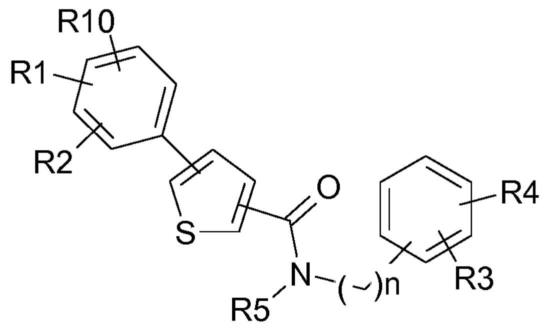

(VIa)

worin die Variablen R1, R2, R3, R4, R5, R10 und n wie im Anspruch 1 definiert sind, oder ein Salz davon,

4. Verbindung zur Verwendung in der Behandlung und/oder Vorbeugung von Sexualhormon-defizienten Erkrankungen nach irgendeinem der Ansprüche 1-3, worin die Variablen, falls vorhanden, die folgende Bedeutung haben:

R1 und R3 sind unabhängig voneinander aus H; -OH; geradkettigem, verzweigtem oder cyclischem Alkoxy, welches gesättigt, ungesättigt oder partiell ungesättigt sein kann; geradkettigem, verzweigtem oder cyclischem Alkyl, welches gesättigt, ungesättigt oder partiell ungesättigt sein kann; $-N(R8)_2$;-Halogen; Haloalkyl und Phenyl, ausgewählt;
R2 und R4 unabhängig voneinander aus H; -OR8; -COR8; -COOR8; -CONHR8; - OCOR8; $-SO_2N(R8)_2$; $-N(R8)_2$; -NHCOR8; $-NHSO_2R8$; -Halogen; $-CH_2N(R8)_2$;$-CH_2OR8$; Aryl optional mit bis zu drei R8 substituiert; $C_{1-6}$ Alkyl optional mit bis zu drei R8 substituiert; und Halo $C_{1-6}$ Alkyl ausgewählt sind;
R5 $C_{1-6}$ Alkyl ist;
R8 aus H; $C_{1-6}$ Alkyl optional mit bis zu drei R9; Halogen; Halo $C_{1-6}$ Alkyl; -OH; $C_{1-6}$ Alkoxy; $-NH_2$; und einem 5- oder 6-gliedrigen aromatischen oder heteroaromatischen Ring optional mit bis zu drei R9 substituiert, ausgewählt;
R9 aus -OH; geradkettigem, verzweigtem oder cyclischem Alkoxy, welches gesättigt, ungesättigt oder partiell ungesättigt sein kann; Halogen; Haloalkyl; $C_{1-4}$ Alkyl; $-NH_2$ und Phenyl ausgewählt ist;
X, Y und P unabhängig voneinander aus CH, N, N(H), und S ausgewählt sind; und/oder n 0 oder 1 ist.

5. Verbindung zur Verwendung in der Behandlung und/oder Vorbeugung von Sexualhormon-defizienten Erkrankungen nach Anspruch 4, worin R5 Methyl, Isopropyl oder Cyclopropyl und/oder R8 ein Phenyl oder Benzyl ist

**6.** Verwendung einer Verbindung zur Behandlung und/oder Vorbeugung von Sexualhormon-defizienten Erkrankungen nach Anspruch 1, worin die Verbindung

(i) die Formel (VIIa) hat

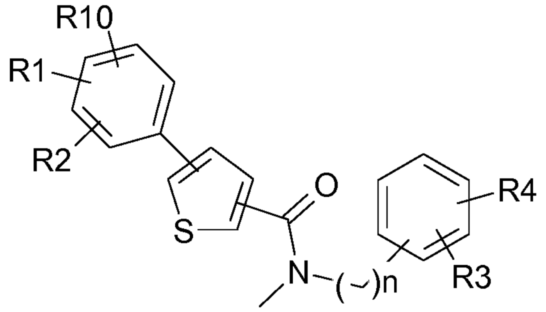

(VIIa)

worin die Variablen R1 and R3 unabhängig voneinander aus H, -OH, -OMe, -Me, $-NMe_2$ und Phenyl; R2, R4 und R10 unabhängig voneinander aus H und Halogen ausgewählt sind; und n 0 oder 1 ist; oder ein Salz davon, oder

(ii) die Formel (VIIIa) hat

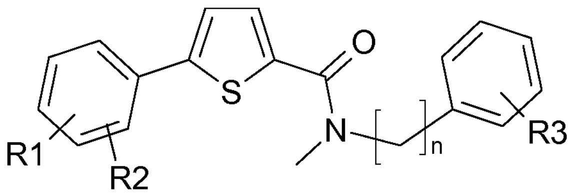

(VIIIa)

worin R1 und R3 unabhängig voneinander aus -OH, $-OCH_3$ and $-CH_3$ ausgewählt sind; R2 H, Fluoro or Methyl ist; und n 0 oder 1 ist; oder ein Salz davon ist.

**7.** Verbindung zur Verwendung in der Behandlung und/oder Vorbeugung von Sexualhormon-defizienten Erkrankungen nach Anspruch 1, worin die Verbindung aus

*N*-(3-Methoxyphenethyl)-5-(3-methoxyphenyl)-*N*-methylthiophen-2-carboxamid (17),
*N*-(3-Hydroxyphenethyl)-5-(3-hydroxyphenyl)-*N*-methylthiophen-2-carboxamid (18),
*N*-(3-Methoxyphenethyl)-*N*-methyl-5-phenylthiophen-2-carboxamid (19),
*N*-(3-Hydroxyphenethyl)-*N*-methyl-5-phenylthiophen-2-carboxamid (20),
5-(3-Fluorophenyl)-*N*-(3-methoxyphenethyl)-*N*-methylthiophen-2-carboxamid (21),
5-(3-Fluorophenyl)-*N*-(3-hydroxyphenethyl)-*N*-methylthiophen-2-carboxamid (22),
*N*-(3-Methoxybenzyl)-5-(3-methoxyphenyl)-*N*-methylthiophen-2-carboxamid (23),
*N*-(3-Hydroxybenzyl)-5-(3-hydroxyphenyl)-*N*-methylthiophen-2-carboxamid (24),
*N*-(3-Methoxybenzyl)-5-(4-methoxyphenyl)-*N*-methylthiophen-2-carboxamid (25),
*N*-(3-Hydroxybenzyl)-5-(4-hydroxyphenyl)-*N*-methylthiophen-2-carboxamid (26),
*N*-(3-Methoxybenzyl)-5-(2-methoxyphenyl)-*N*-methylthiophen-2-carboxamid (27),
*N*-(3-hydroxybenzyl)-5-(2-hydroxyphenyl)-*N*-methylthiophen-2-carboxamid (28),
*N*-(3-Methoxybenzyl)-*N*-methyl-5-phenylthiophen-2-carboxamid (29),
*N*-(3-Hydroxybenzyl)-*N*-methyl-5-phenylthiophen-2-carboxamid (30),
5-(4-Cyanophenyl)-*N*-(3-methoxybenzyl)-*N*-methylthiophen-2-carboxamid (31),
5-(4-Cyanophenyl)-*N*-(3-hydroxybenzyl)-*N*-methylthiophen-2-carboxamid (32),
*N*-Benzyl-5-(3-methoxyphenyl)-*N*-methylthiophen-2-carboxamid (33),
*N*-Benzyl-5-(3-hydroxyphenyl)-*N*-methylthiophen-2-carboxamid (34),

*N*-Benzyl-5-(4-methoxyphenyl)-*N*-methylthiophen-2-carboxamid (35),
*N*-Benzyl-5-(4-hydroxyphenyl)-*N*-methylthiophen-2-carboxamid (36),
*N*-Benzyl-5-(2-methoxyphenyl)-*N*-methylthiophen-2-carboxamid (37),
*N*-Benzyl-5-(2-hydroxyphenyl)-*N*-methylthiophen-2-carboxamid (38),
*N*-(3-Hydroxybenzyl)-5-(4-methoxyphenyl)-*N*-methylthiophen-2-carboxamid (39),
5-(3-(Dimethylamino)phenyl)-*N*-(3-hydroxybenzyl)-*N*-methylthiophen-2-carboxamid (40),
5-(3-Fluorophenyl)-*N*-(3-hydroxybenzyl)-*N*-methylthiophen-2-carboxamid (41),
5-(4-Fluorophenyl)-*N*-(3-hydroxybenzyl)-*N*-methylthiophen-2-carboxamid (42),
5-(2-Fluoro-3-methoxyphenyl)-*N*-(3-hydroxybenzyl)-*N*-methylthiophen-2-carboxamid (43),
5-(2-Fluoro-3-hydroxyphenyl)-*N*-(3-hydroxybenzyl)-*N*-methylthiophen-2-carboxamid (44),
*N*-(3-Hydroxybenzyl)-5-(3-methoxyphenyl)-*N*-methylthiophen-2-carboxamid (45),
*N*-(3-Hydroxybenzyl)-*N*-methyl-5-m-tolylthiophen-2-carboxamid (46),
*N*,5-Bis(3-methoxyphenyl)-*N*-methylthiophen-2-carboxamid (47),
*N*,5-Bis(3-hydroxyphenyl)-*N*-methylthiophen-2-carboxamid (48),
5-(2-Methoxyphenyl)-*N*-(3-methoxyphenyl)-*N*-methylthiophen-2-carboxamid (49),
5-(2-Hydroxyphenyl)-*N*-(3-hydroxyphenyl)-*N*-methylthiophen-2-carboxamid (50),
*N*-(3-Methoxyphenyl)-*N*-methyl-5-phenylthiophen-2-carboxamid (51),
*N*-(3-Hydroxyphenyl)-*N*-methyl-5-phenylthiophen-2-carboxamid (52),
5-(4-Cyanophenyl)-*N*-(3-methoxyphenyl)-*N*-methylthiophen-2-carboxamid (53),
5-(4-Cyanophenyl)-*N*-(3-hydroxyphenyl)-*N*-methylthiophen-2-carboxamid (54),
*N*-(3-Methoxyphenyl)-5-(4-methoxyphenyl)-*N*-methylthiophen-2-carboxamid (55),
5-(2-Fluoro-3-methoxyphenyl)-*N*-(3-methoxyphenyl)-*N*-methylthiophen-2-carboxamid (56),
5-(2,4-dimethoxyphenyl)-*N*-(3-methoxyphenyl)-*N*-methylthiophen-2-carboxamid (57),
5-(3-(Dimethylamino)phenyl)-*N*-(3-methoxyphenyl)-*N*-methylthiophen-2-carboxamid (58),
5-(3-Fluorophenyl)-*N*-(3-methoxyphenyl)-*N*-methylthiophen-2-carboxamid (59),
5-(3,4-Difluorophenyl)-*N*-(3-methoxyphenyl)-*N*-methylthiophen-2-carboxamid (60),
5-(3-Fluoro-4-methoxyphenyl)-*N*-(3-methoxyphenyl)-*N*-methylthiophen-2-carboxamid (61),
5-(4-Fluoro-3-methoxyphenyl)-*N*-(3-methoxyphenyl)-*N*-methylthiophen-2-carboxamid (62),
*N*-(3-Methoxyphenyl)-*N*-methyl-5-(3-(methylthio)phenyl)thiophen-2-carboxamid (63),
*N*-(3-Methoxyphenyl)-*N*-methyl-5-m-tolylthiophen-2-carboxamid (64),
5-(2,6-Difluoro-3-methoxyphenyl)-*N*-(3-methoxyphenyl)-*N*-methylthiophen-2-carboxamid (65),
5-(2-Fluoro-3-methylphenyl)-N-(3-methoxyphenyl)-N-methylthiophen-2-carboxamid (66),
5-(3-Methoxyphenyl)-*N*-methyl-*N*-phenylthiophen-2-carboxamid (67),
5-(3-Hydroxyphenyl)-*N*-methyl-*N*-phenylthiophen-2-carboxamid (68), *N*-Methyl-*N*,5-diphenylthiophen-2-carboxamid (69),
5-(3-Methoxyphenyl)-*N*-methyl-*N*-m-tolylthiophen-2-carboxamid (70),
5-(3-Hydroxyphenyl)-*N*-methyl-*N*-m-tolylthiophen-2-carboxamid (71),
5-(2-Fluoro-3-methoxyphenyl)-*N*-methyl-*N*-(m-tolylthiophen)-2-carboxamid (72),
5-(2-Fluoro-3-methoxyphenyl)-*N*-(4-methoxyphenyl)-*N*-methylthiophen-2-carboxamid (73),
*N*-(2-Fluorophenyl)-*N*-methyl-5-m-tolylthiophen-2-carboxamid (74),
5-(3-Methoxyphenyl)-*N*-methyl-*N*-(3-(trifluoromethyl)phenyl)thiophen-2-carboxamid (75),
*N*-(Biphenyl-3-yl)-5-(3-methoxyphenyl)-*N*-methylthiophen-2-carboxamid (76),
5-(2-Fluoro-3-methoxyphenyl)-*N*-(3-methoxyphenyl)thiophen-2-carboxamid (77) und
5-(2-Fluoro-3-methoxyphenyl)-N-(3-methoxyphenyl)-N-phenylthiophen-2-carboxamid (78) ausgewählt ist,

vorzugsweise wird die Verbindung aus den Verbindungen (28), (30), (39), (43), (44), (46), (47), (55), (56), (58), (64), (65), (72) und (76) selektiert, oder ein Salz davon.

**8.** Verbindung zur Verwendung in der Behandlung und/oder Vorbeugung von Sexualhormon-defizienten Erkrankungen nach irgendeinem der Ansprüche 1 bis 7, die zur Behandlung und Vorbeugung von Erkrankungen, welche durch eine Fehlbalanz in OB/OC Aktivität wie Osteoporose und Osteopenie, Knochenheilung, post-menopausale Symptome, vaginale Atrophie, Kolonkarzinom, neuronale Erkrankungen wie Alzheimer und Morbus Parkison, Depression, Angst, Hypercholesterinämie, kardiovaskuläre Erkrankungen, Haarausfall, rheumatische Erkrankungen und inflammatorische Erkrankungen geeignet ist, vorzugsweise zur Behandlung und Vorbeugung von Osteoporose.

**9.** Verbindung der Formel (Va) mit 17β-HSD2 inhibitorischer Aktivität

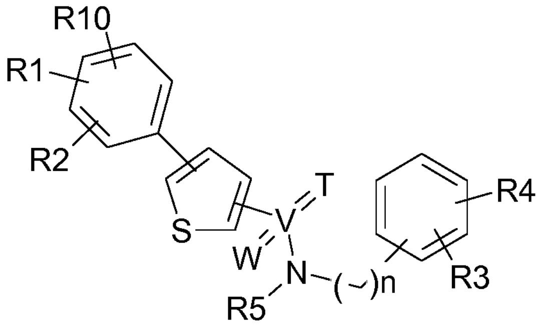

(Va)

worin

R1 und R3 unabhängig voneinander aus H; -OH; geradkettigem, verzweigtem oder cyclischem Alkoxy, welches gesättigt, ungesättigt oder partiell ungesättigt sein kann; Acyloxy; geradkettigem, verzweigtem oder cyclischem Alkyl, welches gesättigt, ungesättigt oder partiell ungesättigt sein kann; $-N(R8)_2$; -SR8; Halogen; Haloalkyl und Phenyl ausgewählt sind;
R2 und R4 unabhängig voneinander aus H; -OR8; -COR8; -COOR8; -CONHR8; - OCOR8; -SR8; $-SON(R8)_2$; $-SO_2N(R8)_2$; $-N(R8)_2$; -NHCOR8; -NHSOR8; $-NHSO_2R8$; -Halogen; $-NO_2$; -CN; $-SO_2R8$; $-CH_2N(R8)_2$; $-CH_2OR8$; Aryl optional mit bis zu drei R8 substituiert; und geradkettigem, verzweigtem, oder cyclischem Alkyl, welches gesättigt, ungesättigt oder partiell gesättigt sein kann, und optional mit bis zu drei R8 substituiert, ausgewählt sind;
R5 $C_{1-6}$ Alkyl ist;
R8 aus H; geradkettigem, verzweigtem, oder cyclischem Alkyl, welches gesättigt, ungesättigt oder partiell ungesättigt und optional mit bis zu drei R9 substituiert sein kann; -OH; $-NO_2$; $C_{1-6}$ Alkoxy; $C_{1-6}$ Alkyl; Halogen; Haloalkyl; $-NH_2$; -CN; - NHCOR9; -CONHR9; $-NHSO_2R9$; $-SO_2NHR9$; einem 5- or 6-gliedrigen aromatischen oder heteroaromatischen Arylring, optional mit bis zu drei R9 substituiert; einem homoaromatischen Ring der mit einem 5- or 6-gliedrigen, aliphatischen oder aromatischen heterocyclischen Ring kondensiert sein kann, und dieser (kondensierte) homoaromatische Ring kann optional mit bis zu drei R9 substituiert sein;
R9 aus -OH; geradkettigem, verzweigtem oder cyclischem Alkoxy, welches gesättigt, ungesättigt oder partiell ungesättigt sein kann; Halogen; Haloalkyl; $C_{1-6}$ Alkyl; $-NH_2$; $-NO_2$; -CN und Phenyl ausgewählt ist;
R10 aus H, Halogen, Haloalkyl, $C_{1-6}$ Alkyl ausgewählt ist;
n 1 oder 2 ist;
V C oder S ist; W O oder S ist; und
T, falls V S ist, entweder O repräsentiert oder abwesend ist, oder, falls V C ist, abwesend ist;

oder ein Salz davon,

**10.** Verbindung nach Anspruch 9, mit der Formel (VIa)

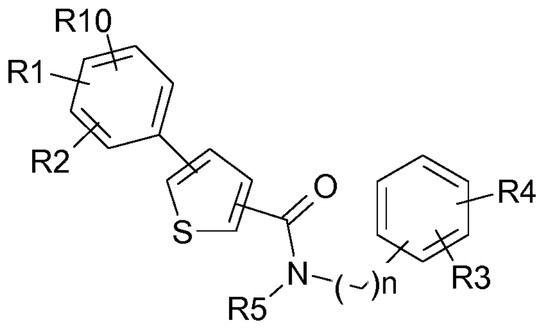

(VIa)

worin die Variablen R1, R2, R3, R4, R5, R10 und n die gleiche Bedeutung als im Ansprüch 9 haben, oder ein Salz davon.

**11.** Verbindung nach Anspruch 9 oder 10, worin die Variablen, falls vorhanden, die folgende Bedeutung haben:

R1 und R3 sind unabhängig voneinander aus H; -OH; geradkettigem, verzweigtem oder cyclischem Alkoxy, welches gesättigt, ungesättigt oder partiell ungesättigt sein kann; geradkettigem, verzweigtem oder cyclischem Alkyl, welches gesättigt, ungesättigt oder partiell ungesättigt sein kann; $-N(R8)_2$;-Halogen; Haloalkyl und Phenyl, ausgewählt;
R2 und R4 unabhängig voneinander aus H; -OR8; -COR8; -COOR8; -CONHR8; - OCOR8; $-SO_2N(R8)_2$; $-N(R8)_2$; -NHCOR8; -NHSOR8; -Halogen; $-CH_2N(R8)_2$;$-CH_2OR8$; Aryl optional mit bis zu drei R8 substituiert; $C_{1-6}$ Alkyl optional mit bis zu drei R8 substituiert, und Halo $C_{1-6}$ Alkyl ausgewählt sind;
R5 $C_{1-6}$ Alkyl ist;
R8 aus H; $C_{1-6}$ Alkyl optional mit bis zu drei R9 substituiert; Halogen; Halo $C_{1-6}$ Alkyl; -OH; $C_{1-6}$ Alkoxy; $-NH_2$; und einem 5- oder 6-gliedrigen aromatischen oder heteroaromatischen Ring optional mit bis zu drei R9 substituiert, ausgewählt ist;
R9 aus -OH; geradkettigem, verzweigtem oder cyclischem Alkoxy, welches gesättigt, ungesättigt oder partiell ungesättigt sein kann; Halogen, Haloalkyl; $C_{1-4}$ Alkyl; $-NH_2$ und Phenyl ausgewählt ist; und/oder
n 1 ist.

**12.** Verbindung nach Anspruch 11, worin R5 Methyl, Isopropyl or Cyclopropyl ist und/oder R8 Phenyl oder Benzyl ist.

**13.** Verbindung nach Anspruch 9, mit

(i) der Formel (VIIa)

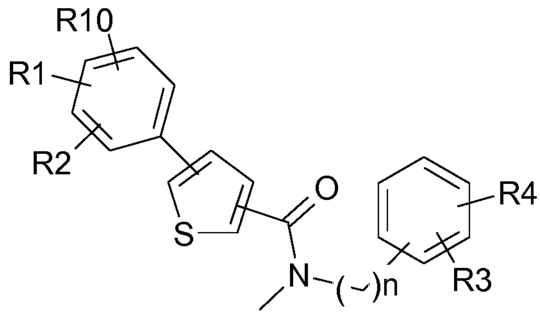

(VIIa)

worin die Variablen R1 and R3 unabhängig voneinander aus H, -OH, -OMe, -Me, $-NMe_2$ und Phenyl; R2, R4 und R10 unabhängig voneinander aus H und Halogen ausgewählt sind; und n 1 ist; oder ein Salz davon, oder
(ii) mit der Formel (VIIIa)

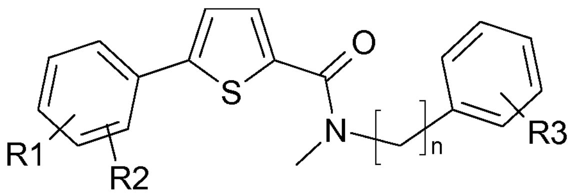

(VIIIa)

worin R1 und R3 unabhängig voneinander aus -OH, $-OCH_3$ und $-CH_3$ ausgewählt sind; R2 H, Fluoro or Methyl ist; und n 1 ist; oder ein Salz davon ist.

**14.** Verbindung nach Anspruch 9, ausgewählt aus

*N*-(3-Methoxyphenethyl)-5-(3-methoxyphenyl)-*N*-methylthiophen-2-carboxamid (17),
*N*-(3-Hydroxyphenethyl)-5-(3-hydroxyphenyl)-*N*-methylthiophen-2-carboxamid (18),
*N*-(3-Methoxyphenethyl)-*N*-methyl-5-phenylthiophen-2-carboxamid (19),
*N*-(3-Hydroxyphenethyl)-*N*-methyl-5-phenylthiophen-2-carboxamid (20),
5-(3-Fluorophenyl)-*N*-(3-methoxyphenethyl)-*N*-methylthiophen-2-carboxamid (21),
5-(3-Fluorophenyl)-*N*-(3-hydroxyphenethyl)-*N*-methylthiophen-2-carboxamid (22),
*N*-(3-Methoxybenzyl)-5-(3-methoxyphenyl)-*N*-methylthiophen-2-carboxamid (23),
*N*-(3-Hydroxybenzyl)-5-(3-hydroxyphenyl)-*N*-methylthiophen-2-carboxamid (24),
*N*-(3-Methoxybenzyl)-5-(4-methoxyphenyl)-*N*-methylthiophen-2-carboxamid (25),
*N*-(3-Hydroxybenzyl)-5-(4-hydroxyphenyl)-*N*-methylthiophen-2-carboxamid (26),
*N*-(3-Methoxybenzyl)-5-(2-methoxyphenyl)-*N*-methylthiophen-2-carboxamid (27),
*N*-(3-Hydroxybenzyl)-5-(2-hydroxyphenyl)-*N*-methylthiophen-2-carboxamid (28),
*N*-(3-Methoxybenzyl)-*N*-methyl-5-phenylthiophen-2-carboxamid (29),
*N*-(3-Hydroxybenzyl)-*N*-methyl-5-phenylthiophen-2-carboxamid (30),
5-(4-Cyanophenyl)-*N*-(3-methoxybenzyl)-*N*-methylthiophen-2-carboxamid (31),
5-(4-Cyanophenyl)-*N*-(3-hydroxybenzyl)-*N*-methylthiophen-2-carboxamid (32),
*N*-Benzyl-5-(3-methoxyphenyl)-*N*-methylthiophen-2-carboxamid (33),
*N*-Benzyl-5-(3-hydroxyphenyl)-*N*-methylthiophen-2-carboxamid (34),
*N*-Benzyl-5-(4-methoxyphenyl)-*N*-methylthiophen-2-carboxamid (35),
*N*-Benzyl-5-(4-hydroxyphenyl)-*N*-methylthiophen-2-carboxamid (36),
*N*-Benzyl-5-(2-methoxyphenyl)-*N*-methylthiophen-2-carboxamid (37),
*N*-Benzyl-5-(2-hydroxyphenyl)-*N*-methylthiophen-2-carboxamid (38),
*N*-(3-Hydroxybenzyl)-5-(4-methoxyphenyl)-*N*-methylthiophen-2-carboxamid (39),
5-(3-(Dimethylamino)phenyl)-*N*-(3-hydroxybenzyl)-*N*-methylthiophen-2-carboxamid (40),
5-(3-Fluorophenyl)-*N*-(3-hydroxybenzyl)-*N*-methylthiophen-2-carboxamid (41),
5-(4-Fluorophenyl)-*N*-(3-hydroxybenzyl)-*N*-methylthiophen-2-carboxamid (42),
5-(2-Fluoro-3-methoxyphenyl)-*N*-(3-hydroxybenzyl)-*N*-methylthiophen-2-carboxamid (43),
5-(2-Fluoro-3-hydroxyphenyl)-*N*-(3-hydroxybenzyl)-*N*-methylthiophen-2-carboxamid (44),
*N*-(3-Hydroxybenzyl)-5-(3-methoxyphenyl)-*N*-methylthiophen-2-carboxamid (45), und
*N*-(3-Hydroxybenzyl)-*N*-methyl-5-m-tolylthiophen-2-carboxamid (46),

vorzugsweise eine Verbindung ausgewählt aus der Verbindungen (28), (30), (39), (43), (44) und (46), oder ein Salz davon.

**15.** Verbindung mit 17β-HSD2 inhibitorischer Aktivität

*N*,5-Bis(3-methoxyphenyl)-*N*-methylthiophen-2-carboxamid (47),
*N*,5-Bis(3-hydroxyphenyl)-*N*-methylthiophen-2-carboxamid (48),
5-(2-Methoxyphenyl)-*N*-(3-methoxyphenyl)-*N*-methylthiophen-2-carboxamid (49),
5-(2-Hydroxyphenyl)-*N*-(3-hydroxyphenyl)-*N*-methylthiophen-2-carboxamid (50),
*N*-(3-Methoxyphenyl)-*N*-methyl-5-phenylthiophen-2-carboxamid (51),
*N*-(3-Hydroxyphenyl)-*N*-methyl-5-phenylthiophen-2-carboxamid (52),
5-(4-Cyanophenyl)-*N*-(3-methoxyphenyl)-*N*-methylthiophen-2-carboxamid (53),
5-(4-Cyanophenyl)-*N*-(3-hydroxyphenyl)-*N*-methylthiophen-2-carboxamid (54),
*N*-(3-Methoxyphenyl)-5-(4-methoxyphenyl)-*N*-methylthiophen-2-carboxamid (55),
5-(2-Fluoro-3-methoxyphenyl)-*N*-(3-methoxyphenyl)-*N*-methylthiophen-2-carboxamid (56),
5-(2,4-Dimethoxyphenyl)-*N*-(3-methoxyphenyl)-*N*-methylthiophen-2-carboxamid (57),
5-(3-(Dimethylamino)phenyl)-*N*-(3-methoxyphenyl)-*N*-methylthiophen-2-carboxamid (58),
5-(3-Fluorophenyl)-*N*-(3-methoxyphenyl)-*N*-methylthiophen-2-carboxamid (59),
5-(3,4-Difluorophenyl)-*N*-(3-methoxyphenyl)-*N*-methylthiophen-2-carboxamid (60),
5-(3-Fluoro-4-methoxyphenyl)-*N*-(3-methoxyphenyl)-*N*-methylthiophen-2-carboxamid (61),
5-(4-Fluoro-3-methoxyphenyl)-*N*-(3-methoxyphenyl)-*N*-methylthiophen-2-carboxamid (62),
*N*-(3-Methoxyphenyl)-*N*-methyl-5-(3-(methylthio)phenyl)thiophen-2-carboxamid (63),
*N*-(3-Methoxyphenyl)-*N*-methyl-5-m-tolylthiophen-2-carboxamid (64),
5-(2,6-Difluoro-3-methoxyphenyl)-*N*-(3-methoxyphenyl)-*N*-methylthiophen-2-carboxamid (65),
5-(2-Fluoro-3-methylphenyl)-N-(3-methoxyphenyl)-N-methylthiophen-2-carboxamid (66),
5-(3-Methoxyphenyl)-*N*-methyl-*N*-phenylthiophen-2-carboxamid (67),
5-(3-Hydroxyphenyl)-*N*-methyl-*N*-phenylthiophen-2-carboxamid (68),

*N*-Methyl-*N*,5-diphenylthiophen-2-carboxamid (69),
5-(3-Methoxyphenyl)-*N*-methyl-*N*-m-tolylthiophen-2-carboxamid (70),
5-(3-Hydroxyphenyl)-*N*-methyl-*N*-m-tolylthiophen-2-carboxamid (71),
5-(2-Fluoro-3-methoxyphenyl)-*N*-methyl-*N*-(*m*-tolylthiophen)-2-carboxamid (72),
5-(2-Fluoro-3-methoxyphenyl)-*N*-(4-methoxyphenyl)-*N*-methylthiophen-2-carboxamid (73),
*N*-(2-Fluorophenyl)-*N*-methyl-5-m-tolylthiophen-2-carboxamid (74),
5-(3-Methoxyphenyl)-*N*-methyl-*N*-(3-(trifluoromethyl)phenyl)thiophen-2-carboxamid (75),
*N*-(Biphenyl-3-yl)-5-(3-methoxyphenyl)-*N*-methylthiophen-2-carboxamid (76),
5-(2-Fluoro-3-methoxyphenyl)-*N*-(3-methoxyphenyl)thiophen-2-carboxamid (77) und
5-(2-Fluoro-3-methoxyphenyl)-N-(3-methoxyphenyl)-N-phenylthiophen-2-carboxamid (78), oder ein Salz davon.

**16.** Verfahren zur Synthese einer Verbindung mit der Formel (Va) oder ein Salz davon, nach irgendeinem der Ansprüche 9 bis 14, das die Kondensierung einer aktivierten Acyl- oder Sulfonyl-Verbindung mit der Formel (B) mit einer sekundären Amine mit der Formel (C), und einer daran anschließenden Kopplung mit einer Verbindung mit der Formel (A) umfasst.

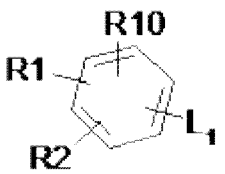

A

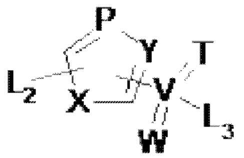

B

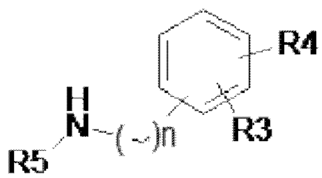

C

worin alle Variablen wie im Anspruch 9 definiert sind, und L1, L2 und L3 Abgangsgruppen sind.

**17.** Pharmazeutische Zusammensetzung oder Medikament mit mindestens einer Verbindung mit 17β-HSD2 inhibitorischer Aktivität gemäß irgendeinem der Ansprüche 9 bis 15 oder ein pharmazeutisch akzeptiertes Salz davon, und optional einem geeigneten Träger oder Hilfsstoff.

**18.** Pharmazeutische Zusammensetzung oder Medikament nach Anspruch 17, die zur Behandlung und Vorbeugung von Sexualhormon-defizienten Erkrankungen, inklusive Erkrankungen verursacht durch eine Fehlbalanz in OB/OC Aktivität wie Osteoporose und Osteopenie, Knochenheilung, post-menopausale Symptome, vaginale Atrophie, Kolonkarzinom, neuronale Erkrankungen wie Alzheimer und Morbus Parkison, Depression, Angst, Hypercholesterinämie, kardiovaskuläre Erkrankungen, Haarausfall, rheumatische Erkrankungen und inflammatorische Erkrankungen geeignet ist, vorzugsweise zur Behandlung und Vorbeugung von Osteoporose.

**19.** Verwendung einer Verbindung mit 17β-HSD2 inhibitorischer Aktivität nach irgendeinem der Ansprüche 9 bis 15, oder ein pharmazeutisch geeignetes Salz davon zur Herstellung eines Medikaments für die Behandlung und Vorbeugung von Sexualhormon-defizienten Erkrankungen.

**20.** Verbindung zur Verwendung in der Behandlung und/oder Vorbeugung von Sexualhormon-defizienten Erkrankungen im Anspruch 1 mit der Formel (IVb)

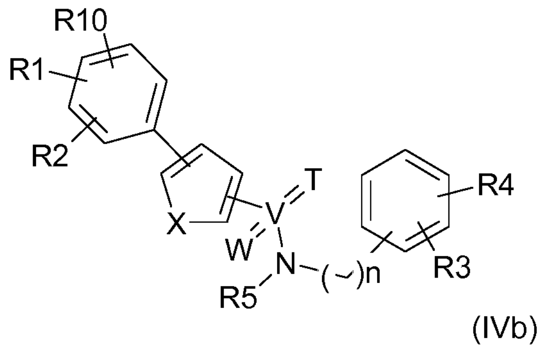

(IVb) ,

worin

R1 und R3 unabhängig voneinander aus H; -OH; geradkettigem, verzweigtem oder cyclischem Alkoxy, welches gesättigt, ungesättigt oder partiell ungesättigt sein kann; Acyloxy; geradkettigem, verzweigtem oder cyclischem Alkyl, welches gesättigt, ungesättigt oder partiell ungesättigt sein kann; $-N(R8)_2$; -SR8; - Halogen; Haloalkyl und Phenyl, ausgewählt sind;
R2 und R4 unabhängig voneinander aus H; -OR8; -COR8; -COOR8; -CONHR8; - OCOR8; -SR8; $-SON(R8)_2$; $-SO_2N(R8)_2$; $-N(R8)_2$; -NHCOR8; -NHSOR8; $-NHSO_2R8$; -Halogen; $-NO_2$; -CN; $-SO_2R8$; $-CH_2N(R8)_2$; $-CH_2OR8$; Aryl optional mit bis zu drei R8 substituiert; und geradkettigem, verzweigtem, oder cyclischem Alkyl, welches gesättigt, ungesättigt oder partiell ungesättigt, und optional mit bis zu drei R8 substituiert, ausgewählt sind;
R5 $C_{1-6}$ Alkyl ist;
R8 aus H; geradkettigem, verzweigtem, oder cyclischem Alkyl, welche gesättigt, ungesättigt oder partiell ungesättigt und optional mit bis zu drei R9 substituiert sein kann; -OH; $-NO_2$; $C_{1-6}$ Alkoxy; $C_{1-6}$ Alkyl; Halogen; Haloalkyl; $-NH_2$; -CN; - NHCOR9; -CONHR9; $-NHSO_2R9$; $-SO_2NHR9$; einem 5- or 6-gliedrigen aromatischen oder heteroaromatischen Arylring, welcher optional mit bis zu drei R9 substituiert sein kann; einem homoaromatischen Ring der mit einem 5- or 6-gliedrigen, aliphatischen oder aromatischen heterocyclischen Ring kondensiert sein kann, und dieser (kondensierte) homoaromatische Ring kann optional mit bis zu drei R9 substituiert sein;
R9 aus -OH; geradkettigem, verzweigtem oder cyclischem Alkoxy, welches gesättigt, ungesättigt oder partiell ungesättigt sein kann; Halogen; Haloalkyl; $C_{1-6}$ Alkyl; $-NH_2$; $-NO_2$; -CN und Phenyl ausgewählt ist;
R10 aus H, Halogen, Haloalkyl, $C_{1-6}$ Alkyl ausgewählt ist;
n ein Integer aus 0 bis 2 ausgewählt ist;
X O ist;
V C oder S ist; W O oder S ist; und
T, falls V S ist, entweder O repräsentiert oder abwesend ist, oder, falls V C ist, abwesend ist;

oder ein Salz davon,
zur Verwendung in der Behandlung und/oder Vorbeugung von inflammatorischen Erkrankungen.

**21.** Verbindung mit der Formel (IVb) zur Verwendung in der Behandlung und/oder Vorbeugung von inflammatorischen Erkrankungen nach Anspruch 20, worin die Variablen, falls vorhanden, die folgende Bedeutung haben:

R1 und R3 unabhängig voneinander aus H; -OH; geradkettigem, verzweigtem oder cyclischem Alkoxy, welches gesättigt, ungesättigt oder partiell ungesättigt sein kann; geradkettigem, verzweigtem oder cyclischem Alkyl, welches gesättigt, ungesättigt oder partiell ungesättigt sein kann; $-N(R8)_2$; -Halogen; Haloalkyl und Phenyl, ausgewählt sind;
R2 und R4 unabhängig voneinander aus H; -OR8; -COR8; -COOR8; -CONHR8; - OCOR8; $-SO_2N(R8)_2$; $-N(R8)_2$; -NHCOR8; $-NHSO_2R8$; Halogen; $-CH_2N(R8)_2$; - $CH_2OR8$; Aryl optional mit bis zu drei R8 substituiert; $C_{1-6}$ Alkyl optional mit bis zu drei R8 substituiert; und halo $C_{1-6}$ Alkyl, ausgewählt sind;
R5 $C_{1-6}$ Alkyl ist;
R8 aus H; $C_{1-6}$ Alkyl optional mit bis zu drei R9; Halogen; Halo $C_{1-6}$ Alkyl; -OH; $C_{1-6}$ Alkoxy; $-NH_2$; und einem 5- or 6-gliedrigen aromatischen oder heteroaromatischen Ring, optional mit bis zu drei R9 substituiert, ausgewählt ist; R9 aus -OH; geradkettigem, verzweigtem oder cyclischem Alkoxy, welches gesättigt, ungesättigt oder partiell ungesättigt sein kann; Halogen; Haloalkyl; $C_{1-4}$ Alkyl; $-NH_2$ and Phenyl ausgewählt ist; und/oder n 0

oder 1 ist.

**22.** Verbindung mit der Formel (IVb) zur Verwendung in der Behandlung und/oder Vorbeugung von inflammatorischen Erkrankungen laut Anspruch 21, worin R5 Methyl, Isopropyl oder Cyclopropyl und/oder R8 Phenyl oder Benzyl sind.

## Revendications

**1.** Un composé de formule (IV) ayant une action inhibitrice sur l'enzyme 17bêta-hydroxystéroïde déhydrogénase type 2 (17β-HSD2)

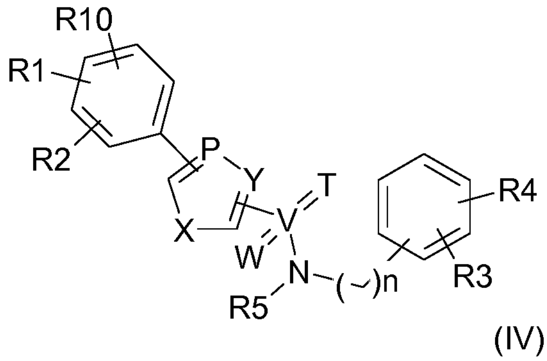

(IV),

pour lequel

R1 et R3 sont sélectionnés, indépendamment l'un de l'autre, parmi H; -OH; alkoxy linéaire, ramifié ou cyclique pouvant être saturé, insaturé ou partiellement insaturé; acyloxy; alkyle linéaire, ramifié ou cyclique pouvant être saturé, insaturé ou partiellement insaturé; $-N(R8)_2$; -SR8; halogène; haloalkyle et phényle;
R2 et R4 sont sélectionnés, indépendamment l'un de l'autre, parmi H; -OR8; - COR8; -COOR8; -CONHR8; -OCOR8; -SR8; $-SON(R8)_2$; $-SO_2N(R8)_2$; $-N(R8)_2$; - NHCOR8; -NHSOR8; $-NHSO_2R8$; -halogène; $-NO_2$; -CN; $-SO_2R8$; $-CH_2N(R8)_2$; - $CH_2OR8$; aryle optionnellement substitué par 3 groupes R8 au maximum; et alkyle linéaire, ramifié ou cyclique pouvant être saturé, insaturé ou partiellement insaturé et pouvant être optionnellement substitué par 3 groupes R8 au maximum;
R5 représente un alkyle $C_{1-6}$;
R8 est sélectionné parmi H; alkyle linéaire, ramifié ou cyclique pouvant être saturé, insaturé ou partiellement insaturé et pouvant être optionnellement substitué par 3 groupes R9 au maximum; -OH; $-NO_2$; alkoxy $C_{1-6}$; alkyle $C_{1-6}$; halogène; haloalkyle; $-NH_2$; -CN; -NHCOR9; -CONHR9; $-NHSO_2R9$; $-SO_2NHR9$; cycle aromatique ou hétéoaromatique à 5- ou 6-atomes pouvant être optionnellement substitué par 3 groupes R9 au maximum; un cycle homoaromatique qui peut être condensé avec un cycle à 5- ou 6-atomes, aliphatique ou un hétérocycle aromatique, et ce cycle homoaromatique (condensé) peut être optionnellement substitué par 3 groupes R9 au maximum;
R9 est sélectionné parmi -OH; alkoxy linéaire, ramifié ou cyclique pouvant être saturé, insaturé ou partiellement insaturé; halogène; haloalkyle; alkyle $C_{1-6}$; - $NH_2$; $-NO_2$; -CN et phényle;
R10 est sélectionné parmi H, halogène, haloalkyle, alkyle $C_{1-6}$;
n est un nombre entier allant de 0 à 2;
X, Y et P sont sélectionnés, indépendamment l'un de l'autre, parmi CH, N, N(H), S et O;
V représente un C ou un S; W représente un O ou un S; et
T, si V est un S, représente O ou est absent, ou, si V est un C, est absent;

ou un sel de ces dérivés,
pour l'utilisation dans le traitement et/ou la prévention de maladies dues à un déficit en stéroïdes sexuels.

**2.** Composé pour l'utilisation dans le traitement et/ou la prévention de maladies dues à un déficit en stéroïdes sexuels selon la revendication 1, pour lequel le composé est de formule (IVb)

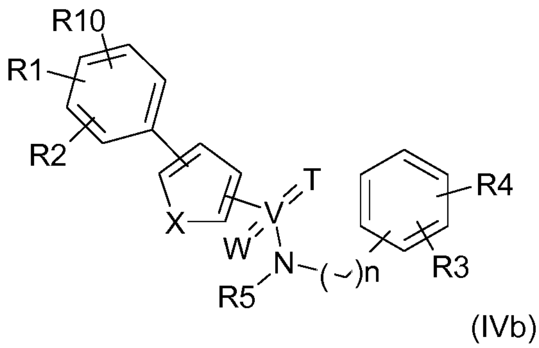

(IVb),

pour lequel les variables R1, R2, R3, R4, R5, R10, n, X, V, W et T, ont la même signification que dans la revendication 1, ou un sel de ces dérivés.

**3.** Composé pour l'utilisation dans le traitement et/ou la prévention de maladies dues à un déficit en stéroïdes sexuels selon la revendication 1, pour lequel le composé est de formule (Va)

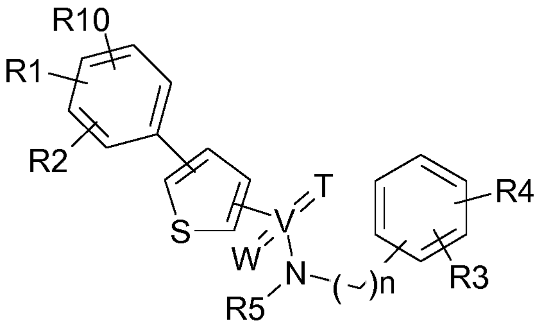

(Va)

pour lequel les variables R1, R2, R3, R4, R5, R10, n, V, W et T ont la même signification que dans la revendication 1, ou un sel de ces dérivés,
de préférence le composé est de formule (VIa)

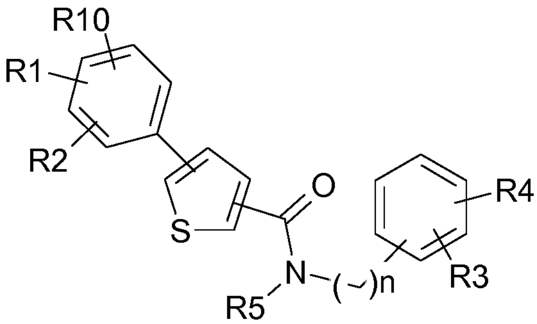

(VIa)

pour lequel les variables R1, R2, R3, R4, R5, R10 et n ont la même signification que dans la revendication 1, ou un sel de ces dérivés.

**4.** Composé pour l'utilisation dans le traitement et/ou la prévention de maladies dues à un déficit en stéroïdes sexuels selon l'une quelconque des revendications 1 à 3, pour lequel les variables, si présentes, ont la signification suivante:

R1 et R3 sont sélectionnés, indépendamment l'un de l'autre, parmi H; -OH; alkoxy linéaire, ramifié ou cyclique pouvant être saturé, insaturé ou partiellement insaturé; alkyle linéaire, ramifié ou cyclique pouvant être saturé, insaturé ou partiellement insaturé; $-N(R8)_2$; halogène; haloalkyle et phényle;
R2 et R4 sont sélectionnés, indépendamment l'un de l'autre, parmi H; -OR8;-COR8; -COOR8; -CONHR8; -OCOR8; $-SO_2N(R8)_2$; $-N(R8)_2$; -NHCOR8;$-NHSO_2R8$; halogène; $-CH_2N(R8)_2$; $-CH_2OR8$; aryle optionnellement substitué par 3 groupes R8 au maximum; alkyle $C_{1-6}$ pouvant être optionnellement substitué par 3 groupes R8 au maximum et un haloalkyle $C_{1-6}$;
R5 représente un alkyle $C_{1-6}$;
R8 est sélectionné parmi H, alkyle $C_{1-6}$ pouvant être optionnellement substitué par 3 groupes R9 au maximum; halogène; haloalkyle $C_{1-6}$; -OH; alkoxy $C_{1-6}$;$-NH_2$; et un cycle aromatique ou hétéoaromatique à 5- ou 6-atomes pouvant être optionnellement substitué par 3 groupes R9 au maximum;
R9 est sélectionné parmi -OH; alkoxy linéaire, ramifié ou cyclique pouvant être saturé, insaturé ou partiellement insaturé; halogène; haloalkyle; alkyle $C_{1-4}$;$-NH_2$ et phényle;
X, Y et P sont sélectionnés, indépendamment l'un de l'autre, parmi CH, N, N(H), et S, et/ou
n est soit 0 soit 1;

**5.** Composé pour l'utilisation dans le traitement et/ou la prévention de maladies dues à un déficit en stéroïdes sexuels selon la revendication 4, pour lequel R5 représente un groupe méthyle, isopropyle ou cyclopropyle et/ou R8 représente un phényle ou un benzyle.

**6.** Composé pour l'utilisation dans le traitement et/ou la prévention de maladies dues à un déficit en stéroïdes sexuels selon la revendication 1, pour lequel le composé

(i) est de formule (VIIa)

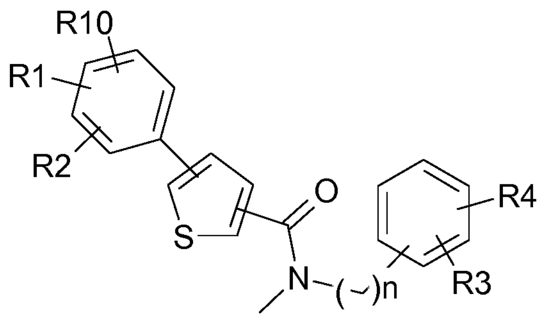

(VIIa)

pour lequel les variables R1 et R3 sont sélectionnées, indépendamment l'un de l'autre, parmi H, -OH, -OMe, -Me, $-NMe_2$ et phényle; R2, R4 et R10 sont sélectionnés, indépendamment l'un de l'autre, parmi -H et -halogène et n est soit 0 soit 1; ou un sel de ces dérivés, ou
(ii) est de formule (VIIIa)

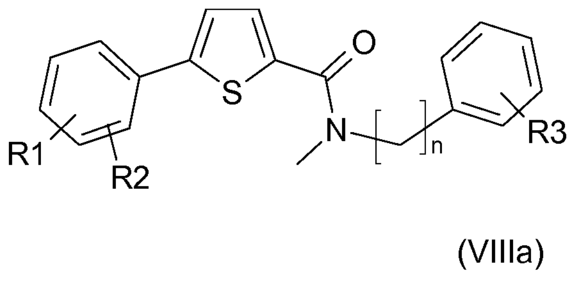

(VIIIa)

pour lequel R1 et R3 sont sélectionnés, indépendamment l'un de l'autre, parmi - OH, -OMe et -Me; R2 représente un H, -fluor ou méthyle; et n est soit 0 soit 1; ou un sel de ces dérivés.

**7.** Composé pour l'utilisation dans le traitement et/ou la prévention de maladies dues à un déficit en stéroïdes sexuels

selon la revendication 1, pour lequel le composé est sélectionné parmi

*N*-(3-methoxyphenethyl)-5-(3-methoxyphenyl)-*N*-methylthiophene-2-carboxamide (17),
*N*-(3-hydroxyphenethyl)-5-(3-hydroxyphenyl)-N-methylthiophene-2-carboxamide (18),
*N*-(3-methoxyphenethyl)-*N*-methyl-5-phenylthiophene-2-carboxamide (19),
*N*-(3-hydroxyphenethyl)-*N*-methyl-5-phenylthiophene-2-carboxamide (20),
5-(3-fluorophenyl)-*N*-(3-methoxyphenethyl)-*N*-methylthiophene-2-carboxamide (21),
5-(3-fluorophenyl)-*N*-(3-hydroxyphenethyl)-*N*-methylthiophene-2-carboxamide (22),
*N*-(3-methoxybenzyl)-5-(3-methoxyphenyl)-*N*-methylthiophene-2-carboxamide (23),
*N*-(3-hydroxybenzyl)-5-(3-hydroxyphenyl)-*N*-methylthiophene-2-carboxamide (24),
*N*-(3-methoxybenzyl)-5-(4-methoxyphenyl)-*N*-methylthiophene-2-carboxamide (25),
*N*-(3-hydroxybenzyl)-5-(4-hydroxyphenyl)-*N*-methylthiophene-2-carboxamide (26),
*N*-(3-methoxybenzyl)-5-(2-methoxyphenyl)-*N*-methylthiophene-2-carboxamide (27),
*N*-(3-hydroxybenzyl)-5-(2-hydroxyphenyl)-*N*-methylthiophene-2-carboxamide (28),
*N*-(3-methoxybenzyl)-*N*-methyl-5-phenylthiophene-2-carboxamide (29),
*N*-(3-hydroxybenzyl)-*N*-methyl-5-phenylthiophene-2-carboxamide (30),
5-(4-cyanophenyl)-*N*-(3-methoxybenzyl)-*N*-methylthiophene-2-carboxamide (31),
5-(4-cyanophenyl)-*N*-(3-hydroxybenzyl)-*N*-methylthiophene-2-carboxamide (32),
*N*-benzyl-5-(3-methoxyphenyl)-*N*-methylthiophene-2-carboxamide (33),
*N*-benzyl-5-(3-hydroxyphenyl)-*N*-methylthiophene-2-carboxamide (34),
*N*-benzyl-5-(4-methoxyphenyl)-*N*-methylthiophene-2-carboxamide (35),
*N*-benzyl-5-(4-hydroxyphenyl)-*N*-methylthiophene-2-carboxamide (36),
*N*-benzyl-5-(2-methoxyphenyl)-*N*-methylthiophene-2-carboxamide (37),
*N*-benzyl-5-(2-hydroxyphenyl)-*N*-methylthiophene-2-carboxamide (38),
*N*-(3-hydroxybenzyl)-5-(4-methoxyphenyl)-*N*-methylthiophene-2-carboxamide (39),
5-(3-(dimethylamino)phenyl)-*N*-(3-hydroxybenzyl)-*N*-methylthiophene-2-carboxamide (40),
5-(3-fluorophenyl)-*N*-(3-hydroxybenzyl)-*N*-methylthiophene-2-carboxamide (41),
5-(4-fluorophenyl)-*N*-(3-hydroxybenzyl)-*N*-methylthiophene-2-carboxamide (42),
5-(2-fluoro-3-methoxyphenyl)-*N*-(3-hydroxybenzyl)-*N*-methylthiophene-2-carboxamide (43),
5-(2-fluoro-3-hydroxyphenyl)-*N*-(3-hydroxybenzyl)-*N*-methylthiophene-2-carboxamide (44),
*N*-(3-hydroxybenzyl)-5-(3-methoxyphenyl)-*N*-methylthiophene-2-carboxamide (45),
*N*-(3-hydroxybenzyl)-*N*-methyl-5-m-tolylthiophene-2-carboxamide (46),
*N*,5-bis(3-methoxyphenyl)-*N*-methylthiophene-2-carboxamide (47),
*N*,5-bis(3-hydroxyphenyl)-*N*-methylthiophene-2-carboxamide (48),
5-(2-methoxyphenyl)-*N*-(3-methoxyphenyl)-*N*-methylthiophene-2-carboxamide (49),
5-(2-hydroxyphenyl)-*N*-(3-hydroxyphenyl)-*N*-methylthiophene-2-carboxamide (50),
*N*-(3-methoxyphenyl)-*N*-methyl-5-phenylthiophene-2-carboxamide (51),
*N*-(3-hydroxyphenyl)-*N*-methyl-5-phenylthiophene-2-carboxamide (52),
5-(4-cyanophenyl)-*N*-(3-methoxyphenyl)-*N*-methylthiophene-2-carboxamide (53),
5-(4-cyanophenyl)-*N*-(3-hydroxyphenyl)-*N*-methylthiophene-2-carboxamide (54),
*N*-(3-methoxyphenyl)-5-(4-methoxyphenyl)-*N*-methylthiophene-2-carboxamide (55),
5-(2-fluoro-3-methoxyphenyl)-*N*-(3-methoxyphenyl)-*N*-methylthiophene-2-carboxamide (56),
5-(2,4-dimethoxyphenyl)-*N*-(3-methoxyphenyl)-*N*-methylthiophene-2-carboxamide (57),
5-(3-(dimethylamino)phenyl)-*N*-(3-methoxyphenyl)-*N*-methylthiophene-2-carboxamide (58),
5-(3-fluorophenyl)-*N*-(3-methoxyphenyl)-*N*-methylthiophene-2-carboxamide (59),
5-(3,4-difluorophenyl)-*N*-(3-methoxyphenyl)-*N*-methylthiophene-2-carboxamide (60),
5-(3-fluoro-4-methoxyphenyl)-*N*-(3-methoxyphenyl)-*N*-methylthiophene-2-carboxamide (61),
5-(4-fluoro-3-methoxyphenyl)-*N*-(3-methoxyphenyl)-*N*-methylthiophene-2-carboxamide (62),
*N*-(3-methoxyphenyl)-*N*-methyl-5-(3-(methylthio)phenyl)thiophene-2-carboxamide (63),
*N*-(3-methoxyphenyl)-*N*-methyl-5-m-tolylthiophene-2-carboxamide (64),
5-(2,6-difluoro-3-methoxyphenyl)-*N*-(3-methoxyphenyl)-*N*-methylthiophene-2-carboxamide (65),
5-(2-fluoro-3-methylphenyl)-*N*-(3-methoxyphenyl)-*N*-methylthiophene-2-carboxamide (66),
5-(3-methoxyphenyl)-*N*-methyl-*N*-phenylthiophene-2-carboxamide (67),
5-(3-hydroxyphenyl)-*N*-methyl-*N*-phenylthiophene-2-carboxamide (68),
*N*-methyl-*N*,5-diphenylthiophene-2-carboxamide (69),
5-(3-methoxyphenyl)-*N*-methyl-*N*-m-tolylthiophene-2-carboxamide (70),
5-(3-hydroxyphenyl)-*N*-methyl-*N*-m-tolylthiophene-2-carboxamide (71),
5-(2-fluoro-3-methoxyphenyl)-*N*-methyl-*N*-(*m*-tolylthiophene)-2-carboxamide (72),

5-(2-fluoro-3-methoxyphenyl)-*N*-(4-methoxyphenyl)-*N*-methylthiophene-2-carboxamide (73),
*N*-(2-fluorophenyl)-*N*-methyl-5-m-tolylthiophene-2-carboxamide (74),
5-(3-methoxyphenyl)-*N*-methyl-*N*-(3-(trifluoromethyl)phenyl)thiophene-2-carboxamide (75),
*N*-(biphenyl-3-yl)-5-(3-methoxyphenyl)-*N*-methylthiophene-2-carboxamide (76),
5-(2-fluoro-3-methoxyphenyl)-*N*-(3-methoxyphenyl)thiophene-2-carboxamide (77) et
5-(2-fluoro-3-methoxyphenyl)-*N*-(3-methoxyphenyl)-*N*-phenylthiophene-2-carboxamide (78),

de préférence le composé est sélectionné parmi les composés (28), (30), (39), (43), (44), (46), (47), (55), (56), (58), (64), (65), (72) et (76), ou un sel de ces dérivés.

**8.** Composé pour l'utilisation dans le traitement et/ou la prévention de maladies dues à un déficit en stéroïdes sexuels selon l'une quelconque des revendications de 1 à 7, afin de traiter et prévenir les maladies causées par le déséquilibre entre l'activité des OB/OC comme dans l'ostéoporose et l'ostéopénie, la consolidation osseuse, les symptômes associés à la post-ménopause, l'atrophie du vagin, le cancer du colon, les maladies neuronales comme les maladies d'Alzheimer et de Parkinson, la dépression, l'anxiété, l'hypercholestérolémie, les maladies cardio-vasculaires, la chute des cheveux, les maladies rhumatismales et inflammatoires, une préférence est accordée au traitement et la prévention de l'ostéoporose.

**9.** Un composé de formule (Va) ayant une action inhibitrice sur l'enzyme 17bêta-hydroxystéroïde déhydrogénase type 2 (17β-HSD2)

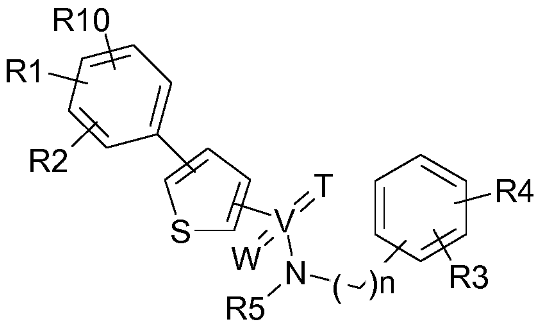

(Va)

pour lequel

R1 et R3 sont sélectionnés, indépendamment l'un de l'autre, parmi H; -OH; alkoxy linéaire, ramifié ou cyclique pouvant être saturé, insaturé ou partiellement insaturé; acyloxy; alkyle linéaire, ramifié ou cyclique pouvant être saturé, insaturé ou partiellement insaturé; $-N(R8)_2$; -SR8; halogène; haloalkyle et phényle;
R2 et R4 sont sélectionnés, indépendamment l'un de l'autre, parmi H; -OR8; - COR8; -COOR8; -CONHR8; -OCOR8; -SR8; $-SON(R8)_2$; $-SO_2N(R8)_2$; $-N(R8)_2$;-NHCOR8; -NHSOR8 ; $-NHSO_2R8$; halogène; $-NO_2$; -CN ; $-SO_2R8$ ; $-CH_2N(R8)_2$;$-CH_2OR8$; aryle optionnellement substitué par 3 groupes R8 au maximum; et un alkyle linéaire, ramifié ou cyclique pouvant être saturé, insaturé ou partiellement insaturé et pouvant être optionnellement substitué par 3 groupes R8 au maximum;
R5 représente un alkyle $C_{1-6}$;
R8 est sélectionné parmi H; alkyle linéaire, ramifié ou cyclique pouvant être saturé, insaturé ou partiellement insaturé et pouvant être optionnellement substitué par 3 groupes R9 au maximum; -OH; $-NO_2$; alkoxy $C_{1-6}$; alkyle $C_{1-6}$; halogène; haloalkyl; $-NH_2$; -CN; -NHCOR9; -CONHR9; $-NHSO_2R9$; $-SO_2NHR9$; un cycle aromatique ou hétéoaromatique à 5- ou 6-atomes pouvant être optionnellement substitué par 3 groupes R9 au maximum; un cycle homoaromatique qui peut être condensé avec un cycle à 5- ou 6-atomes, aliphatique ou un hétérocycle aromatique, et ce cycle homoaromatique (condensé) peut être optionnellement substitué par 3 groupes R9 au maximum; R9 est sélectionné parmi -OH; alkoxy linéaire, ramifié ou cyclique pouvant être saturé, insaturé ou partiellement insaturé ; halogène; haloalkyle; alkyle $C_{1-6}$;$-NH_2$; $-NO_2$; -CN et phényle;
R10 est sélectionné parmi H, halogène, haloalkyle, alkyle $C_{1-6}$;
n est soit 1 soit 2;
V représente un C ou un S; W représente un O ou un S; et
T, si V est un S, représente O ou est absent, ou, si V est un C, est absent;

ou un sel de ces dérivés.

**10.** Composé de formule (VIa) selon la revendication 9

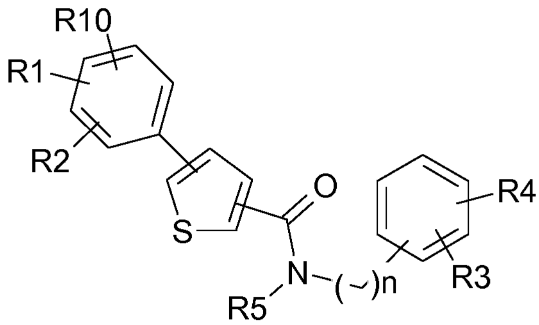

(VIa)

pour lequel les variables R1, R2, R3, R4, R5, R10 et n ont la même signification que dans la revendication 9, ou un sel de ces dérivés.

**11.** Composé selon l'une quelconque des revendications 9 ou 10, pour lequel les variables, si présentes, ont la signification suivante:

R1 et R3 sont sélectionnés, indépendamment l'un de l'autre, parmi H; -OH; alkoxy linéaire, ramifié ou cyclique pouvant être saturé, insaturé ou partiellement insaturé; alkyle linéaire, ramifié ou cyclique pouvant être saturé, insaturé ou partiellement insaturé; $-N(R8)_2$; halogène; haloalkyle et phényle;
R2 et R4 sont sélectionnés, indépendamment l'un de l'autre, parmi H; -OR8; - COR8; -COOR8; -CONHR8; -OCOR8; $-SO_2N(R8)_2$; $-N(R8)_2$; -NHCOR8; - $NHSO_2R8$; halogène; $-CH_2N(R8)_2$; $-CH_2OR8$; aryle optionnellement substitué par 3 groupes R8 au maximum; un alkyle $C_{1-6}$ pouvant être optionnellement substitué par 3 groupes R8 au maximum; et haloalkyle $C_{1-6}$;
R5 représente un alkyle $C_{1-6}$;
R8 est sélectionné parmi H; alkyle $C_{1-6}$ pouvant être optionnellement substitué par 3 groupes R9 au maximum; halogène; haloalkyle $C_{1-6}$; -OH; alkoxy $C_{1-6}$;$-NH_2$; et un cycle aromatique ou hétéoaromatique à 5- ou 6-atomes pouvant être optionnellement substitué par 3 groupes R9 au maximum;
R9 est sélectionné parmi -OH; alkoxy linéaire, ramifié ou cyclique pouvant être saturé, insaturé ou partiellement insaturé; halogène; haloalkyle; alkyle $C_{1-4}$;$-NH_2$ et phényle; et/ou
n est 1.

**12.** Composé selon la revendication 11, pour lequel R5 représente un méthyle, isopropyle ou cyclopropyle et/ou R8 représente un phényle ou benzyle.

**13.** Composé selon la revendication 9, qui

(i) est de formula (VIIa)

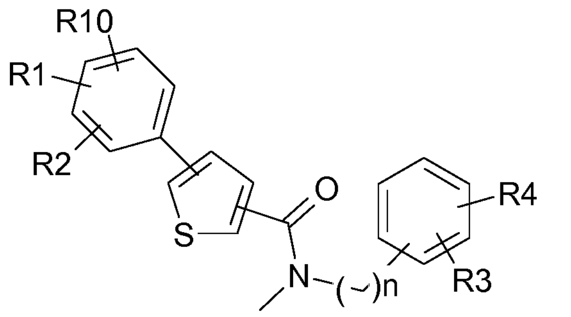

(VIIa)

pour lequel les variables R1 et R3 sont sélectionnées, indépendamment l'une de l'autre, parmi H, -OH, -OMe, -Me, $-NMe_2$ et phényle; R2, R4 et R10 sont sélectionnés, indépendamment l'un de l'autre, parmi H et halogène; et n est 1; ou un sel de ces dérivés, ou
(ii) est de formule (VIIIa)

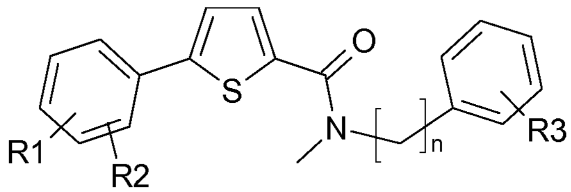

(VIIIa)

pour lequel R1 et R3 sont sélectionnés, indépendamment l'un de l'autre, parmi - OH, -OMe et -Me; R2 est sélectionné parmi H, -F ou -Me; et n est 1; ou un sel de ces dérivés.

**14.** Composé selon la revendication 9 sélectionné parmi

N-(3-methoxyphenethyl)-5-(3-methoxyphenyl)-N-methylthiophene-2-carboxamide (17),
N-(3-hydroxyphenethyl)-5-(3-hydroxyphenyl)-N-methylthiophene-2-carboxamide (18),
*N*-(3-methoxyphenethyl)-*N*-methyl-5-phenylthiophene-2-carboxamide (19),
*N*-(3-hydroxyphenethyl)-*N*-methyl-5-phenylthiophene-2-carboxamide (20),
5-(3-fluorophenyl)-*N*-(3-methoxyphenethyl)-*N*-methylthiophene-2-carboxamide (21),
5-(3-fluorophenyl)-*N*-(3-hydroxyphenethyl)-*N*-methylthiophene-2-carboxamide (22),
*N*-(3-methoxybenzyl)-5-(3-methoxyphenyl)-*N*-methylthiophene-2-carboxamide (23),
*N*-(3-hydroxybenzyl)-5-(3-hydroxyphenyl)-*N*-methylthiophene-2-carboxamide (24),
*N*-(3-methoxybenzyl)-5-(4-methoxyphenyl)-*N*-methylthiophene-2-carboxamide (25),
*N*-(3-hydroxybenzyl)-5-(4-hydroxyphenyl)-*N*-methylthiophene-2-carboxamide (26),
*N*-(3-methoxybenzyl)-5-(2-methoxyphenyl)-*N*-methylthiophene-2-carboxamide (27),
*N*-(3-hydroxybenzyl)-5-(2-hydroxyphenyl)-*N*-methylthiophene-2-carboxamide (28),
*N*-(3-methoxybenzyl)-*N*-methyl-5-phenylthiophene-2-carboxamide (29),
*N*-(3-hydroxybenzyl)-*N*-methyl-5-phenylthiophene-2-carboxamide (30),
5-(4-cyanophenyl)-*N*-(3-methoxybenzyl)-*N*-methylthiophene-2-carboxamide (31),
5-(4-cyanophenyl)-*N*-(3-hydroxybenzyl)-*N*-methylthiophene-2-carboxamide (32),
*N*-benzyl-5-(3-methoxyphenyl)-*N*-methylthiophene-2-carboxamide (33),
*N*-benzyl-5-(3-hydroxyphenyl)-*N*-methylthiophene-2-carboxamide (34),
*N*-benzyl-5-(4-methoxyphenyl)-*N*-methylthiophene-2-carboxamide (35),
*N*-benzyl-5-(4-hydroxyphenyl)-*N*-methylthiophene-2-carboxamide (36),
*N*-benzyl-5-(2-methoxyphenyl)-*N*-methylthiophene-2-carboxamide (37),
*N*-benzyl-5-(2-hydroxyphenyl)-*N*-methylthiophene-2-carboxamide (38),
*N*-(3-hydroxybenzyl)-5-(4-methoxyphenyl)-*N*-methylthiophene-2-carboxamide (39),
5-(3-(dimethylamino)phenyl)-*N*-(3-hydroxybenzyl)-*N*-methylthiophene-2-carboxamide (40),

5-(3-fluorophenyl)-*N*-(3-hydroxybenzyl)-*N*-methylthiophene-2-carboxamide (41),
5-(4-fluorophenyl)-*N*-(3-hydroxybenzyl)-*N*-methylthiophene-2-carboxamide (42),
5-(2-fluoro-3-methoxyphenyl)-*N*-(3-hydroxybenzyl)-*N*-methylthiophene-2-carboxamide (43),
5-(2-fluoro-3-hydroxyphenyl)-*N*-(3-hydroxybenzyl)-*N*-methylthiophene-2-carboxamide (44),
*N*-(3-hydroxybenzyl)-5-(3-methoxyphenyl)-*N*-methylthiophene-2-carboxamide (45), et
*N*-(3-hydroxybenzyl)-*N*-methyl-5-m-tolylthiophene-2-carboxamide (46),

de préférence le composé est sélectionné parmi les composés (28), (30), (39), (43), (44) et (46), ou un sel de ces dérivés.

**15.** Un composé ayant une action inhibitrice sur l'enzyme 17bêta-hydroxystéroïde déhydrogénase type 2 (17β-HSD2) :

*N*,5-bis(3-methoxyphenyl)-*N*-methylthiophene-2-carboxamide (47),
*N*,5-bis(3-hydroxyphenyl)-*N*-methylthiophene-2-carboxamide (48),
5-(2-methoxyphenyl)-*N*-(3-methoxyphenyl)-*N*-methylthiophene-2-carboxamide (49),
5-(2-hydroxyphenyl)-*N*-(3-hydroxyphenyl)-*N*-methylthiophene-2-carboxamide (50),
*N*-(3-methoxyphenyl)-*N*-methyl-5-phenylthiophene-2-carboxamide (51),
*N*-(3-hydroxyphenyl)-*N*-methyl-5-phenylthiophene-2-carboxamide (52),
5-(4-cyanophenyl)-*N*-(3-methoxyphenyl)-*N*-methylthiophene-2-carboxamide (53),
5-(4-cyanophenyl)-*N*-(3-hydroxyphenyl)-*N*-methylthiophene-2-carboxamide (54),
*N*-(3-methoxyphenyl)-5-(4-methoxyphenyl)-*N*-methylthiophene-2-carboxamide (55),
5-(2-fluoro-3-methoxyphenyl)-*N*-(3-methoxyphenyl)-*N*-methylthiophene-2-carboxamide (56),
5-(2,4-dimethoxyphenyl)-*N*-(3-methoxyphenyl)-*N*-methylthiophene-2-carboxamide (57),
5-(3-(dimethylamino)phenyl)-*N*-(3-methoxyphenyl)-*N*-methylthiophene-2-carboxamide (58),
5-(3-fluorophenyl)-*N*-(3-methoxyphenyl)-*N*-methylthiophene-2-carboxamide (59),
5-(3,4-difluorophenyl)-*N*-(3-methoxyphenyl)-*N*-methylthiophene-2-carboxamide (60),
5-(3-fluoro-4-methoxyphenyl)-*N*-(3-methoxyphenyl)-*N*-methylthiophene-2-carboxamide (61),
5-(4-fluoro-3-methoxyphenyl)-*N*-(3-methoxyphenyl)-*N*-methylthiophene-2-carboxamide (62),
*N*-(3-methoxyphenyl)-*N*-methyl-5-(3-(methylthio)phenyl)thiophene-2-carboxamide (63),
*N*-(3-methoxyphenyl)-*N*-methyl-5-m-tolylthiophene-2-carboxamide (64),
5-(2,6-difluoro-3-methoxyphenyl)-*N*-(3-methoxyphenyl)-*N*-methylthiophene-2-carboxamide (65),
5-(2-fluoro-3-methylphenyl)-*N*-(3-methoxyphenyl)-*N*-methylthiophene-2-carboxamide (66),
5-(3-methoxyphenyl)-*N*-methyl-*N*-phenylthiophene-2-carboxamide (67),
5-(3-hydroxyphenyl)-*N*-methyl-*N*-phenylthiophene-2-carboxamide (68),
*N*-methyl-*N*,5-diphenylthiophene-2-carboxamide (69), 5-(3-methoxyphenyl)-*N*-methyl-*N*-m-tolylthiophene-2-carboxamide (70),
5-(3-hydroxyphenyl)-*N*-methyl-*N*-m-tolylthiophene-2-carboxamide (71),
5-(2-fluoro-3-methoxyphenyl)-*N*-methyl-*N*-(m-tolylthiophene)-2-carboxamide (72),
5-(2-fluoro-3-methoxyphenyl)-*N*-(4-methoxyphenyl)-*N*-methylthiophene-2-carboxamide (73),
*N*-(2-fluorophenyl)-*N*-methyl-5-m-tolylthiophene-2-carboxamide (74),
5-(3-methoxyphenyl)-*N*-methyl-*N*-(3-(trifluoromethyl)phenyl)thiophene-2-carboxamide (75),
*N*-(biphenyl-3-yl)-5-(3-methoxyphenyl)-*N*-methylthiophene-2-carboxamide (76),
5-(2-fluoro-3-methoxyphenyl)-*N*-(3-methoxyphenyl)thiophene-2-carboxamide (77) et
5-(2-fluoro-3-methoxyphenyl)-*N*-(3-methoxyphenyl)-*N*-phenylthiophene-2-carboxamide (78),

ou un sel de ces dérivés.

**16.** Procédé de préparation du composé de formule (Va) ou un sel de ces dérivés selon l'une quelconque des revendications 9 à 14, qui inclut la condensation d'un dérivé acylé activé ou d'un dérivé sulfonyle de formule (B) avec une amine secondaire de formule (C), suivie par une réaction de cross-coupling avec le composé de formule (A)

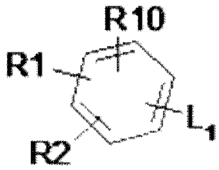 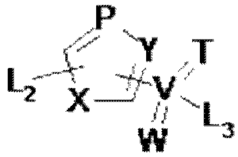 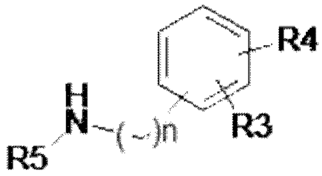

A B C

pour lequel toutes les variables sont définies dans la revendication 9, et L1, L2 and L3 représentent des groupes partants.

**17.** Composition pharmaceutique ou médicament incluant au moins un composé ayant une action inhibitrice sur l'enzyme 17bêta-hydroxystéroïde déhydrogénase type 2 (17β-HSD2) selon l'une quelconque des revendications 9 à 15 ou un sel de ce composé pharmaceutiquement acceptable, and optionnellement un transporteur approprié ou un excipient.

**18.** Composition pharmaceutique ou médicament selon la revendication 17, dans le traitement et la prévention de maladies dues à un déficit en stéroïdes sexuels incluant les maladies causées par le déséquilibre entre l'activité des OB/OC comme dans l'ostéoporose et l'ostéopénie, la consolidation osseuse, les symptômes associés à la post-ménopause, l'atrophie du vagin, le cancer du colon, les maladies neuronales comme les maladies d'Alzheimer et de Parkinson, la dépression, l'anxiété, l'hypercholestérolémie, les maladies cardio-vasculaires, la chute des cheveux, les maladies rhumatismales et inflammatoires, une préférence est accordée au traitement et la prévention de l'ostéoporose.

**19.** Utilisation d'un composé ayant une action inhibitrice sur l'enzyme 17bêta-hydroxystéroïde déhydrogénase type 2 (17β-HSD2) selon l'une quelconque des revendications 9 à 15 ou un sel de ce composé pharmaceutiquement acceptable pour la préparation d'un médicament pour le traitement et la prévention de maladies dues à un déficit en stéroïdes sexuels.

**20.** Composé pour l'utilisation dans le traitement et/ou la prévention de maladies dues à un déficit en stéroïdes sexuels selon la revendication 1, pour lequel le composé est de formule (IVb)

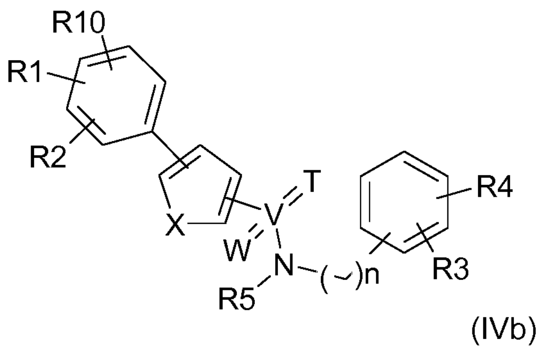

(IVb),

pour lequel

R1 et R3 sont sélectionnés, indépendamment l'un de l'autre, parmi H; -OH; alkoxy linéaire, ramifié ou cyclique pouvant être saturé, insaturé ou partiellement insaturé ; acyloxy; alkyle linéaire, ramifié ou cyclique pouvant être saturé, insaturé ou partiellement insaturé; $-N(R8)_2$; -SR8; halogène; haloalkyle et phényle;
R2 et R4 sont sélectionnés, indépendamment l'un de l'autre, parmi H; -OR8; - COR8; -COOR8; -CONHR8; -OCOR8; -SR8; $-SON(R8)_2$; $-SO_2N(R8)_2$; $-N(R8)_2$;-NHCOR8; -NHSOR8; $-NHSO_2R8$; -halogène; $-NO_2$; -CN; $-SO_2R8$; $-CH_2N(R8)_2$;$-CH_2OR8$; aryle optionnellement substitué par 3 groupes R8 au maximum; et un alkyle linéaire, ramifié ou cyclique pouvant être saturé, insaturé ou partiellement insaturé et pouvant être optionnellement substitué par 3 groupes R8 au maximum;

R5 représente un alkyle $C_{1-6}$;
R8 est sélectionné parmi H; alkyle linéaire, ramifié ou cyclique pouvant être saturé, insaturé ou partiellement insaturé et pouvant être optionnellement substitué par 3 groupes R9 au maximum; -OH; $-NO_2$; alkoxy $C_{1-6}$; alkyle $C_{1-6}$; halogène; haloalkyle; $-NH_2$; -CN; -NHCOR9; -CONHR9; $-NHSO_2R9$; $-SO_2NHR9$; cycle aromatique ou hétéoaromatique à 5- ou 6 atomes pouvant être optionnellement substitué par 3 groupes R9 au maximum; un cycle homoaromatique qui peut être condensé avec un cycle à 5- ou 6-atomes, aliphatique ou un hétérocycle aromatique et ce cycle homoaromatique (condensé) peut être optionnellement substitué par 3 groupes R9 au maximum; R9 est sélectionné parmi -OH; alkoxy linéaire, ramifié ou cyclique pouvant être saturé, insaturé ou partiellement insaturé; halogène; haloalkyle; alkyle $C_{1-6}$;$-NH_2$; $-NO_2$; -CN et phényle;
R10 est sélectionné parmi H, halogène, haloalkyle, alkyle $C_{1-6}$;
n est un nombre entier allant de 0 à 2;
X représente un O;
V représente un C ou un S; W représente un O ou un S; et
T, si V est un S, représente un O ou est absent, ou, si V est un C, est absent;

ou un sel de ces dérivés,
qui est pour l'utilisation dans le traitement et/ou la prévention de maladies inflammatoires.

**21.** Composé de formule (IVb) pour l'utilisation dans le traitement et/ou la prévention de maladies inflammatoires selon la revendication 20, pour lequel les variables, si présentes, ont la signification suivante:

R1 et R3 sont sélectionnés, indépendamment l'un de l'autre, parmi H; un -OH; alkoxy linéaire, ramifié ou cyclique pouvant être saturé, insaturé ou partiellement insaturé; alkyle linéaire, ramifié ou cyclique pouvant être saturé, insaturé ou partiellement insaturé; $-N(R8)_2$ ; halogène; haloalkyle et phényle;
R2 et R4 sont sélectionnés, indépendamment l'un de l'autre, parmi H; -OR8;-COR8; -COOR8; -CONHR8; -OCOR8; $-SO_2N(R8)_2$; $-N(R8)_2$; -NHCOR8; - $NHSO_2R8$; halogène; $-CH_2N(R8)_2$; $-CH_2OR8$; aryle optionnellement substitué par 3 groupes R8 au maximum; alkyle $C_{1-6}$ pouvant être optionnellement substitué par 3 groupes R8 au maximum et haloalkyle $C_{1-6}$;
R5 représente un alkyle $C_{1-6}$;
R8 est sélectionné parmi H; alkyle $C_{1-6}$ pouvant être optionnellement substitué par 3 groupes R9 au maximum; halogène; haloalkyle $C_{1-6}$; -OH; alkoxy $C_{1-6}$;$-NH_2$; et cycle aromatique ou hétéoaromatique à 5- ou 6-atomes pouvant être optionnellement substitué par 3 groupes R9 au maximum;
R9 est sélectionné parmi -OH; alkoxy linéaire, ramifié ou cyclique pouvant être saturé, insaturé ou partiellement insaturé; halogène; haloalkyle; alkyle $C_{1-4}$;$-NH_2$ et phényle; et/ou
n est 0 ou 1.

**22.** Composé de formule (IVb) pour l'utilisation dans le traitement et/ou la prévention de maladies inflammatoires selon la revendication 21, pour lequel R5 représente un méthyle, isopropyle ou cyclopropyle et/ou R8 représente un phényle ou benzyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TRAVIS, R.C. et al.** *Breast Cancer Res.,* 2003, vol. 5, 239-247
- **WILDING, G.** *Cancer Surv.,* 1992, vol. 14, 113-130
- **PIETSCHMANN, P. et al.** *Gerontology,* 2009, vol. 55, 3-12
- **CREE, M. et al.** *J. Am. Geriatr. Soc.,* 2000, vol. 48, 283-288
- **FINK, H.A. et al.** *J. Clin. Endocrinol. Metab.,* 2006, vol. 91, 3908-3915
- **MEIER, C. et al.** *Arch. Intern. Med.,* 2008, vol. 168, 47-54
- **HOYLAND, J.A. et al.** *Bone,* 1997, vol. 20, 87-92
- **VANDERSCHUEREN, D. et al.** *Endocr. Rev.,* 2004, vol. 25, 389-425
- **VANDERSCHUEREN, D. et al.** *Curr. Opin. Endocrinol. Diabetes Obes.,* 2008, vol. 15, 250-254
- **BLAND, R.** *Clin. Sci.,* 2000, vol. 98, 217-240
- **DONG, Y. et al.** *J. Bone Miner. Res.,* 1998, vol. 13, 1539-1546
- **FEIX, M. et al.** *Mol. Cell. Endocrinol.,* 2001, vol. 171, 163-164
- **JANSSEN, J:M.** *J. Cell. Biochem.,* 1999, vol. 75, 528-37
- **LABRIE, F.** *Mol. Cell. Endocrinol.,* 1991, vol. 78, C113-118
- **WU, L. et al.** *J. Biol. Chem.,* 1993, vol. 268, 12964-12969
- **LU, M.** *J. Biol. Chem.,* 2002, vol. 277, 22123-22130
- **PENNING, T.M. et al.** *Endocr. Rev.,* 1997, vol. 18, 281-305
- **LUKACIK, P. et al.** *Mol. Cell. Endocrinol.,* 2006, vol. 248, 61-71
- **LUU-THE, V. et al.** *Best Pract. Res. Clin. Endo-crinol. Metab.,* 2008, vol. 22, 207-221
- **MOELLER, G. et al.** *Mol. Cell. Endocrinol.,* 2009, vol. 301, 7-19
- **EYRE, L.J. et al.** *J. Bone Miner. Res.,* 1998, vol. 13, 996-1004
- **VAN DER EERDEN, B.C.J. et al.** *J. Endocrinol.,* 2004, vol. 180, 457-467
- **SAM, K.M.** *J. Med. Chem.,* 1995, vol. 38, 4518-4528
- **POIRIER, D. et al.** *Mol. Cell. Endocrinol.,* 2001, vol. 171, 119-128
- **BYDAL, P. et al.** *Eur. J. Med. Chem.,* 2009, vol. 44, 632-644
- **COOK, J.H. et al.** *Tetrahedron Letters,* 2005, vol. 46, 1525-1528
- **GUNN, D.** *Bioorg. Med. Chem. Lett.,* 2005, vol. 15, 3053-3057
- **WOOD, J. et al.** *Bioorg. Med. Chem. Lett.,* 2006, vol. 16, 4965-4968
- **BAGI, C.M. et al.** *J. Musculoskelet. Neuronal Interact.,* 2008, vol. 8, 267-280
- **LINDSAY, R. et al.** *Obst. Gynecol.,* 1990, vol. 76, 290-295
- **TURNER, R.T. et al.** *Endocr. Rev.,* 1994, vol. 16, 275-300
- **MECZEKALSKI, B. et al.** *Gynecol. Endocrinol.,* 2010, vol. 26, 652-657
- **KRYCHMAN, J.L. et al.** *J. Sex. Med.,* 2010
- **MAC BRIDE, M.B.** *Mayo Clin. Proc.,* 2010, vol. 85, 87-94
- **SMITH, A.L et al.** *Discov. Med.,* 2010, vol. 10, 500-510
- **ODUWOLE O.O. et al.** *Steroid. Biochem.,* 2003, vol. 87, 133-140
- **BEHL, C. et al.** *Biochem. Biophys. Res. Commun.,* 1995, vol. 216, 473-482
- **PIKE, C.J.** *Front Neuroendocrinol.,* 2009, vol. 30, 239-258
- **BOURQUE, M. et al.** *Front Neuroendocrinol.,* 2009, vol. 30, 142-157
- **SCHMIDT, P.J. et al.** *Ann. N. Y. Acad. Sci.,* 2009, vol. 179, 70-85
- **KARJALAINEN, A. et al.** *Arterioscler. Thromb. Vase. Biol.,* 2000, vol. 4, 1101-1106
- **TRAISH, A.M. et al.** *J. Androl.,* 2009, vol. 30, 477-494
- **XING D. et al.** *Arterioscler. Thromb.Vasc. Biol.,* 2009, vol. 29, 289-295
- **MOORADIAN, A.D. et al.** *Endocr. Rev.,* 1987, vol. 8, 1-28
- **GEORGALA S et al.** *Dermatology,* 2004, vol. 208, 178-9
- **CUTOLO, M. et al.** *Ann. N. Y. Sci.,* 2010, vol. 1193, 36-42
- **ISLANDER U. et al.** *Mol. Cell. Endocrinol.,* 2010
- **KRUCHTEN, P. et al.** *Mol. Cell. Endocrinol.,* 2009, vol. 301, 154-159
- **ZIMMERMANN, J. et al.** *J. Steroid Biochem. Mol. Biol.,* 2005, vol. 94, 57-66